# EUROPEAN PATENT SPECIFICATION

(11) **EP 4 154 905 B1**
(45) Date of publication and mention of the grant of the patent: **19.08.2026**
(21) Application number: 21809500.8
(22) Date of filing: 17.05.2021
(51) Int. Cl.: A61K 35/74, A61P 35/04, A61K 45/06, A61P 35/00, A61P 37/04, A61K 9/14, A61K 9/51, A61K 47/46, A61K 39/39, A61K 39/00, A61K 35/13, A61K 40/10, A61K 40/42

(54) **TUMOR VACCINE COMPRISING MEMBRANE COMPONENTS, PREPARATION METHOD THEREFOR AND USE THEREOF**
TUMORIMPFSTOFF ENTHALTEND MEMBRANKOMPONENTEN, HERSTELLUNGSVERFAHREN DAFÜR UND VERWENDUNG DAVON
VACCIN ANTITUMORAL COMPRENANT DES COMPOSANTS DE MEMBRANES, SON PROCÉDÉ DE PRÉPARATION ET SON UTILISATION

(30) Priority: 18.05.2020 CN 202010418720
(43) Date of publication of application: 29.03.2023
(73) Proprietor: Intell Nanovax Biotech Co., Ltd., Beijing 100094 (CN)
(72) Inventor: NIE, Guangjun, Beijing 100190 (CN); ZHAO, Ruifang, Beijing 100190 (CN); CHEN, Long, Beijing 100190 (CN); QIN, Hao, Beijing 100190 (CN); ZHAO, Yuliang, Beijing 100190 (CN)
(74) Representative: Weickmann & Weickmann PartmbB
(86) International application number: PCT/CN2021/094037
(87) International publication number: WO 2021/233237

(56) References cited:
- WO-A1-2019/134036
- WO-A2-2016/172551
- CN-A- 103 857 387
- CN-A- 109 414 461
- DIANA DEHAINI ET AL: "Erythrocyte-Platelet Hybrid Membrane Coating for Enhanced Nanoparticle Functionalization", ADVANCED MATERIALS, VCH PUBLISHERS, DE, vol. 29, no. 16, 15 February 2017 (2017-02-15), pages n/a, XP071871511, ISSN: 0935-9648, DOI: 10.1002/ADMA.201606209
- JIN JIEFU ET AL: "Biomimetic Nanoparticles Camouflaged in Cancer Cell Membranes and Their Applications in Cancer Theranostics", FRONTIERS IN ONCOLOGY, vol. 9, 1 January 2020 (2020-01-01), CH, XP055808473, ISSN: 2234-943X, DOI: 10.3389/fonc.2019.01560
- YANG RONG ET AL: "Cancer Cell Membrane-Coated Adjuvant Nanoparticles with Mannose Modification for Effective Anticancer Vaccination", vol. 12, no. 6, 17 May 2018 (2018-05-17), US, pages 5121 - 5129, XP055791273, ISSN: 1936-0851, Retrieved from the Internet <URL:https://pubs.acs.org/doi/pdf/10.1021/acsnano.7b09041> DOI: 10.1021/acsnano.7b09041
- RONNIE H. FANG ET AL: "Cell Membrane Coating Nanotechnology", ADVANCED MATERIALS, vol. 30, no. 23, 27 March 2018 (2018-03-27), DE, pages 1 - 34, XP055664194, ISSN: 0935-9648, DOI: 10.1002/adma.201706759
- XIANG GONG ET AL: "Emerging Approaches of Cell-Based Nanosystems to Target Cancer Metastasis", ADVANCED FUNCTIONAL MATERIALS, WILEY - V C H VERLAG GMBH & CO. KGAA, DE, vol. 29, no. 48, 8 September 2019 (2019-09-08), pages n/a, XP072408926, ISSN: 1616-301X, DOI: 10.1002/ADFM.201903441
- TANISHQ ABRAHAM ET AL: "Engineering approaches of smart, bio-inspired vesicles for biomedical applications", PHYSICAL BIOLOGY, INSTITUTE OF PHYSICS PUBLISHING, BRISTOL, GB, vol. 15, no. 6, 25 June 2018 (2018-06-25), pages 61001, XP020331760, ISSN: 1478-3975, [retrieved on 20180625], DOI: 10.1088/1478-3975/AAC7A2
- STRAHL HENRIK ET AL: "Bacterial membranes: structure, domains, and function", 1 January 2017 (2017-01-01), XP093135967, Retrieved from the Internet <URL:https://www.annualreviews.org/doi/pdf/10.1146/annurev-micro-102215-095630> [retrieved on 20240228], DOI: 10.1146/annurev-micro-102215
- CHEN LONG ET AL: "Bacterial cytoplasmic membranes synergistically enhance the antitumor activity of autologous cancer vaccines", SCIENCE TRANSLATIONAL MEDICINE, vol. 13, no. 601, 7 July 2021 (2021-07-07), pages eabc2816, XP093121940, ISSN: 1946-6234, DOI: 10.1126/scitranslmed.abc2816
- HUANG WEIWEI; SHU CONGYAN; HUA LIANGQUN; ZHAO YILIN; XIE HANGHANG; QI JIALONG; GAO FULAN; GAO RUIYU; CHEN YONGJUN; ZHANG QISHU; LI: "Modified bacterial outer membrane vesicles induce autoantibodies for tumor therapy", ACTA BIOMATERIALIA, vol. 108, 3 April 2020 (2020-04-03), AMSTERDAM, NL, pages 300 - 312, XP086152610, ISSN: 1742-7061, DOI: 10.1016/j.actbio.2020.03.030
- JAIN SAPNA, PILLAI JONATHAN: "Bacterial membrane vesicles as novel nanosystems for drug delivery", INTERNATIONAL JOURNAL OF NANOMEDICINE, vol. 12, 29 August 2017 (2017-08-29), pages 6329 - 6341, XP055870015, DOI: 10.2147/IJN.S137368
- LIU, CHANG ET AL.: "Research on Bacterial Outer Membrane Vesicles and Its Application Progress in the Field of Medical Biotechnology", CHINESE MEDICINAL BIOTECHNOLOGY, vol. 13, no. 5, 31 October 2018 (2018-10-31), pages 452 - 457, XP055870022

## Description

### TECHNICAL FIELD

The present application relates to the field of biological medicine, and in particular to a tumor vaccine, and a preparation method therefor and use thereof.

### BACKGROUND OF THE INVENTION

Surgery remains the preferred therapeutic schedule for cancer therapy and plays a significant role in the diagnosis, staging, reconstruction and remission of cancer. Advances in the fields of conventional chemoradiotherapy and novel adjuvant chemoradiotherapy have also improved the prognosis of patients with cancer to a large extent. However, despite the ongoing development of therapeutic techniques, the recurrence and metastasis of localized tumors remain a major source of morbidity and mortality in patients with cancer. Many studies have found that both surgical procedures and destruction of the tumor resection margins may result in shedding of individual tumor cells into the peripheral circulation. At the same time, surgical stress can also affect neuro-endocrine regulation, metabolism, inflammatory responses, and the immune microenvironment around tumor cells, resulting in immunosuppression. Cellular immunosuppression can persist for several days after major surgery, and the period of time can become a window of tumor invasion into the immune system. Immunosuppression at this stage is often manifested by loss of tumor cell surveillance, and reduction of NK cells, cytotoxic T lymphocytes, dendritic cells, and helper T lymphocytes in the peripheral blood.

Currently, the types of vaccines used to prevent postoperative tumor recurrence are limited. CN104619833A describes a peptide vaccine against cancer, specifically an epitope peptide derived from UBE2T and eliciting CTL, and further provides a pharmaceutical composition containing such epitope peptide derived from UBE2T or a polynucleotide encoding the polypeptide as an active ingredient. In addition, the disclosure provides a method for treating and/or guarding against, and/or preventing postoperative recurrence thereof, a method for inducing CTL, and a method for inducing anti-tumor immunity. The method is performed by using an epitope peptide derived from UBE2T, a polynucleotide encoding the peptide, or an antigen-presenting cell presenting the peptide, or the pharmaceutical composition of the present disclosure. CN109481418A discloses an anti-tumor nanoparticle, and a preparation method and use thereof. The provided anti-tumor nanoparticle comprises a core, a wrapping layer wrapping the core and an immune layer coating the wrapping layer. The anti-tumor nanoparticle obtained by wrapping a nanoparticle with an immune adjuvant can achieve a combined effect of photodynamic therapy and immunotherapy. However, the types of current vaccines for preventing postoperative tumor recurrence are limited. Besides, there is a need in the art for a vaccine that has better efficacy, higher safety, convenient preparation, and a wide application range. Therefore, it is very meaningful to develop a novel tumor vaccine having a remarkable effect of preventing postoperative recurrence of cancer, and a method for preparing the same.

### SUMMARY OF THE INVENTION

The invention is as defined in the appended claims.

### BRIEF DESCRIPTION OF THE DRAWING

Specific features of the present application disclosed by the present disclosure are as shown in the appended claims. The features and advantages of the present application disclosed by the present application will be better understood by reference to the exemplary embodiments and drawings described in detail below. The drawings are briefly described below:

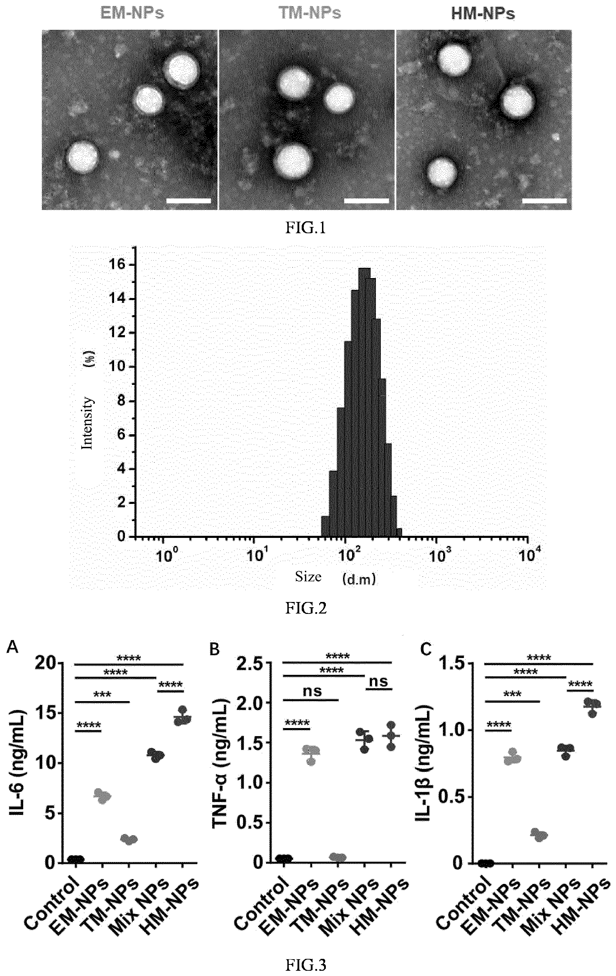

FIG. 1 shows transmission electron micrographs of EM-NPs, TM-NPs, and HM-NPs (hybrid membrane nanoparticles) with a scale bar of 100 nm.
FIG. 2 shows dynamic light scattering of HM-NPs (hybrid membrane nanoparticles).
FIGs. 3A-3C show comparisons of levels of IL-6, TNF-α and IL-1β in supernatants of a control group, an EM-NPs group, a TM-NPs group, a Mix NPs group, and an HM-NPs group (A is a level of IL-6, B is a level of TNF-α, and C is a level of IL-1β).

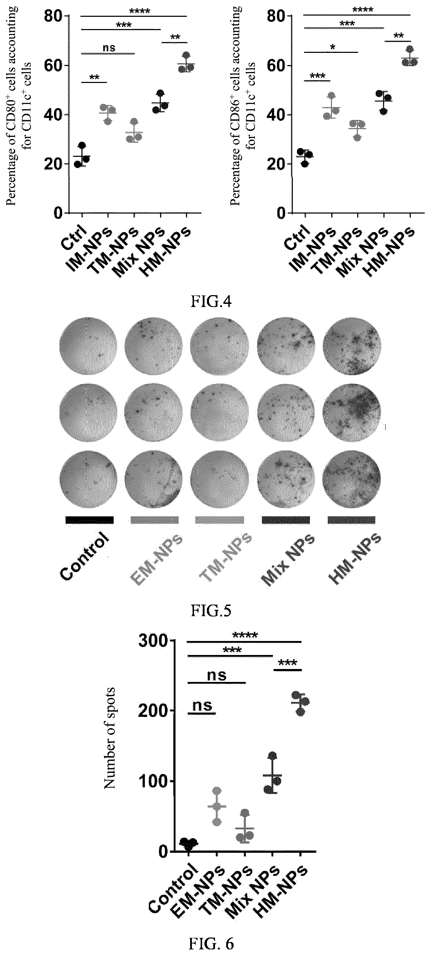

FIG. 4 shows comparisons of levels of CD80 and CD86 in supernatants of a control group, an EM-NPs group, a TM-NPs group, a Mix NPs group, and an HM-NPs group (the left is a level of CD80 and the right is a level of CD86).
FIG. 5 shows secretion amounts of IFN-γ in supernatants of the control group, the EM-NPs group, the TM-NPs group, the Mix NPs group, and the HM-NPs group detected by an ELISPOT reagent kit.
FIG. 6 shows a statistical result of the number of spots in the control group, the EM-NPs group, the TM-NPs group, the Mix NPs group, and the HM-NPs group using a CTL-ImmunoSpot Plate Reader software.

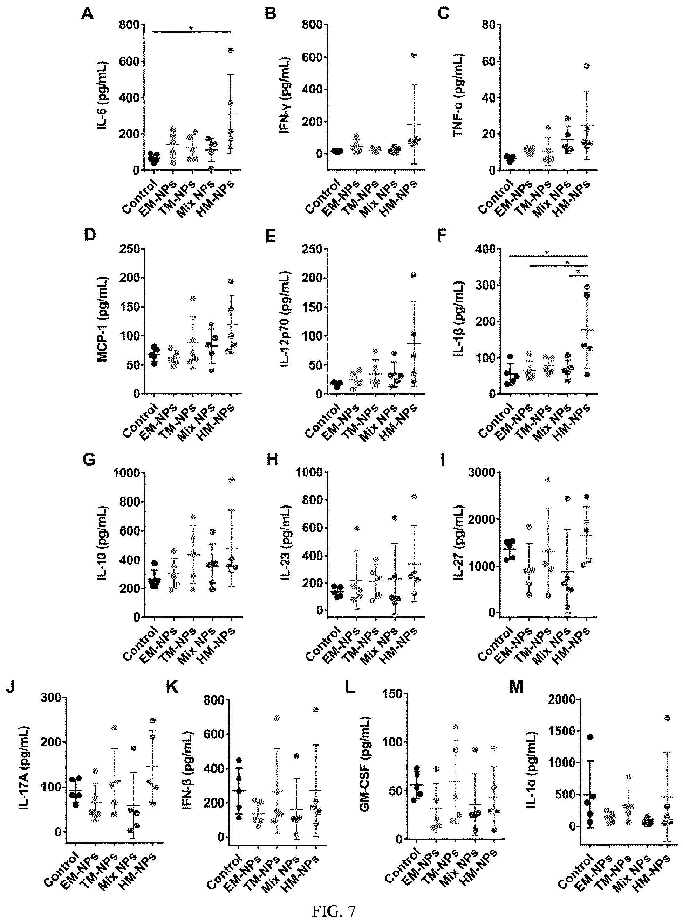

FIGs. 7A-7M show results of secretion levels of inflammatory factors and chemokines in serums of mice in the control group, the EM-NPs group, the TM-NPs group, the Mix NPs group, and the HM-NPs group (A, B, C, D, E, F, G, H, I, J, K, L, and M respectively correspond to IL-6, IFN-γ, TNF-α, MCP-1, IL-12p70, IL-1β, IL-10, IL-23, IL-27, IL-17A, IFN-β, GM-CSF, and IL-1α).

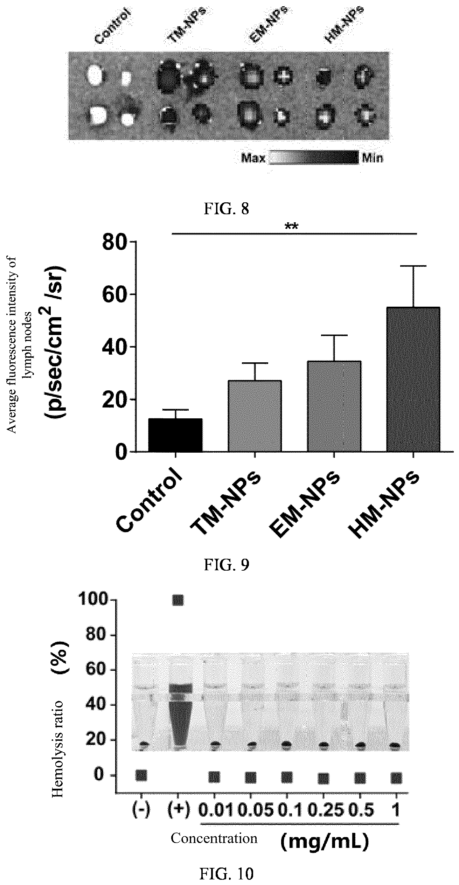

FIG. 8 shows *in-vivo* imaging of an effect of the product prepared in example 1 in enriching lymph nodes.
FIG. 9 shows a statistical result of the effect of the product prepared in example 1 in enriching lymph nodes.
FIG. 10 shows a result of a hemolysis test of HM-NPs (hybrid membrane nanoparticles).

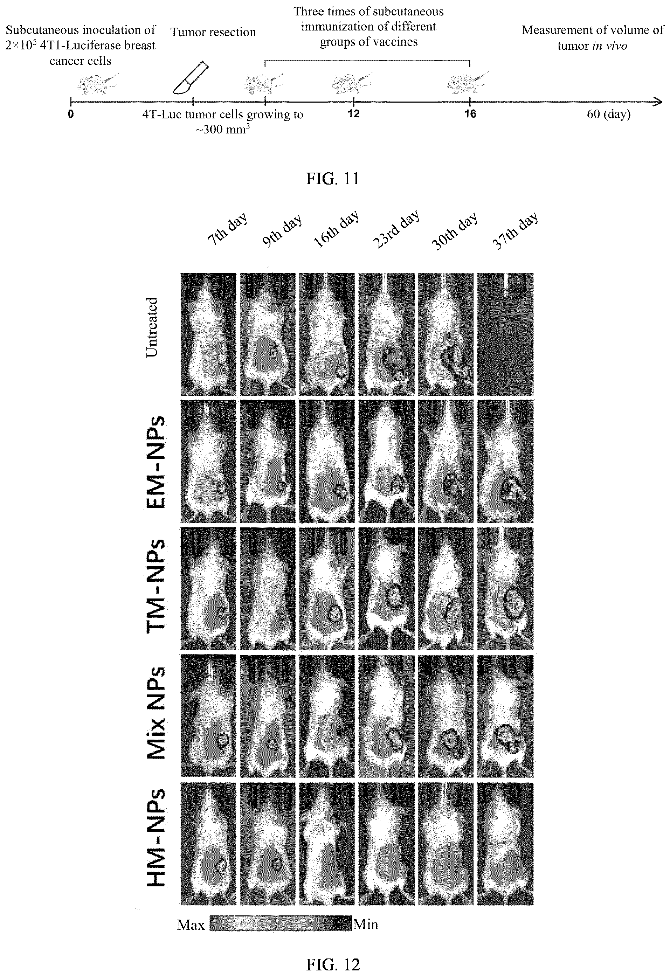

FIG. 11 shows a flow chart of a test operation of example 6.
FIG. 12 shows *in-vivo* imaging of fluorescence intensity of tumors of tumor-bearing mice in the control group, the EM-NPs group, the TM-NPs group, the Mix NPs group, and the HM-NPs group.

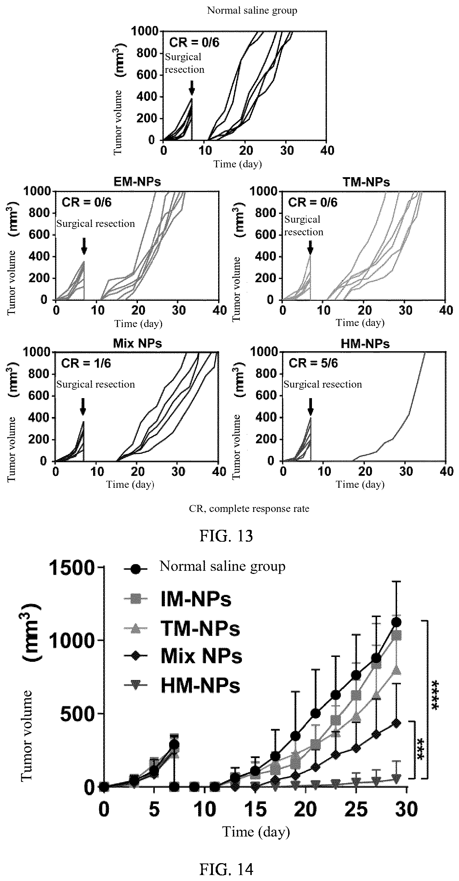

FIG. 13 shows a change of a tumor volume with time of each tumor-bearing mouse in the control group, the EM-NPs group, the TM-NPs group, the Mix NPs group, and the HM-NPs group.
FIG. 14 shows the change of the tumor volume with time of the tumor-bearing mice in the control group, the EM-NPs group, the TM-NPs group, the Mix NPs group, and the HM-NPs group.

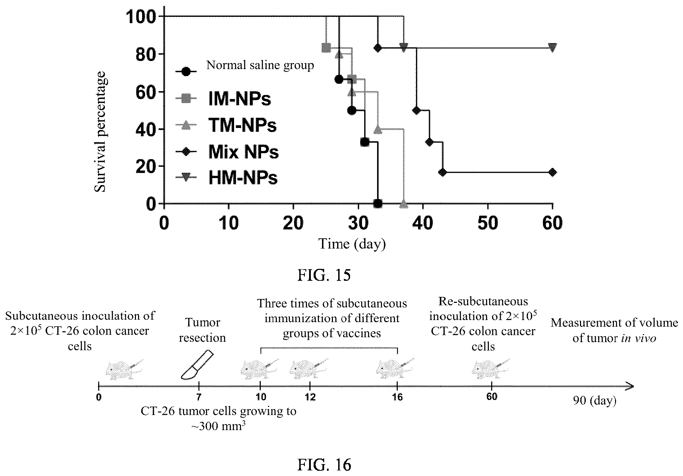

FIG. 15 shows survival curves of the tumor-bearing mice in the control group, the EM-NPs group, the TM-NPs group, the Mix NPs group, and the HM-NPs group.
FIG. 16 shows a flow chart of a test operation of example 7.

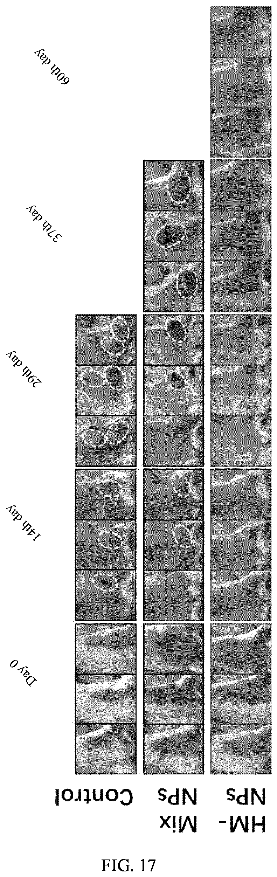

FIG. 17 shows the change of the tumor with time of the tumor-bearing mice in the control group, the Mix NPs group, and the HM-NPs group.

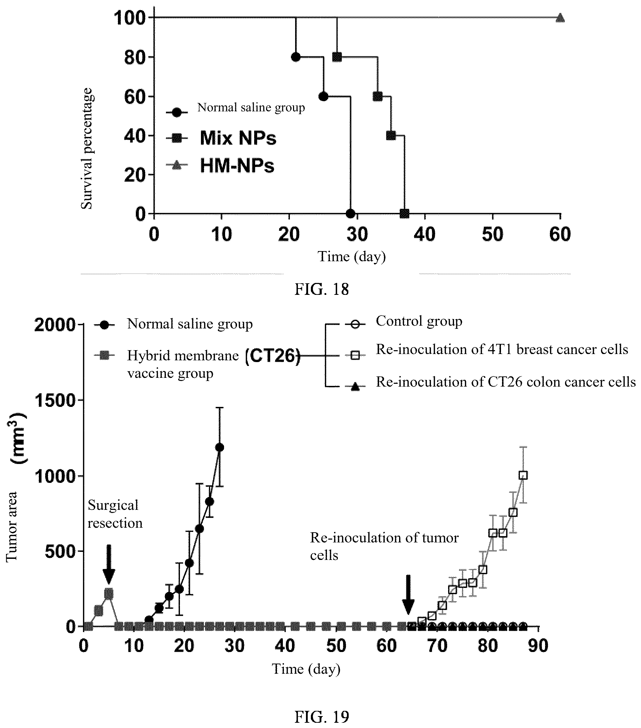

FIG. 18 shows survival curves of the tumor-bearing mice in the control group, the Mix NPs group, and the HM-NPs group.
FIG. 19 shows a result of the change of the tumor with time in each group of mice in a post-operative re-challenge test.

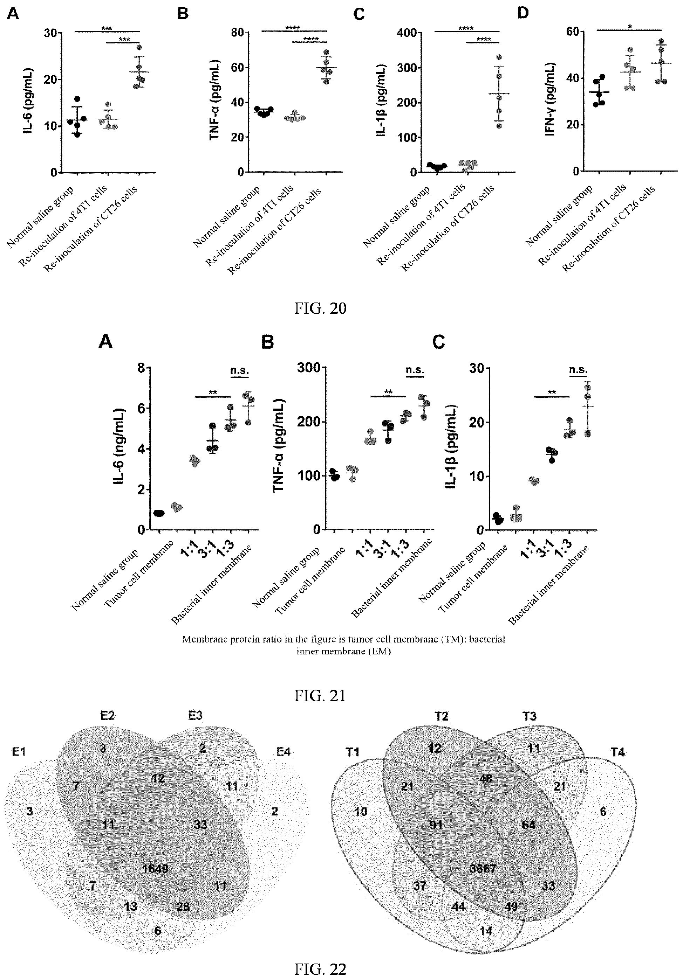

FIGs. 20A-20D show comparison of expression levels of IL-6, TNF-α, IL-1β, and IFN-γ in serums of mice in each group in the post-operative re-challenge test (A, B, C, and D correspond to IL-6, TNF-α, IL-1β, and IFN-γ respectively).
FIGs. 21A-21C show comparison of levels of IL-6, TNF-α, and IL-1β in supernatants of products of hybrid membrane nanoparticles with different membrane protein concentration ratios (A, B, and C correspond to IL-6, TNF-α, and IL-1β respectively).
FIG. 22 shows Venn diagrams of different protein expression profiles of EM (left) and TM (right) from four batches. The sum of the numbers in each big circle represents the total number of proteins expressed in four biologically independent duplicate samples (n = 4). A total of 2,302 proteins are detected in EM samples and 5,353 proteins are detected in TM samples. An overlapping part of the circles represents the co-expressed proteins. E1, E2, E3, and E4 represent four batches of the EM samples respectively. T1, T2, T3, and T4 represent four batches of the TM samples respectively.

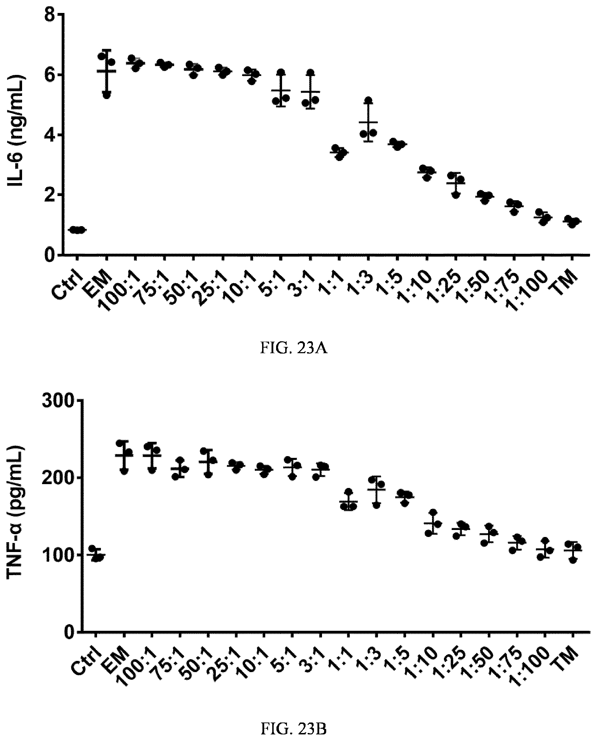

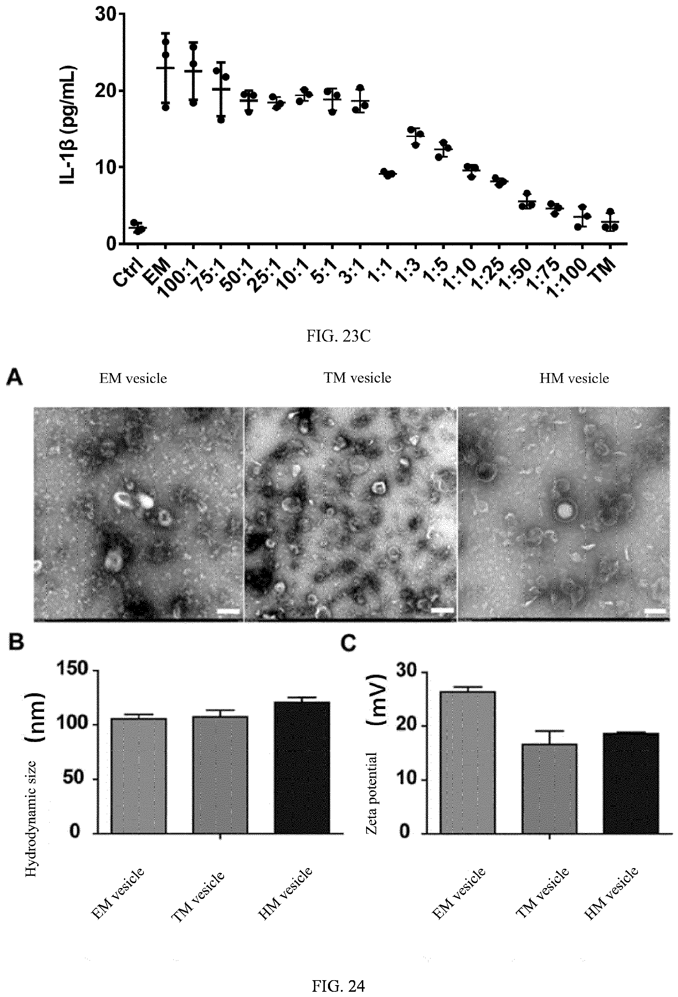

FIGs. 23A-23C show concentration results of proinflammatory cytokines in BMDC supernatants at 24 h after incubation with the indicated membrane protein mass ratio of EM to TM (EM:TM), 1:100, 1:75, 1:50, 1:25, 1:10, 1:5, 1:3, 1:1, 1:0, 0:1, 3:1, 5:1, 10:1, 25:1, 50:1, 75:1 or 100:1. (A to C) The corresponding specific ELISA kits are used to analyze production of IL-6, TNF-α, and IL-1β. *n* = 3 represents biologically independent duplicate samples. The data are expressed as mean ± standard deviation. *, *p* < 0.05; and **, *p <* 0.01. *ns* indicates no significant difference.
FIGs. 24A-24C show physical and chemical properties of three membrane vesicles before wrapping an inner core of a PLGA nanoparticle. (A) TEM images of EM, TM and HM vesicles. Scale bar, 100 nm. The experiment is performed at least three times (N = 3). (B) Hydrodynamic size and (C) Zeta potential of the EM, TM and HM vesicles. *n* = 3 represents biologically independent duplicates. The data are expressed as mean ± standard deviation.

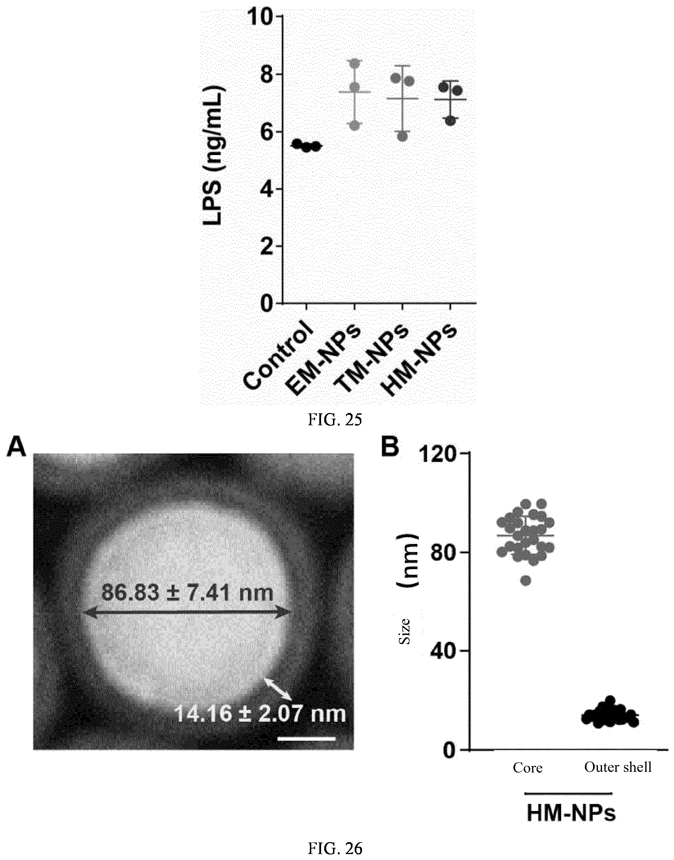

FIG. 25 shows the LPS content in EM-NPs, TM-NPs, and HM-NPs. During the extraction of EM, most LPS is removed. There is no significant difference in the LPS content among the three membrane NPs (*p >* 0.9999) (*n =* 3, biologically independent duplicates). The data are expressed as mean ± standard deviation.
FIGs. 26A-26B show a schematic diagram of the HM-NPs with a core/shell structure. (A) A representative TEM image of a single HM-NP. (B) A core size (diameter) and an outer shell thickness of the HM-NPs based on TEM statistics (n = 26 nanoparticles). The diameter of the HM-NP core is 86.83±7.41 nm (internal line) and the shell thickness is 14.16±2.07 nm (external line). The experiment is performed at least three times (N = 3). Scale bar, 20 nm. The data are expressed as mean ± standard deviation.

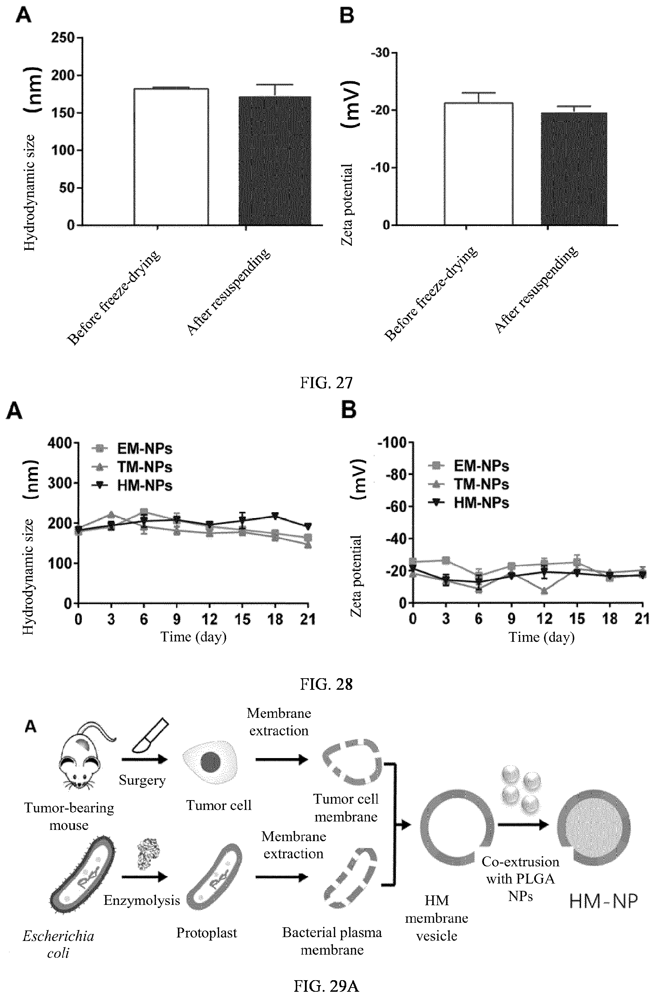

FIG. 27A-27B show physical and chemical stability of HM-NPs before freeze-drying and suspended in PBS after freeze-drying. (A and B) The size and Zeta potential of the HM-NPs in 10 wt% of sucrose before freeze-drying and resuspended in PBS after freeze-drying (n = 3, biologically independent duplicates). The data are expressed as mean ± standard deviation.
FIGs. 28A-28B show results of a particle size and charge of the EM-NPs, TM-NPs, and HM-NPs in PBS at 4° C at different time intervals. (A) The size of the EM-NPs, TM-NPs, and HM-NPs stored in PBS for 3 weeks and (B) ζ (Zeta) potential (n = 3, biologically independent duplicates). The data are expressed as mean ± standard deviation.
FIGs. 29A-29E show a schematic diagram of construction of the cancer vaccine HM-NPs and characterization results of the EM-NPs, TM-NPs, and HM-NPs. (A) Preparation of the HM-NPs, A tumor is removed from a tumor-bearing mouse by a surgery to obtain TM. Amplified *Escherichia coli* DH5α is treated with a lysozyme to remove a cell wall, a protoplast is obtained by a low-speed centrifugation, and then EM is prepared using an extraction buffer. PLGA NPs are prepared by a double emulsion method. The two membranes are mixed and repeatedly physically extruded through a liposome extruder with a pore diameter of a filter membrane of 400 nm. Then a PLGA polymer nanoparticle inner core is added and the mixture is repeatedly physically extruded through a liposome extruder with a pore diameter of a filter membrane of 200 nm. (B) TEM images of the EM-NPs, TM-NPs, and HM-NPs. Scale bar, 100 nm. (C) Size distribution and Zeta potential of the EM-NPs, TM-NPs, and HM-NPs. (D) western blot analysis of characteristic proteins in the EM-NPs, TM-NPs, and HM-NPs. Each lane is loaded with the same amount of proteins (10 µg, *n* = 3, biologically independent duplicates). (E) TEM images of the EM-NPs, TM-NPs, and HM-NPs with FtsZ (dark arrow, 5 nm gold NPs) and Na⁺/K⁺-ATPase (light arrow, 10 nm gold NPs) immunogold labeling and stained with uranyl acetate. Scale bar, 100 nm. The experiment is performed at least three times (N = 3).

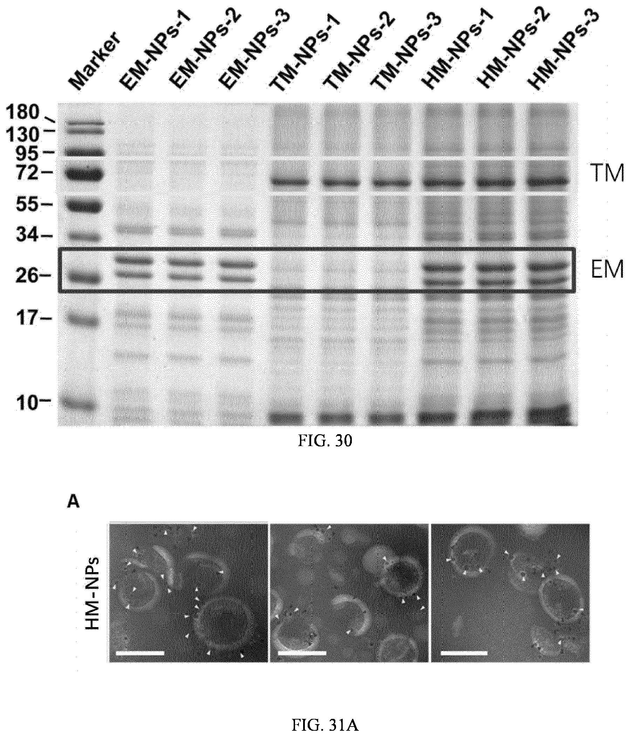

FIG. 30 shows whole protein expression profiles of the EM-NPs, TM-NPs, and HM-NPs. The protein profiles of the EM-NPs, TM-NPs, and HM-NPs are analyzed by SDS-PAGE and the NPs are stained with Coomassie brilliant blue. TM rectangle: representative proteins on the tumor cell plasma membrane (TM) of the resected autologous tumor tissue; and EM rectangle: bands of proteins with a prominent molecular weight range and derived from the *Escherichia coli* cell plasma membrane (EM). Each lane is loaded with the same amount of proteins (10 µg). The experiment is performed 3 times with 3 biological duplicates in each group.

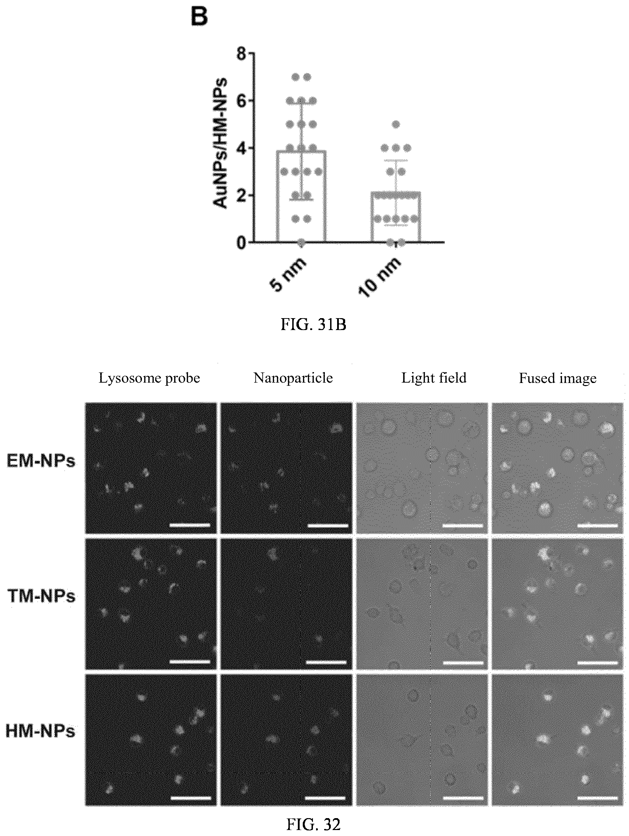

FIGs. 31A-31B show TEM images of the HM-NPs with FtsZ (dark arrow, 5 nm gold NPs) and Na⁺/K⁺-ATPase (light arrow, 10 nm gold NPs) immunogold labeling and then stained with uranyl acetate. (A) Three TEM observation areas are used to calculate a percentage of the hybrid membrane nanoparticles labeled with 5 nm and 10 nm gold nanoparticles (AuNPs) in an HM-NP preparation. As shown in (B), a total of twenty hybrid membrane nanoparticles are counted. The experiment is performed at least three times. Scale bar, 100 nm. The data are expressed as mean ± standard deviation.
FIG. 32 shows laser confocal fluorescence images of EM-NPs-RhoB, TM-NPs-RhoB, and HM-NPs-RhoB internalized into lysosomal compartments of BMDCs. The three fluorescently labeled membrane nanoparticles are incubated with BDMCs for 1 h respectively and observed by a confocal microscope. The experiment is performed 3 times with 3 biological duplicates in each group. NP loaded with rhodamine B: the second column; lysosomal compartment: the first column; bright field (BF): the third column; and merge image: the fourth column. Scale bar, 20 µm.

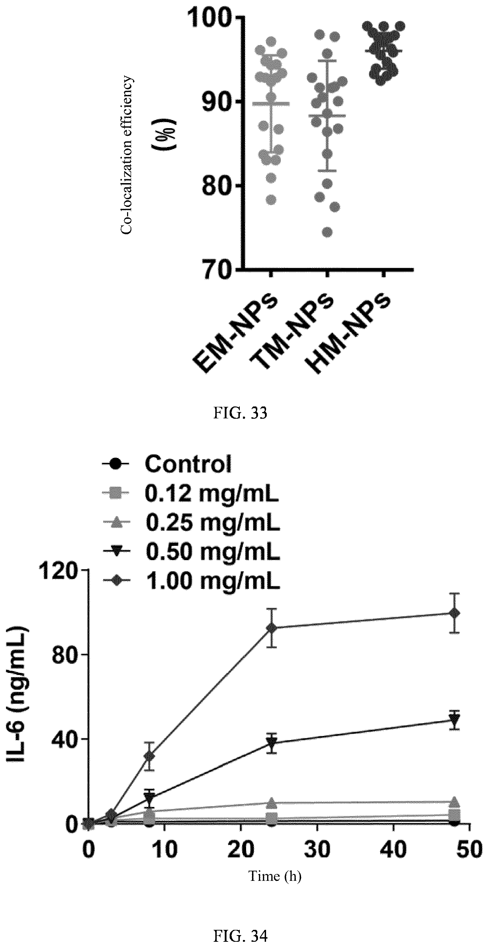

FIG. 33 shows a result of a Pearson correlation coefficient analysis between the lysosomal compartments and the membrane NPs. *n* = 20 BMDCs, statistical significance is calculated by using a Bonferroni multiple comparison test through an one-way analysis of variance. The data are expressed as mean ± standard deviation. The experiment is performed 3 times with 3 biological duplicates in each group.
FIG. 34 shows a result of the content of a proinflammatory cytokine IL-6 in a cell supernatant detected by a specific ELISA kit, wherein bacterial inner membranes (EM) of different concentrations (0, 0.12, 0.25, 0.50, and 1.00 mg/mL) were incubated with BMDCs, the cell supernatant and NPs in the BMDCs were taken at a specified time point (0, 3, 8, 24, and 48 h). In all the groups, after 24 h of incubation, the secretion of the IL-6 in each group entered a platform stage. The curve shows the change of the IL-6 secretion within 48 h. The production of the IL-6 was positively correlated with the concentrations of the EM-NPs. The data are expressed as mean ± standard deviation. The experiment is performed 3 times with 3 biological duplicates in each group.

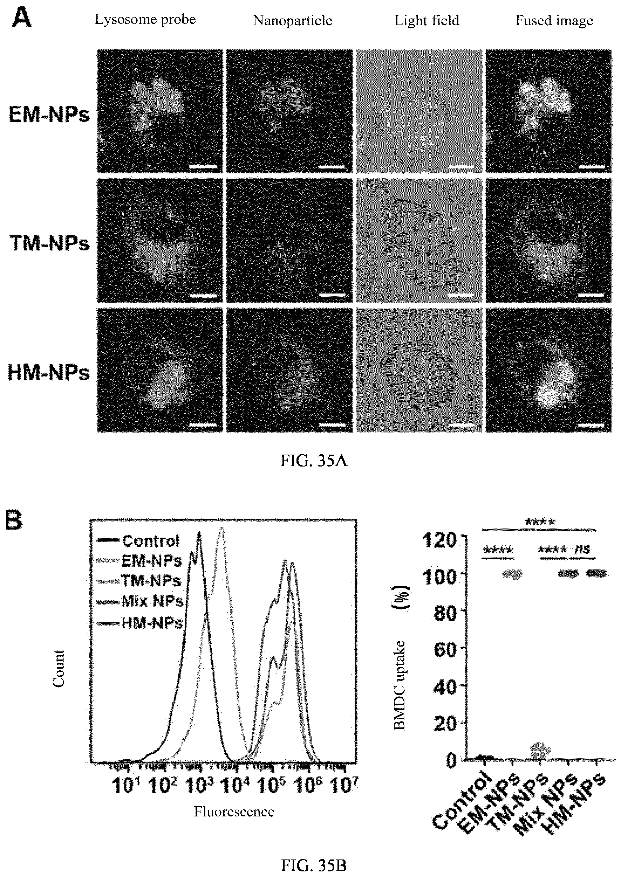

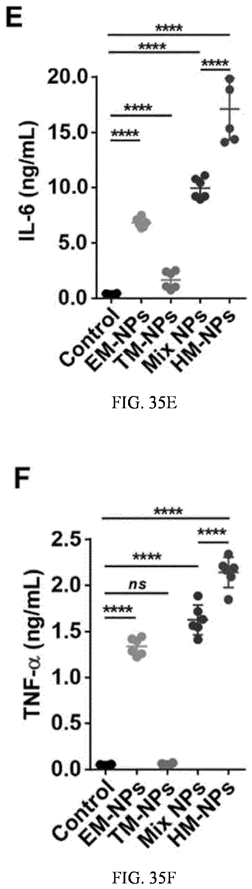

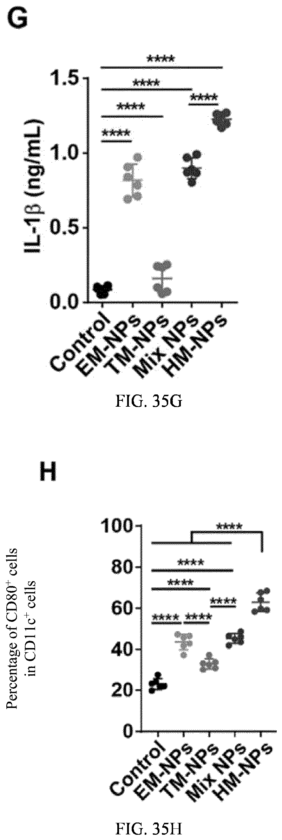

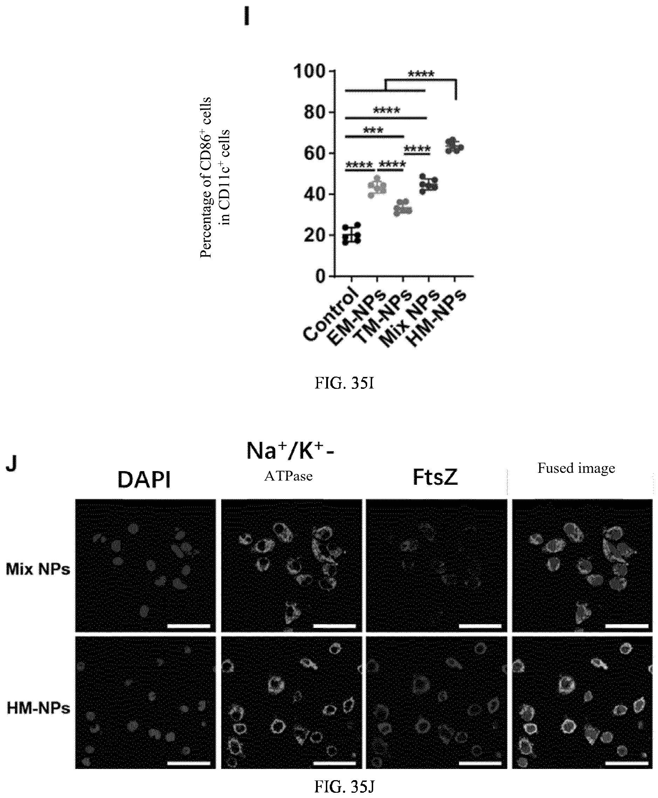

FIGs. 35A-35K show that the HM-NPs enhance the uptake of tumor antigens, activate the expression of TLRs on the surfaces of the BMDCs, and co-deliver antigens and adjuvants to the BMDCs. (A) Co-localization analysis of the EM-NPs, TM-NPs, and HM-NPs (rhodamine B; the second column) in the BMDCs, and the lysosomal compartments (the first column) by the confocal microscope (incubation for 1 h). The third column, bright field. Scale bar, 5 µm. (B) Cell uptake of membrane NPs loaded with rhodamine B after incubation with the BMDCs for 8 h assessed by flow cytometry (*n* = 6, biologically independent samples). (C) Western blot analysis of a series of BMDCs membrane TLR proteins and NF-κB protein in the membrane-coated nanoparticles. Each lane is filled with the same amount of proteins. (D) Schematic diagram of activation of TLRs and their downstream signal pathways. (E-G) Concentrations of three proinflammatory cytokines in the BMDC supernatant (*n* = 6, biologically independent samples) after incubation with membrane NPs for 24 h. (H and I) Flow cytometry analysis of CD80⁺ or CD86⁺ BMDCs (n = 6, biologically independent samples) after incubation with membrane NPs for 24 h. (J) Immunofluorescence experiment of confocal laser scanning microscopic images of mouse BMDCs treated with Mix NPs or HM-NPs for 8 h. DAPI-labeled nuclei: the first column; Na⁺/K⁺-ATPase-labeled TM: the second column; and FtsZ-labeled EM: the third column. Scale bar, 20 µm. (K) Pearson correlation coefficient analysis between TM and EM in the samples (*n* = 20 cells). The experiment is performed three times. The data are expressed as mean ± standard deviation. The statistical significance is calculated by a Bonferroni multiple comparison test through an one-way analysis of variance, wherein *** *p <* 0.001, ***** p* < 0.0001, and *ns* means no significant difference.

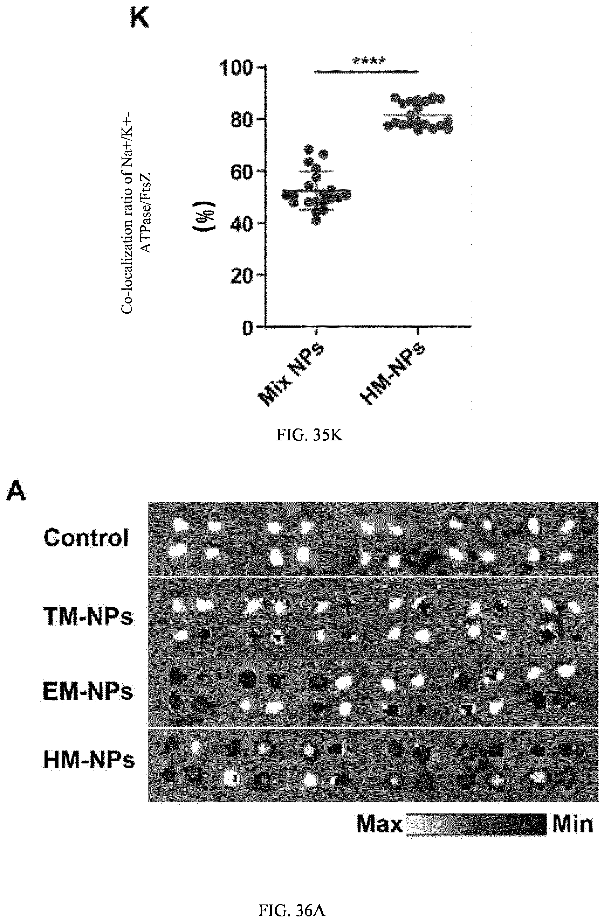

FIGs. 36A-36B show verification results of enhanced accumulation of the HM-NPs in draining lymph nodes (LNs). (A) *Ex-vivo* optical imaging of inguinal LNs. IR-780-labeled EM-NPs, TM-NPs or HM-NPs are subcutaneously injected into Balb/c mice. LNs (blank group, *n* = 5 mice; EM-NPs, TM-NPs or HM-NPs treatment groups, *n* = 6 mice in each group) were harvested 12 h later. (B) Quantification of average fluorescence intensity in FIG. A. The data are expressed as mean ± standard deviation. The experiment is performed at least three times. The statistical significance is calculated by a Bonferroni multiple comparison test through a two-way analysis of variance, wherein **** *p* < 0.0001 and *ns* means no significant difference.

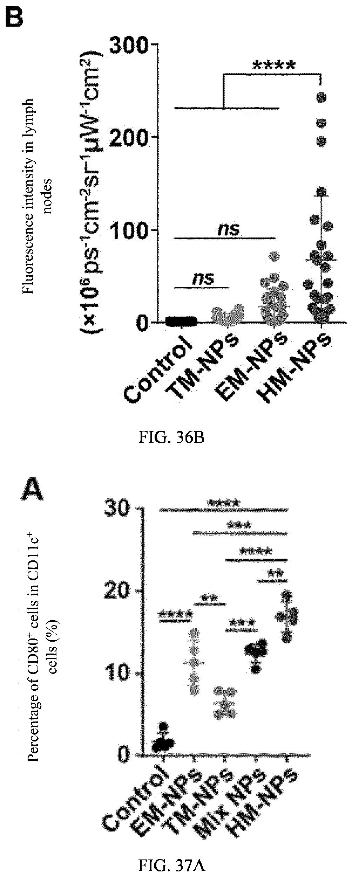

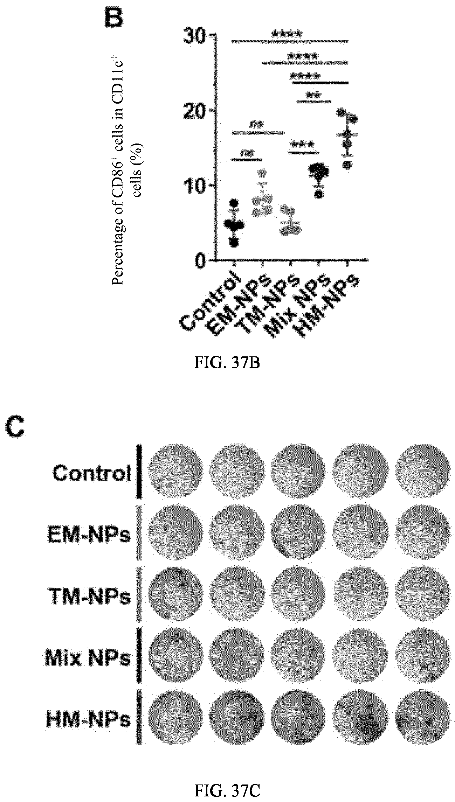

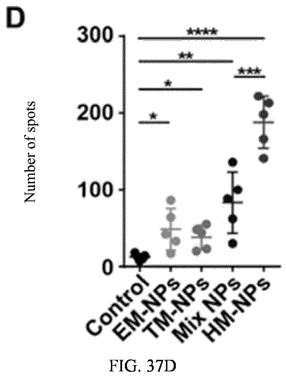

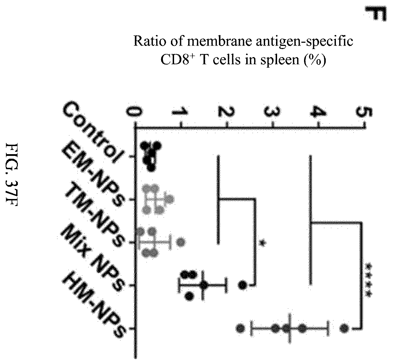

FIGs. 37A-37G show verification results of the HM-NPs promoting DC maturation in LNs and spleen T cell activation *in vivo.* (A and B) Flow cytometry analysis of CD80⁺ and CD86⁺ DCs in the inguinal lymph nodes of tumor-bearing mice subcutaneously inoculated with 4T1 through a membrane NP. A ratio of autologous membrane antigen specific T cells is determined by IFN-γ ELISPOT analysis and flow cytometry. (C-D) Representative result of IFN-γ ELISPOT analysis and quantitative number of spots in the corresponding 4T1 tumor-bearing mice (5 mice in each group). (E-F) Percentage of CD³⁺CD⁸⁺IFNγ⁺ cells in spleen (5 mice in each group) after tumor-bearing mice subcutaneously inoculated with 4T1 through a membrane NP. (G) Low-level systemic inflammatory response caused by the HM-NPs. Inflammatory cytokines and chemokines in serums (n = 5 mice in each group) are detected by using an ELISA kit based on Luminex magnetic beads in a ProcartaPlex multiple immunoassay. The experiment is performed at least three times. The data are expressed as mean ± standard deviation. In FIGs. A-D, statistical significance is calculated by using a Bonferroni multiple comparison test through an one-way analysis of variance. In G, heat maps of ProcartaPlex multiple immunoassay based on Luminex are grouped and analyzed by a two-way analysis of variance and a Bonferroni multiple comparison test, wherein ^{*}*p* < 0.05, ^{**}*p* < 0.01, *^{***}p* < 0.001, *^{****}p* < 0.0001, and *ns* means no significant difference.

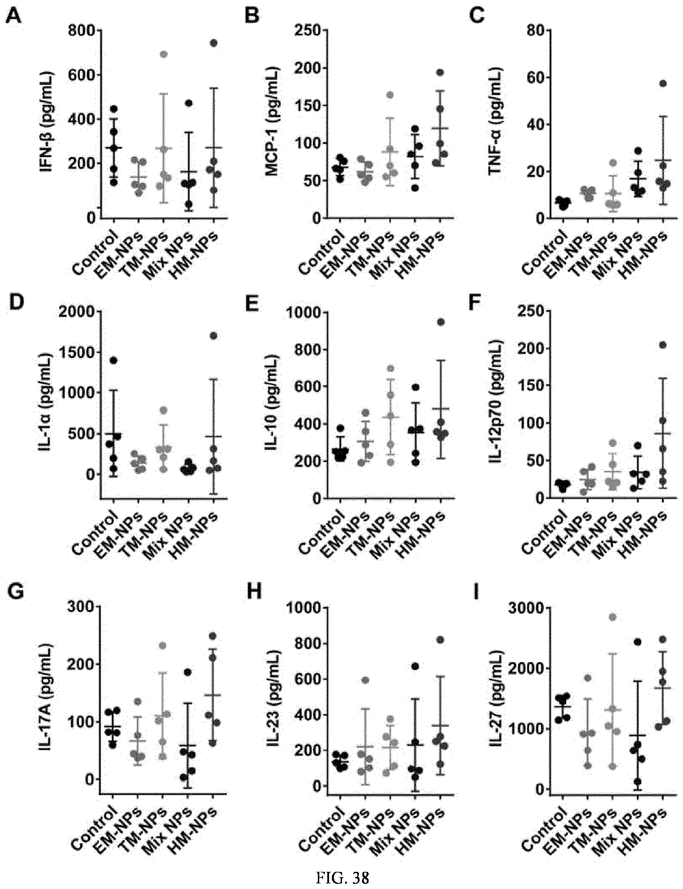

FIGs. 38A-38I show verification results of a low-level systemic inflammatory response induced by the HM-NPs. Mice are subcutaneously injected with vaccine preparations on the 3rd, 5th, and 9th days after a surgery *(n =* 5 mice in each group). The concentrations of the inflammatory cytokines and chemokines in serum are detected by using an ELISA kit based on Luminex magnetic beads and analyzed by using a BD Accuri C6 FACS flow cytometry and a ProcartaPlex Analysis software. The data are expressed as mean ± standard deviation. The experiment is performed three times. The statistical significance is calculated by a Bonferroni multiple comparison test through an one-way analysis of variance.

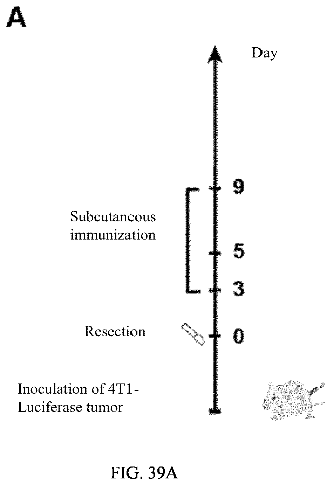

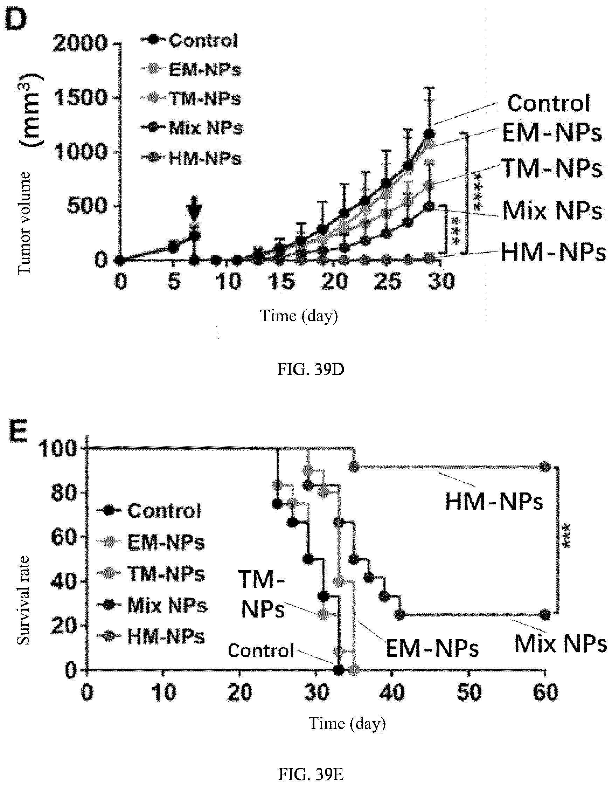

FIGs. 39A-39E show verification results of tumor regression induced by vaccination with the HM-NPs in a mouse model of 4T1-Luc tumor. (A) shows a design plan of animal experiment. Female BALB/c mice are subcutaneously inoculated with mouse 4T1 Luc breast tumor cells. When the volume of the tumor reaches about 300 mm³, it is surgically resected. Then the mice are immunized with the designated vaccine preparation on the 3rd, 5th, and 9th day after the surgery, the tumor growth of the mice is monitored by an IVIS imaging system every week (B) to obtain *in-vivo* bioluminescence images of the mice carrying 4T1-Luc tumors on different days. (C and D) show single and average tumor growth curves. Complete response (CR) means that all target lesions disappear, no new lesions appear, and tumor markers are normal, which at least last for 4 weeks. (E) shows survival curves of the mice treated with each vaccine (n = 12 mice in each group). The experiment is performed at least three times. The data are expressed as mean ± standard deviation. The experimental statistical significance is calculated by a Bonferroni growth curve multiple comparison test through a two-way analysis of variance and a log-rank test (Mantel-Cox) for comparing survival curves, wherein *** *p <* 0.001 and **** *p* < 0.0001.

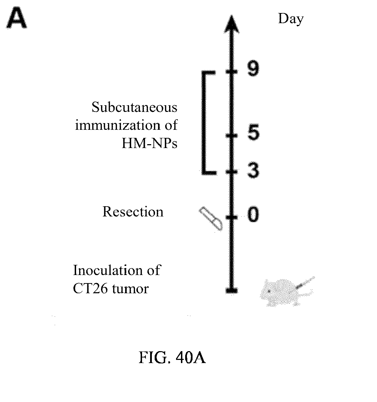

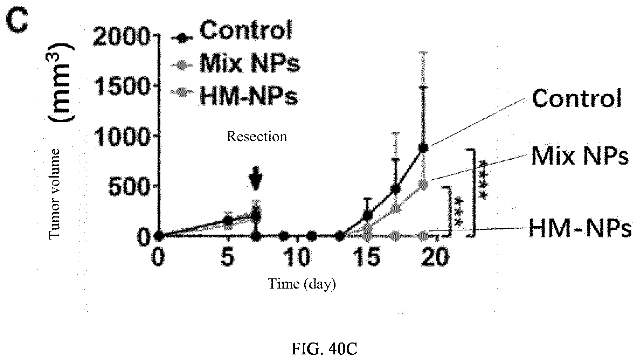

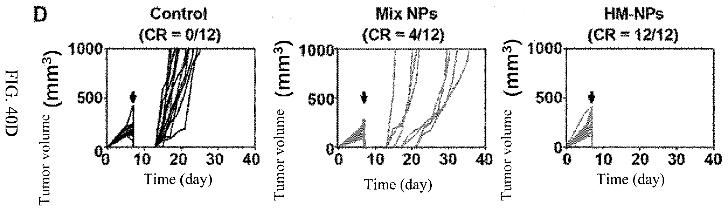

FIGs. 40A-40G show verification results of inhibition of tumor recurrence in various mouse tumor models by HM-NP vaccination. (A) shows a design plan of animal experiment. The mice are subcutaneously inoculated with tumor cells. When the volume of the tumor reaches about 300 mm³, it was surgically resected. The mice are immunized with different vaccine preparations on the 3rd, 5th, and 9th day after the surgery, and the observation lasts for 60 days (B). Images of the mice in a control group, a Mix NPs group, and an HM-NPs group. Average tumor growth curves (C), single tumor growth curves (D), and survival curves (E) of each group in a CT26 tumor model *(n =* 12 mice in each group). CR, complete response. (F) shows survival curves of each group in a B16F10 melanoma model (n = 10 mice in each group). (G) shows survival curves of each group in an EMT-6 tumor model (n = 10 mice in each group). The data are expressed as mean ± standard deviation. The experiment is performed at least three times. Statistical significance is calculated by a Bonferroni growth curve multiple comparison test through a two-way analysis of variance and a log-rank test (Mantel-Cox) of survival curves, wherein *** *p* < 0.001 and **** *p <* 0.0001.

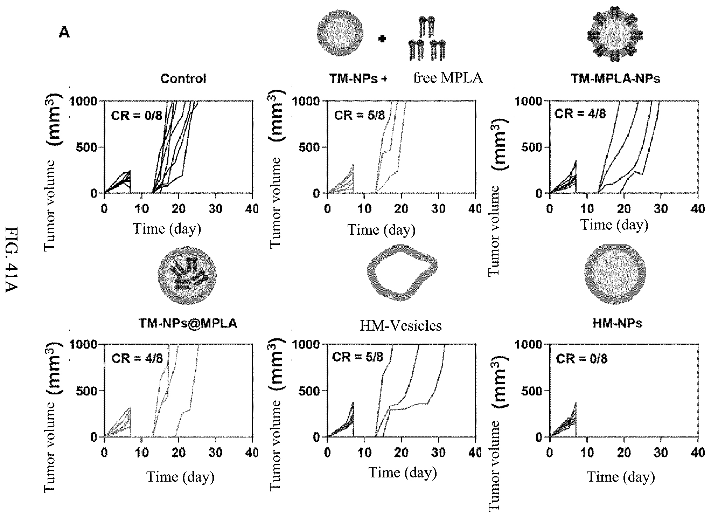

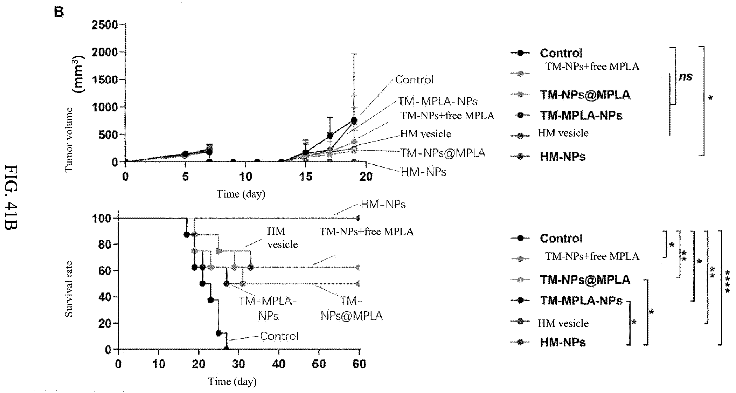

FIGs. 41A-41B show verification results that in the mouse model of CT-26 tumor, the HM-NPs have higher therapeutic efficacy than MPLA and tumor membrane preparations in three dosage forms or an HM vesicle preparation. Female BALB/c mice are subcutaneously inoculated with CT-26 colon adenocarcinoma cells (2×10⁵ cells per mouse) according to the above surgical procedure and inoculation schedule. The three vaccine dosage forms of the MPLA include TM-NP+free MPLA, MPLA anchored on tumor membrane (TM-MPLA-NPs), and PLGA nanoparticles containing MPLA with tumor membrane (TM-NPs@MPLA). In addition, hybrid membrane vesicles (HM vesicles) not containing PLGA are subcutaneously inoculated to the mice after the surgery to compare the therapeutic potential of preventing tumor recurrence with PLGA NPs coated with hybrid membrane (HM-NPs). (A) Tumor growth curves of single mouse in each group and (B) average tumor growth curves and survival curves. CR, complete response. The data are expressed as mean ± standard deviation. The experiment is performed at least three times. Statistical significance is calculated by a Bonferroni growth curve multiple comparison test through a two-way analysis of variance and a log-rank test (Mantel-Cox) of logarithmic survival curves, wherein ^{*} *p* < 0.05, ^{**} *p <* 0.01, ^{****} *p <* 0.0001, and *ns* means no significant difference.

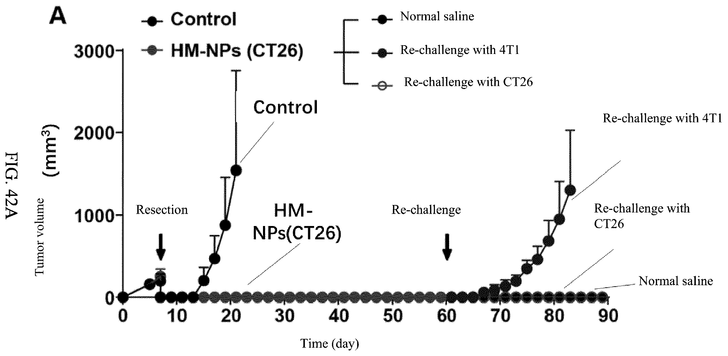

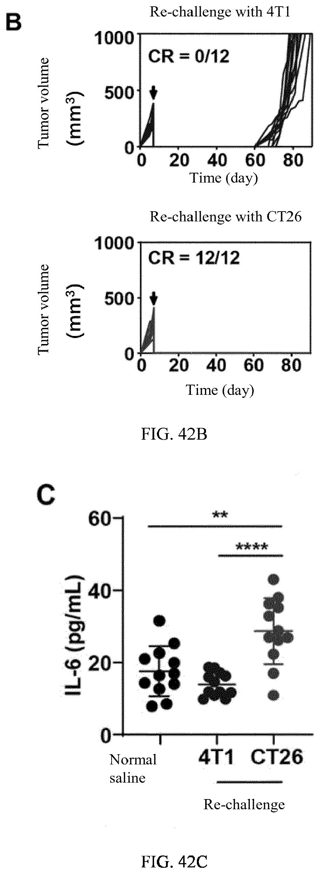

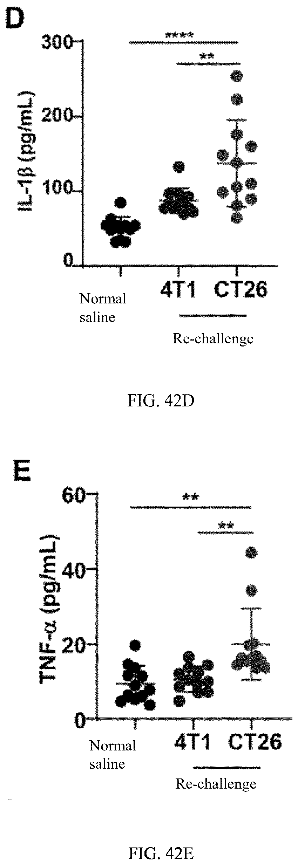

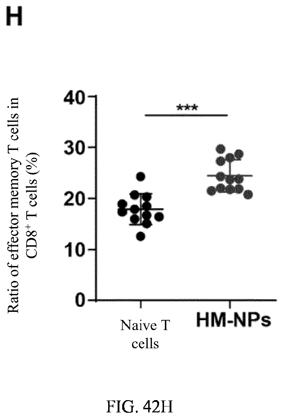

FIGs. 42A-42I show verification results that the HM-NP vaccination can provide long-term protective anti-tumor immunity; and congenital immunity and adaptive immunity are critical to vaccine efficacy. (A) The mice after the surgery are immunized with the HM-NPs. On the 60th day, the mice treated with the HM-NPs are randomly divided into different groups and inoculated with normal saline, CT-26 cells or 4T1 cells, respectively (n = 12 mice in each group). Single tumor growth curves (B) of the mice treated with the HM-NPs and inoculated with CT-26 or 4T1 cells again after 60 days. Measurement of concentrations of proinflammatory cytokines of (C) IL-6, (D) IL-1β, (E) TNF-α, and (F) IFN-γ (*n* = 12 mice in each group) in serums of the mice inoculated with CT-26 or 4T1 cells by ELISA. Spleen cells are isolated from young mice or tumor-eradicated mice treated with the HM-NPs (90 days after inoculation with primary tumor). (G-H) Representative scatter plot and percentage (%) of T cell subsets with high expression of CD44 and low expression of CD62L in CD8⁺ T cells (*n* = 12 mice in each group). The experiment is performed at least three times. (I) Average tumor growth curves of the mice consuming specific immune cell subsets (NK cells, macrophages, CD8⁺ T cells or CD4⁺ T cells) to explore their relative contribution to the observed antitumor efficacy of the HM-NPs (*n* = 5 mice in each group). The data are expressed as mean ± standard deviation. The significance of statistical data is calculated by a Bonferroni multiple comparison test through two analysis of variance, wherein ^{**} *p* < 0.01, ^{***} *p* < 0.001, and ^{****} *p* < 0.0001.

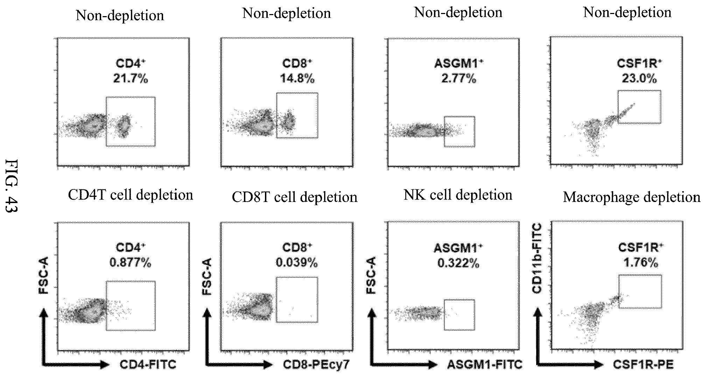

FIG. 43 shows results of specific immune cell subsets consumed during HM-NPs treatment. As shown in Table 3, the corresponding immune cell subsets are reduced by intraperitoneal injection of depleting antibodies one day before the start of the HM-NPs treatment. The peripheral blood of the mice is analyzed by flow cytometry to confirm the depletion of the macrophages (CSF1R antibody), the NK cells (ASGM1 antibody), the CD8⁺ T cells (CD8 antibody) or the CD4⁺ T cells (CD4 antibody) (*n* = 5, biologically independent samples).

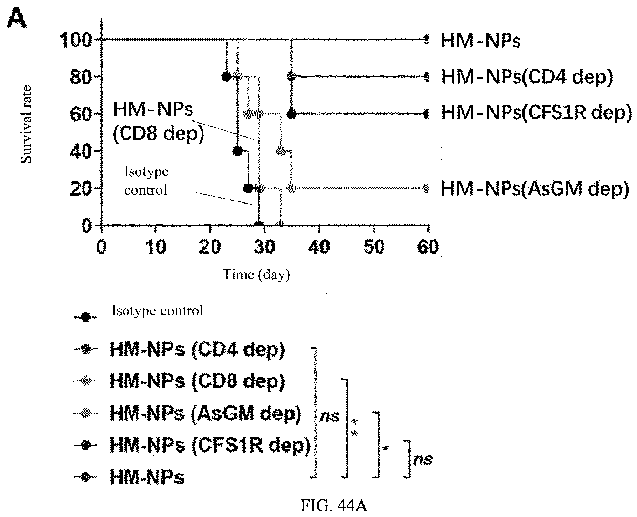

FIGs. 44A-44B show verification results of both innate immunity and adaptive immunity are necessary to effectively inhibit a tumor after the HM-NPs treatment. Female BALB/c mice are subcutaneously inoculated with CT-26 colon adenocarcinoma cells (2×10⁵ cells per mouse). When the volume of the tumor reaches about 300 mm³, it was surgically resected. Most tumor tissues are resected to prepare a vaccine preparation, and the remaining 1% is used to simulate the presence of residual micro-tumor in an operating table. The mice are evaluated on the 2nd day after the surgery and randomly divided into six groups (*n* = 5 mice in each group). In addition to the control group, the mice are immunized on the 3rd, 5th and 9th days after the surgery. One day before the start of the HM-NPs treatment, the mice macrophages (CSF1R antibody), the NK cells (ASGM1 antibody), the CD8⁺ T cells (CD8 antibody) or the CD4⁺ T cells (CD4 antibody) are depleted by intraperitoneal injection of depleting antibodies targeting designated surface markers. (A) Survival curves and (B) single tumor growth curves. CR, complete response (n = 5 mice in each group). The statistical significance is calculated by a two-way analysis of variance and a Bonferroni growth curve multiple comparison test. A log-rank test (Mantel-Cox) is used to compare survival curves, wherein ^{*} *p* < 0.05, ^{**} *p* < 0.01, and *ns* means no significant difference.

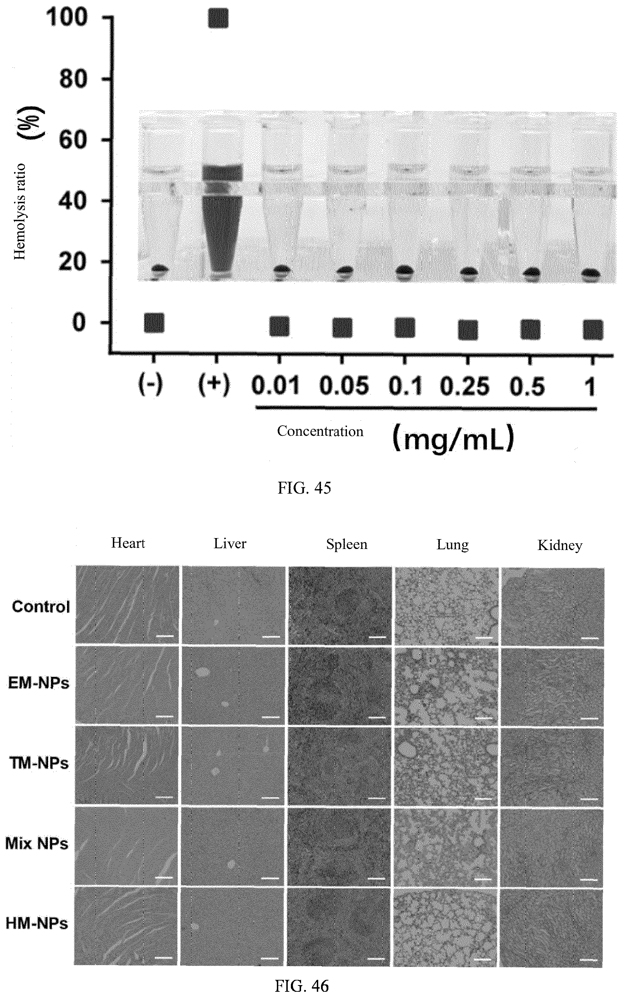

FIG. 45 shows a result of a hemolysis test of the HM-NPs with different concentrations. A series of concentrations of the HM-NPs are added to red blood cells. A hemolysis percentage is evaluated and calculated after 3 h. The red blood cells treated with PBS (0% hemolysis) and tertiary water (100% hemolysis) are used as controls. No hemolysis is observed in the HM-NPs at all test concentrations (*n* = 3, biologically independent duplicates).
FIG. 46 shows representative microscopic images of H&E staining sections of main organs (i.e. heart, liver, spleen, lungs, and kidneys) of the mice in each group at an end point of a tumor regression experiment of a 4T1-Luc model. The experiment is performed 3 times with 3 biological duplicates in each group. Scale bar, 50 µm.

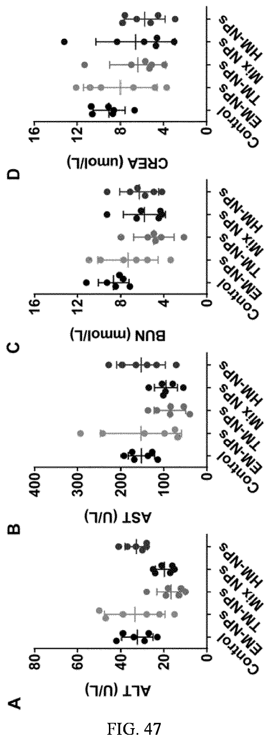

FIGs. 47A-47D show results of evaluating liver and kidney functions of all groups of the mice at the end point of the tumor regression experiment of the 4T1-Luc model. (A to D) In the 4T1-Luc model (*n* = 6 mice in each group), biochemical analysis of serums from the mice at the end of the tumor regression experiment. ALT, alanine aminotransferase; AST, aspartate transaminase; BUN, blood urea nitrogen; and CREA, creatinine. The data are expressed as mean ± standard deviation. The statistical significance is calculated by using a Bonferroni multiple comparison test through an one-way analysis of variance.

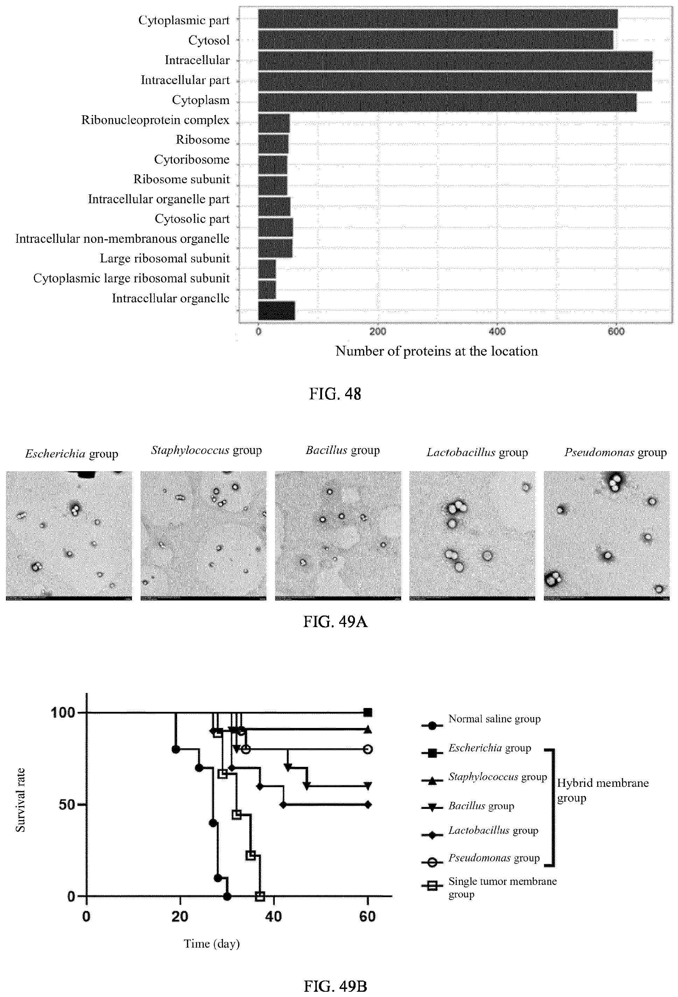

FIG. 48 shows distribution of proteins in an inner membrane of *Escherichia coli.*
FIGs. 49A-49B show results of inhibiting recurrence of a colon cancer model after a surgery by preparing HM-NPs hybrid membrane nano-vaccines from inner membranes of bacteria of the same genus. (A) Morphologies of hybrid membrane nanoparticles prepared by different bacterial inner membranes and tumor membranes. (B) Survival curves of the mice after immunotherapy with the hybrid membrane nanoparticles prepared by the different bacteria.

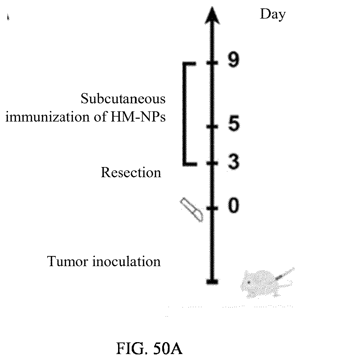

FIGs. 50A-50C show results of inhibiting recurrence of corresponding tumor models after a surgery by HM-NPs hybrid membrane nano-vaccines of different tumor cell membranes. (A) Schematic diagram of construction of tumor model and immune procedure. (B) Morphologies of hybrid membrane nanoparticles prepared by different tumor cell membranes and bacterial inner membranes. (C) Survival curves of the mice immunized with hybrid membrane nano-vaccines, the HM-NPs, prepared from the cell membranes of liver cancer, stomach cancer, kidney cancer, pancreatic cancer, ovarian cancer, lymphoma, osteosarcoma, glioma, prostate cancer, and melanoma.

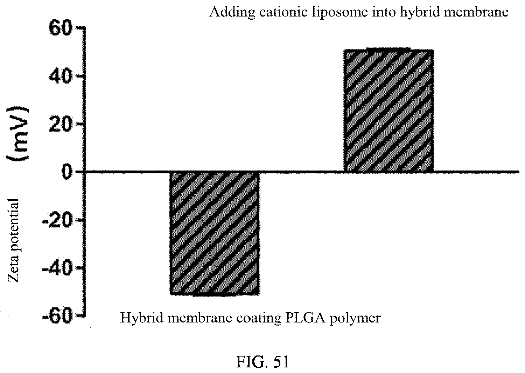

FIG. 51 shows that a hybrid membrane coating PLGA polymer is extruded through a liposome extruder with a pore diameter of 200 nm and the surface potential of the outer shell of the hybrid membrane can be changed from +50 mV to -50 mV by doping polymers with different charges.

### DETAILED DESCRIPTION

Particular examples of the present disclosure will be described below, and other advantages and effects of the present disclosure will be easily understood by a person skilled in the art from the disclosure of the description.

### Definitions of terms

In the application, the term "bacterial inner membrane" usually refers to a membrane structure of bacteria. For example, bacteria can have multiple structures, successively from the outside to the inside, a cell wall, a bacterial outer membrane, a bacterial inner membrane, and a cytoplasm. For example, the bacterial inner membrane of the present application may be simply the inner bacterial membrane structure near the cytoplasm.

In the present application, the term "pharmaceutical combination" generally refers to a product produced by mixing or combining one or more active ingredients. The term "pharmaceutical combination" may be administered to a patient separately, together or sequentially (without specific time limits), wherein such administration provides therapeutically effective levels of the first membrane component and a component derived from other organism than the bacterium of the present application in the body of the patient.

In the present application, the term "membrane component" generally refers to a component having an ingredient in a membrane structure. For example, the membrane component of the present application may include a natural or synthetic biological membrane component. For example, the membrane component of the present application may also do not include a phospholipid component. The membrane component of the present application may only include membrane proteins, membrane lipids, membrane sugar molecules, membrane glycoproteins, or membrane lipoproteins of biological membranes. For example, the membrane component of the present application may include a functionally active fragment and any other domains of the membrane component.

In the present application, the term "organism" generally refers to an organism having a boundary structure. For example, the organism of the present application may be an object having a life. For example, the organism of the present application may be a cellular organism having a cellular structure or a viral organism not having a cellular structure. For example, the organism of the present application may include a bacterium, a prokaryote, an eukaryote, and a tissue, a cell, or a non-cellular structure as described above.

In the present application, the term "immunogenicity" generally means capable of inducing an immune response (to stimulate the production of specific antibodies and/or the proliferation of specific T cells).

In the present application, the term "compatibility" generally refers to a material having compatibility with a host. For example, biocompatibility may refer to a material that does not cause an abnormal blood response, immune response, and/or tissue response.

In the present application, the term "gram-positive bacteria" or "gram-negative bacteria" generally means that the bacteria can be divided into two main categories, depending on the basic characteristics of the gram-staining reaction: G positive (G+) and G negative (G-). The former can still retain bluish purple of crystal violet after primary dyeing, and the latter can firstly remove the color of the crystal violet after the primary dyeing and is provided with complex pink or sandy yellow red.

In the present application, the term "interleukin-6" or "IL-6" refers generally to one of the cytokines. For example, IL-6 can be found in GenBank accession number P05231. The IL-6 protein of the present application may also include a functionally active fragment thereof, and is not limited to a substance, including a functionally active fragment of the IL-6, produced after processing and/or modification occurring in cells. For example, the IL-6 of the present application may include a functionally active fragment and any other domains of the IL-6.

In the present application, the term "interferon-γ" or "IFN- γ" refers generally to one of the cytokines. For example, IFN-γ can be found in GenBank accession number P01579. The IFN-γ protein of the present application may also include a functionally active fragment thereof, and is not limited to a substance, including a functionally active fragment of the IFN-γ, produced after processing and/or modification occurring in cells. For example, the IFN-γ of the present application may include a functionally active fragment and any other domains of the IFN-γ.

In the present application, the term "interleukin-1β" or "IL-1β" refers generally to one of the cytokines. For example, IL-1β can be found in GenBank accession number P01584. The IL-1β protein of the present application may also include a functionally active fragment thereof, and is not limited to a substance, including a functionally active fragment of the IL-1β, produced after processing and/or modification occurring in cells. For example, the IL-1β of the present application may include a functionally active fragment and any other domains of the IL-1β.

In the present application, the term "tumor necrosis factor-α" or "TNF-α" refers generally to one of the cytokines. For example, TNF-α can be found in GenBank accession number P01375. The TNF-α protein of the present application may also include a functionally active fragment thereof, and is not limited to a substance, including a functionally active fragment of the TNF-α, produced after processing and/or modification occurring in cells. For example, the TNF-α of the present application may include a functionally active fragment and any other domains of the TNF-α.

In the present application, the term "lipopolysaccharide" generally refers to LPS for short. For example, LPS may be a constituent of an outer wall of a cell wall of gram-negative bacteria, and a substance (glycolipids) consisting of lipids and polysaccharides.

In the present application, the term "FtsZ" generally refers to a protein in a bacterial inner membrane. For example, FtsZ can be used to detect whether a hybrid membrane contains a membrane component derived from a bacterial inner membrane. For example, FtsZ can be found in GenBank accession number P0A9A6. For example, the FtsZ of the present application may include a functionally active fragment and any other domains of the FtsZ. The bacterial inner membrane may also include lipopolysaccharide (LPS, commonly known as endotoxin) and phosphatidylethanolamine (PE). The bacterial inner membrane may also include a minor lipid category such as N-acylated PE (acyl-PE) and O-acylated PG (acyl-PG), etc., and may be used to detect whether a hybrid membrane contains a membrane component derived from the bacterial inner membrane. The lipopolysaccharide molecules are located on an outer layer of a bacterial outer membrane and can be composed of a hydrophilic polysaccharide chain and a hydrophobic lipid A. The amphiphilic molecule phosphatidylethanolamine can be the lipid molecule with the highest content in the cell inner membrane of gram-negative bacteria, and accounts for about 77%; anionic lipid phosphatidylglycerol (PG) accounts for about 13%; and cardiolipin (CL) accounts for about 10%. The bacterial inner membrane is rich in lipoproteins and lipids. The phosphatidylethanolamine may be the most important phospholipid molecule of gram-negative bacteria, and plays a very important role in maintaining the normal morphology of cells, the growth and differentiation of cells, and the synthesis of substances. The bacterial membrane can have a diversity of amphipathic lipids, including the common phosphatidylglycerol, phosphatidylethanolamine and cardiolipin, the less common phosphatidylcholine, phosphatidylinositol and a variety of other membrane lipids, such as ornithine lipid, glycolipid, sphingolipid, etc.

In the present application, the term "Na+/K+-ATPase" generally refers to a protein of the cell membrane. For example, it can be a protein of a mammalian cell membrane. For example, Na+/K+-ATPase can be used to detect whether a hybrid membrane contains a membrane component derived from other sources than a bacterial inner membrane. For example, Na+/K+-ATPase can be found in GenBank accession number P13637. For example, the Na+/K+-ATPase of the present application can contain a functionally active fragment and any other domains of the Na+/K+-ATPase.

### Detailed description of the present disclosure

In one aspect, the present application provides a pharmaceutical combination, wherein the pharmaceutical combination comprises a first membrane component and a second membrane component, the first membrane component comprises a membrane derived from an inner membrane of a bacterium, the second membrane component comprises a membrane derived from a tumor cell, and the first membrane component and the second membrane component are fused to formed an outer shell.

For example, the tumor cell is a solid tumor cell.

For example, the tumor cell is selected from the following group consisting of: a breast cancer cell, a colon cancer cell, a liver cancer cell, a stomach cancer cell, a kidney cancer cell, a pancreatic cancer cell, an ovarian cancer cell, a lymphoma cell, an osteosarcoma cell, a glioma cell, a prostate cancer celland a melanoma cell.

For example, the second membrane component may include a component with immunogenicity. For example, the second membrane component may trigger an immune response to the organism.

For example, the second membrane component may further include a protein or a functionally active fragment thereof selected from the following group consisting of: an antigen peptide transporter 1, an H-2 class II histocompatibility antigen γ chain, a protein tyrosine kinase SYK, a high affinity immunoglobulin epsilon receptor subunit γ, a Ras-related C3 botulinum toxin substrate 2, a protein tyrosine kinase BTK, a protein tyrosine phosphatase receptor type C, a Na+/K+-ATPase, ATP5A (IM CVa), a ubiquinol-cytochrome C reductase core protein I (IM Core I), a VDAC 1/porin (OM Porin), a matrix cyclophilin D (Matrix CypD), an intermembrane space cytochrome C (IMS Cytc), a basement membrane-specific heparan sulfate proteoglycan core protein (HSPG2), a plasma membrane calcium-transporting ATPase 1 (ATP2b1), an endoplasmic reticulum membrane protein complex subunit 1 (EMC1), a transmembrane protein 43 (TMEM43), a vesicular integral-membrane protein VIP36 (LMAN2), a vesicle-associated membrane protein-associated protein A (VAPA), a transmembrane 9 superfamily member 2 (TM9SF2), a transmembrane emp24 domain-containing protein 10 (TMED10), an adipocyte plasma membrane associated protein (APAMP), a synaptic vesicle membrane protein VAT (VAT1), a nuclear pore membrane glycoprotein 210 (NUP210), a plasma membrane calcium-transporting ATPase 4 (ATP2b4), a plasma membrane calcium-transporting ATPase 2 (ATP2b2), an erythrocyte band 7 integral membrane protein (STOM), a transmembrane emp24 domain-containing protein 9 (TMED9), a mitochondrial import inner membrane translocase subunit TIM44 (TIMM44), a progesterone receptor membrane component 1 (PGRMC1), an endoplasmic reticulum membrane protein complex subunit 3 (EMC3), a vesicle-associated membrane-protein-associated protein B (VAPB), a membrane primary amine oxidase (AOC3), a vesicle-associated membrane protein 7 (VAMP7), a Golgi membrane protein 4 (GOLIM4), a progesterone receptor membrane component 2 (PGRMC2), a transmembrane 9 superfamily member 3 (TM9SF3), a transmembrane 9 superfamily member 4 (TM9SF4), an endoplasmic reticulum membrane protein complex subunit 2 (EMC2), a mitochondrial import inner membrane translocase subunit TIM50 (TIMM50), a peroxisomal membrane protein 11B (PEX11b), a transmembrane emp24 domain-containing protein 2 (TMED2), a secretory carrier membrane protein 3 (SCAMP3), a thioredoxin-related transmembrane protein 4 (TMX4), a peroxisomal membrane protein PMP34 (SLC25a17), a peroxisomal membrane protein PEX14 (PEX14), an endoplasmic reticulum membrane protein complex subunit 8 (EMC8), an interferon-induced transmembrane protein 3 (IFITM3), a lysosome-associated membrane glycoprotein 2 (LAMP2), a thioredoxin-related transmembrane protein 2 (TMX2), a vesicle-associated membrane protein 3 (VAMP3), a lysosome-associated membrane glycoprotein 1 (LAMP1), a mitochondrial import inner membrane translocase subunit TIM8 A (TIMM8a1), a lysosomal integral membrane protein-2 (SCARB2), an Ig gamma-2A chain C region (IGHG2a), a transmembrane 9 superfamily member 1 (TM9SF1), an inner nuclear membrane protein Man1 (LEMD3), a transmembrane emp24 domain-containing protein 4 (TMED4), a Thy-1 membrane glycoprotein (Thy1), a mitochondrial import inner membrane translocase subunit TIM23 (TIMM23), a mitochondrial import inner membrane translocase subunit TIM9 (TIMM9), a mitochondrial import inner membrane translocase subunit TIM13 (TIMM13), a secretory carrier membrane protein 2 (SCAMP2), a secretory carrier membrane protein 1 (SCAMP1), an integrin membrane protein 2B (ITM2b), a mitochondrial import inner membrane translocase subunit TIM10 (TIMM10), a vacuole membrane protein 1 (VMP1), a growth hormone-inducible transmembrane protein (GHITM), a membrane protein with a death domain (NRADD), a vesicle-associated membrane protein 8 (VAMP8), a transmembrane anterior posterior transformation 1 (TAPT1), a mitochondrial intermembrane space import and assembly protein 40 (CHCHD4), a glycosylated lysosomal membrane protein (GLMP), a membrane-spanning 4-domains, subfamily A, member 6D (MS4A6D), a translocation associated membrane protein 1 (TRAM1), an interferon-induced transmembrane protein 2 (IFITM2), a sarcolemma associated protein (SLMAP), a membrane magnesium transporter 1 (MMGT1), a nuclear envelope pore membrane protein POM 121 (POM121), a transmembrane protein C16orf54 homolog (AI467606), a vacuolar ATPase assembly integral membrane protein VMA21 (VMA21), an epithelial membrane protein 1 (EMP1), a membrane-associated phosphatidylinositol transfer protein 1 (PITPNM1), a small integral membrane protein 15 (SMIM15), a nuclear envelope integral membrane protein 1 (NEMP1), an integral membrane protein GPR180 (GPR180), a secretory carrier membrane protein 4 (SCAMP4), a translocation associated membrane protein 2 (TRAM2), a transmembrane and coiled-coil domain family 3 (TMCC3), a stromal membrane-associated protein 1 (SMAP1), a neuronal membrane glycoprotein M6-b (GPM6b), an epithelial membrane protein 2 (EMP2), a Golgi membrane protein 1 (GOLM1), an SID1 transmembrane family member 2 (SIDT2), a trans-Golgi network integral membrane protein 2 (TGOLN2), a Golgi apparatus membrane protein TVP23 homolog B (FAM18b), a mitochondrial inner membrane protein OXA1L (OXA1L), an osteopetrosis-associated transmembrane protein 1 (OSTM1), a peroxisomal membrane protein PEX13 (PEX13), a single-pass membrane and coiled-coil domain-containing protein 1 (SMCO1), a distal membrane arm assembly complex 1 (DMAC1), a secretory carrier membrane protein 5 (SCAMP5), a cystic fibrosis transmembrane conductance regulator (CFTR), an osteoclast stimulatory transmembrane protein (OCSTAMP), a fat storage-inducing transmembrane protein 2 (FITM2), and a transmembrane channel-like protein 5 (TMC5), and any combination of the above.

For example, the pharmaceutical combination of the present application, wherein the bacterium may include a gram-negative bacterium and/or a gram-positive bacterium.

For example, the pharmaceutical combination of the present application, wherein the bacterium is selected from the following group of genera consisting of: *Escherichia, Staphylococcus, Bacillus, Lactobacillus, Klebsiella, Brucella, Proteus, Acinetobacter,* and *Pseudomonas,* and any combination of the above. For example, the pharmaceutical combination of the present application, wherein the bacterium is selected from the following group consisting of: *Escherichia, Staphylococcus, Bacillus, Lactobacillus,* and *Pseudomonas.* For example, the pharmaceutical combination of the present application, wherein the bacterium may include *Escherichia* and/or *Staphylococcus.*

For example, the pharmaceutical combination of the present application, wherein the inner membrane of the bacterium may include a protein or a functionally active fragment thereof selected from the following group consisting of: a cell division protein FtsZ, an inner membrane protein YhcB, an inner membrane protein transposase YidC, a cell division protein NlpI, an ABC transporter MsbA, an inner membrane transporter TatA, an intermembrane phospholipid transport system lipoprotein MlaA, an inner membrane protein TolQ, an intermembrane transport lipoprotein PqiC, an outer membrane protein TolC, an outer membrane usher protein FimD, an outer membrane porin OmpC, a transmembrane transport protein PqiB, a major outer membrane lipoprotein Lpp, a membrane-bound lytic murein transglycosylase MltB, a UPF0194 membrane protein YbhG, a putative membrane protein IgaA homolog, an intermembrane phospholipid transport system lipoprotein MlaA, an inner membrane protein YejM, a membrane-bound lytic murein transglycosylase MltA, an intermembrane phospholipid transport system binding protein MlaC, an inner membrane protein YlaC, an intermembrane transport protein YebT, a membrane-bound lytic murein transglycosylase MltC, an intermembrane phospholipid transport system ATP-binding protein MlaF, an intermembrane phospholipid transport system binding protein MlaD, an inner membrane protein YqjE, a UPF0053 inner membrane protein YfjD, a UPF0053 inner membrane protein YoaE, an inner membrane lysis murein transglycosylase A, a UPF0394 inner membrane protein YedE, an intermembrane phospholipid transport system binding protein MlaB, an inner membrane protein YccF, an intermembrane phospholipid transport system permease protein MlaE, an inner membrane protein YedI, an inner membrane protein YgaP, a transmembrane transport protein PqiA, a UPF0056 membrane protein YhcE, an inner membrane protein YbhL, an inner membrane protein YhjD, an inner membrane transporter YbaT, an inner membrane protein YjjP, an inner membrane protein YhaH, an inner membrane protein YbjJ, an inner membrane transporter YqeG, a UPF0053 inner membrane protein YtfL, an arsenical pump membrane protein ArsB, an inner membrane protein YpjD, a membrane-bound lysozyme inhibitor of C-type lysozyme MliC, a UPF0283 membrane protein YcjF, a UPF0259 membrane protein YciC, an inner membrane protein YgfX, an inner membrane protein YbbJ, a lipoprotein-releasing system transmembrane protein LolE, an inner membrane protein YjcH, a protein-export membrane protein SecG, an inner membrane protein YfdC, a UPF0324 inner membrane protein YeiH, a UPF0266 membrane protein YobD, a TVP38/TMEM64 family inner membrane protein YdjZ, a membrane-associated protein UidC, an inner membrane protein YbiR, an inner membrane protein YhiM, a membrane transporter protein YfcA, an inner membrane protein YdgK, a membrane-bound lytic murein transglycosylase F, a multidrug resistance outer membrane protein MdtP, a UPF0208 membrane protein YfbV, a peptidoglycan-associated lipoprotein, a lipoprotein YiaD, a lipoprotein YeaY, an apolipoprotein N-acyltransferase, a D-methionine-binding lipoprotein MetQ, a lipoprotein GfcD, a lipoprotein YbjP, a lipoprotein YgeR, a lipoprotein-releasing system ATP-binding protein LolD, an osmotically-inducible lipoprotein OsmE, an osmotically-inducible lipoprotein OsmB, a lipoprotein YdcL, a lipoprotein YajG, a lipoprotein NlpE, a phosphatidylglycerol:prolipoprotein diacylglyceryl transferase, a lipoprotein YghJ, a lipoprotein YedD, a lipoprotein YifL, a lipoprotein YgdI, a lipoprotein YbaY, a lipoprotein signal peptidase, a lipoprotein YceB, a lipoprotein YajI, and an inner membrane protein YebE, and any combination of the above.

For example, the pharmaceutical combination of the present application may further include an inner core. For example, the inner core of the pharmaceutical combination of the present application may be used to support the membrane component of the present application.

For example, the inner core of the pharmaceutical combination of the present application may further include a biocompatible material. For example, the inner core of the pharmaceutical combination of the present application may further include an artificially synthetic material. For example, a granular and/or non-granular material known in the art that can be covered or partially covered by the membrane of the present application may be used in the inner core of the pharmaceutical combination of the present application.

For example, the inner core of the pharmaceutical combination of the present application may further include a substance selected from the following group consisting of: a poly(lactic-co-glycolic acid) (PLGA), a metal-organic framework (MOF), polycaprolactone (PCL), polyamideamine (PAMAM), a carbon nanotube, graphene, a gold nanoparticle, a mesoporous silica nanoparticle, an iron oxide nanoparticle, a silver nanoparticle, a tungsten nanoparticle, a manganese nanoparticle, a platinum nanoparticle, a quantum dot, an alumina nanoparticle, a hydroxyapatite nanoparticle, a lipid nanoparticle (LNP), a DNA nanostructure, a nano hydrogel, a rare earth fluoride nanocrystal, and a NaYF₄ nanoparticle, and any combination of the above.

For example, the inner core of the pharmaceutical combination of the present application may further include a substance selected from the following group consisting of: an immune adjuvant, an immune checkpoint inhibitor, a nucleic acid molecule, and a chemotherapeutic agent, and any combination of the above.

For example, the inner core of the pharmaceutical combination of the present application may further include monophosphoryl lipid A (MPLA), R848, CpG, and poly (I:C), and any combination of the above.

For example, the inner core of the pharmaceutical combination of the present application may further include adriamycin, paclitaxel, docetaxel, gemcitabine, capecitabine, cyclophosphamide, fluorouracil, pemetrexed, raltitrexed, bleomycin, daunorubicin, doxorubicin, vincristine, and etoposide, and any combination of the above.

For example, the inner core of the pharmaceutical combination of the present application may further include an indoleamine 2,3-dioxygenase (IDO) inhibitor.

For example, the inner core of the pharmaceutical combination of the present application may further include a small interfering RNA (siRNA).

For example, the inner core of the pharmaceutical combination of the present application may have a diameter of about 60 nm to about 100 nm. For example, the size of the inner core of the pharmaceutical combination of the present application may basically not affect an effect of the pharmaceutical combination of the present application. For example, the inner core of the pharmaceutical combination of the present application may have a diameter of about 60 nm to about 100 nm, about 70 nm to about 100 nm, about 80 nm to about 100 nm, about 90 nm to about 100 nm, about 60 nm to about 90 nm, about 70 nm to about 90 nm, or about 80 nm to about 90 nm.

For example, the inner core of the pharmaceutical combination may have a diameter of about 86 nm. For example, the diameter of the inner core of the present application may be measured by a transmission electron microscope.

The pharmaceutical combination of the present application includes an outer shell and the outer shell includes the first membrane component and the second membrane component.

The outer shell of the present application includes a membrane fused by the first membrane component and the second membrane component. For example, an ingredient derived from a bacterial inner membrane may be used to enhance an immune response of a subject. For example, a tumor cell membrane as an ingredient derived from other organism may be used to induce and/or enhance an immune response of the subject to the tumor.

For example, the outer shell of the present application may have a thickness of about 10 nm to about 20 nm. For example, the outer shell of the present application may have a thickness of about 10 nm to about 20 nm, about 11 nm to about 20 nm, about 12 nm to about 20 nm, about 13 nm to about 20 nm, about 14 nm to about 20 nm, about 15 nm to about 20 nm, about 16 nm to about 20 nm, about 17 nm to about 20 nm, about 18 nm to about 20 nm, about 19 nm to about 20 nm, about 10 nm to about 15 nm, about 11 nm to about 15 nm, about 12 nm to about 15 nm, about 13 nm to about 15 nm, or about 14 nm to about 15 nm. For example, the outer shell of the present application may have a thickness of about 14 nm.

For example, the outer shell of the present application may have a diameter of about 100 nm. For example, the thickness or diameter of the outer shell of the present application may be measured by a transmission electron microscope.

For example, the outer shell of the present application may have a surface potential (Zeta potential) of about +50 mV to about -50 mV.

For example, the outer shell of the present application may have a surface potential (Zeta potential) of about -21 mV. For example, the outer shell may have a surface potential (Zeta potential) of about +50 mV to about -50 mV, about -15 mV to about -50 mV, about -21 mV to about -50 mV, about -25 mV to about -50 mV, about +50 mV to about -25 mV, about +50 mV to about -21 mV, about -15 mV to about -25 mV, about -17 mV to about -25 mV, about -19 mV to about -25 mV, about -21 mV to about -25 mV, about -23 mV to about -25 mV, about -15 mV to about -21 mV, about -17 mV to about -21 mV, or about -19 mV to about -21 mV.

For example, the mass ratio of the first membrane component to the second membrane component of the outer shell of the present application may be 1:100 to 100:1.

For example, the mass ratio of the first membrane component to the second membrane component of the outer shell of the present application may be 1:100 to 100:1, for example, 1:100, 1:75, 1:50, 1:25, 1:10, 1:5, 1:3, 1:1, 1:0, 0:1, 3:1, 5:1, 10:1, 25:1, 50:1, 75:1 or 100:1. For example, the mass ratio of the first membrane component to the second membrane component of the outer shell of the present application may be 1:100, 1:90, 1:80, 1:70, 1:60, 1:50, 1:40, 1:20, 1:10, 1:9, 1:8, 1:7, 1:6, 1:5, 1:4, 1:3, 1:2, 1:1, 0:1, 2:1, 3:1, 4:1, 5:1, 6:1, 7:1, 8:1, 9:1, 10:1, 20:1, 40:1, 50:1, 60:1, 70:1, 80:1, 90:1 or 100:1.

For example, the presence and/or the ratio of the first membrane component and the second membrane component in the pharmaceutical combination of the present application may be confirmed by western blot and/or immunogold staining.

For example, the pharmaceutical combination of the present application may include a particle and the particle may include the inner core and the outer shell.

For example, the mass ratio of the outer shell to the inner core material of the pharmaceutical combination of the present application may be about 1:1 to about 1:10.

For example, the mass ratio of the outer shell to the inner core material of the pharmaceutical combination of the present application may be about 1:4 to about 1:6. For example, the mass ratio of the outer shell to the inner core material of the pharmaceutical combination of the present application may be about 1:1 to about 1:10, about 1:2 to about 1:10, about 1:4 to about 1:10, about 1:6 to about 1:10, about 1:8 to about 1:10, about 1:1 to about 1:6, about 1:2 to about 1:6, or about 1:4 to about 1:6. For example, the mass ratio of the outer shell to the inner core material of the pharmaceutical combination of the present application may be about 1:1, about 1:2, about 1:3, about 1:4, about 1:5, about 1:6, about 1:7, about 1:8, about 1:9 or about 1:10.

For example, the content of lipopolysaccharide (LPS) of the pharmaceutical combination of the present application may have no significant difference compared with the content of lipopolysaccharide in a mammalian cell. For example, the pharmaceutical combination of the present application may basically do not include lipopolysaccharide from a bacterium.

For example, the particle of the pharmaceutical combination of the present application may have a diameter of about 70 nm to about 120 nm.

For example, the particle of the pharmaceutical combination of the present application may have a diameter of about 100 nm. For example, the particle of the pharmaceutical combination of the present application may have a diameter of about 70 nm to about 120 nm, about 80 nm to about 120 nm, about 90 nm to about 120 nm, about 100 nm to about 120 nm, about 110 nm to about 120 nm, about 70 nm to about 100 nm, about 80 nm to about 100 nm, or about 90 nm to about 100 nm. For example, the diameter may be measured by a transmission electron microscope (TEM).

For example, the particle may have a hydrodynamic size of about 150 nm to about 250 nm. For example, the particle may have a hydrodynamic size of about 180 nm. For example, the particle may have a hydrodynamic size of about 150 nm to about 250 nm, 160 nm to about 250 nm, 180 nm to about 250 nm, 200 nm to about 250 nm, 220 nm to about 250 nm, 240 nm to about 250 nm, about 150 nm to about 180 nm, or 160 nm to about 180 nm.

For example, the particle of the present application may have a surface potential (Zeta potential) of about +50 mV to about -50 mV. For example, the particle of the present application may have a surface potential (Zeta potential) of about -21 mV. For example, the particle may have a surface potential (Zeta potential) of about +50 mV to about -50 mV, about -15 mV to about -50 mV, about -21 mV to about -50 mV, about -25 mV to about -50 mV, about +50 mV to about -25 mV, about +50 mV to about -21 mV, about -15 mV to about -25 mV, about -17 mV to about -25 mV, about -19 mV to about -25 mV, about -21 mV to about -25 mV, about -23 mV to about -25 mV, about -15 mV to about -21 mV, about -17 mV to about -21 mV, or about -19 mV to about - 21 mV.

For example, the hydrodynamic size and/or the surface potential of the particle may be measured by a dynamic light scattering (DLS) instrument.

For example, the particle may maintain stability in a solution. For example, the stability of the particle may include that the hydrodynamic size and/or the surface potential of the particle after being stored for a period of time are not significantly different from those before the period of time.

For example, the stability of the particle may include that the hydrodynamic size and/or the surface potential of the particle after being stored in a phosphate buffer solution (PBS) at 4°C for a period of time are not significantly different from those before the period of time.

For example, the stability of the particle may include that the hydrodynamic size and/or the surface potential of the particle after being stored in a phosphate buffer solution (PBS) at 4°C for a period of time are not significantly different from those before the period of time. For example, the period of time may be not less than about 21 days. For example, the period of time may be not less than about 21 days, not less than 20 days, not less than 15 days, not less than 10 days, not less than 5 days, not less than 4 days, not less than 3 days, not less than 2 days, or not less than 1 day.

For example, the pharmaceutical combination of the present application may further include a substance selected from the following group consisting of: an immune adjuvant, an immune checkpoint inhibitor, a nucleic acid molecule, a chemotherapeutic agent, and a photosensitizer, and any combination of the above. For example, the component in the pharmaceutical combination of the present application may be administered simultaneously and/or administered separately.

For example, the pharmaceutical combination of the present application may further include monophosphoryl lipid A (MPLA). For example, the pharmaceutical combination of the present application may further include an indoleamine 2,3-dioxygenase (IDO) inhibitor. For example, the pharmaceutical combination of the present application may further include a small interfering RNA (siRNA).

In another aspect, the present application provides a vaccine and may include the pharmaceutical combination of the present application.

In another aspect, the present application provides a kit and may include the pharmaceutical combination of the present application and/or the vaccine of the present application.

In another aspect, the present application further provides a method for preparing the pharmaceutical combination of the present application, the vaccine of the present application and/or the kit of the present application, and the method may include providing the membrane derived from the bacterial inner membrane.

For example, the method of the present application may further include providing the component derived from other organism than the bacterium.

For example, the method of the present application may further include mixing the membrane derived from an inner membrane of a bacterium with the component derived from other organism than the bacterium to provide an outer shell.

For example, the method of the present application may further include providing an inner core.

A method for enhancing an uptake of a target antigen by an immune cell is described, wherein the method may include providing a pharmaceutical combination, the pharmaceutical combination may include a first membrane component, the first membrane component may include a membrane derived from a bacterial inner membrane, and the pharmaceutical combination may further include the target antigen. For example, the method of the present application may be *in vitro* or *ex vivo.* For example, the method of the present application may be for non-prophylactic, non-therapeutic and/or non-diagnostic purposes.

A method is described, wherein the pharmaceutical combination may include the pharmaceutical combination of the present application.

A method is described, wherein the immune cell may include an immune presenting cell.

A method is described, wherein the immune cell may include the following group consisting of: a dendritic cell (DC), a T lymphocyte, a macrophage, and a natural killer (NK) cell, and any combination of the above.

A method is described, wherein the immune cell may include a bone marrow-derived dendritic cell (BMDC).

A method is described, wherein the immune cell may include a CD8 positive cell and/or a CD4 positive cell.

In another aspect, the present application provides a method for activating an immune cell, wherein the method may include administering the pharmaceutical combination of the present application, the vaccine of the present application and/or the kit of the present application. For example, the method of the present application may be *in vitro* or *ex vivo.* For example, the method of the present application may be for non-prophylactic, non-therapeutic and/or non-diagnostic purposes.

For example, in the method of the present application, the immune cell may include an immune presenting cell.

For example, in the method of the present application, the immune cell may include the following group consisting of: a dendritic cell (DC), a T lymphocyte, a macrophage, and a natural killer (NK) cell, and any combination of the above.

For example, in the method of the present application, the immune cell may include a bone marrow-derived dendritic cell (BMDC).

For example, in the method of the present application, the immune cell may include a CD8 positive cell and/or a CD4 positive cell.

For example, in the method of the present application, the immune cell may include the immune cell in a lymph node and/or a spleen.

For example, in the method of the present application, compared with the immune cell not administrated with the pharmaceutical combination, the immune cell administrated with the pharmaceutical combination may include the activating effect selected from the following group consisting of: increasing an expression level of an antigen recognition receptor of the immune cell, an expression level of a nuclear factor κB (NF-κB) of the immune cell, an expression and/or a secretion level of a cytokine of the immune cell, and a ratio of the mature immune cell, and any combination of the above. For example, the increase comprises that compared with the immune cell not administrated with the pharmaceutical combination, the effect of the immune cell administrated with the pharmaceutical combination selected from the above group is improved by about 90%, about 80%, about 70%, about 60%, about 50%, about 40%, about 30%, about 20%, about 10%, about 5%, about 4%, about 3%, about 2%, or about 1%. For example, the increase comprises that compared with the immune cell not administrated with the pharmaceutical combination, the expression level of the antigen recognition receptor of the immune cell administrated with the pharmaceutical combination is improved by about 90%, about 80%, about 70%, about 60%, about 50%, about 40%, about 30%, about 20%, about 10%, about 5%, about 4%, about 3%, about 2%, or about 1%. For example, the increase comprises that compared with the immune cell not administrated with the pharmaceutical combination, the expression level of the nuclear factor κB (NF-κB) of the immune cell administrated with the pharmaceutical combination is improved by about 90%, about 80%, about 70%, about 60%, about 50%, about 40%, about 30%, about 20%, about 10%, about 5%, about 4%, about 3%, about 2%, or about 1%. For example, the increase comprises that compared with the immune cell not administrated with the pharmaceutical combination, the expression and/or secretion level of a cytokine of the immune cell administrated with the pharmaceutical combination is improved by about 90%, about 80%, about 70%, about 60%, about 50%, about 40%, about 30%, about 20%, about 10%, about 5%, about 4%, about 3%, about 2%, or about 1%. For example, the increase comprises that compared with the immune cell not administrated with the pharmaceutical combination, the ratio of the mature immune cell administrated with the pharmaceutical combination is improved by about 90%, about 80%, about 70%, about 60%, about 50%, about 40%, about 30%, about 20%, about 10%, about 5%, about 4%, about 3%, about 2%, or about 1%.

For example, in the method of the present application, the antigen recognition receptor may include a pattern recognition receptor (PRR).

For example, in the method of the present application, the antigen recognition receptor may include a Toll-like receptor (TLR).

For example, in the method of the present application, the antigen recognition receptor may include TLR1, TLR2 and/or TLR6.

For example, in the method of the present application, compared with the immune cell not administrated with the pharmaceutical combination, the immune cell administrated with the pharmaceutical combination may have a basically unchanged expression of TLR4.

For example, in the method of the present application, the cytokine may include a proinflammatory cytokine.

For example, in the method of the present application, the cytokine may include interleukin (IL)-6, tumor necrosis factor (TNF)-α, IL-1β and/or interferon (IFN)-γ.

For example, in the method of the present application, the mature immune cell may include a CD80 positive cell, a CD86 positive cell and/or an effector memory cell.

For example, in the method of the present application, a ratio of the mature immune cell may include a ratio of the CD80 positive and/or CD86 positive immune cell accounting for the CD11c positive immune cell.

For example, in the method of the present application, a ratio of the mature immune cell may include a ratio of the immune cell with a high expression of CD44 and a low expression of CD62L accounting for the CD8 positive immune cell.

In another aspect, the present application provides a method for enhancing an innate immunity and/or a specific immune response, and may include administrating a subject in need with the pharmaceutical combination of the present application, the vaccine of the present application and/or the kit of the present application.

For example, in the method of the present application, compared with no administration with the pharmaceutical combination, administration with the pharmaceutical combination may promote maturation of a dendritic cell and/or may improve cytokine secretion of a lymphocyte.

For example, in the method of the present application, the use may basically do not induce a systemic inflammatory response.

For example, in the method of the present application, the use basically does not induce a systemic inflammatory response, which may include compared with no administration with the pharmaceutical combination, administration with the pharmaceutical combination basically does not increase concentrations of IFN-γ, TNF-α, macrophage chemoattractant protein-1 (MCP-1), IL-12p70, IL-10, IL-23, IL-27, IL-17A, IFN-β, a granulocyte-macrophage colony-stimulating factor (GM-CSF) and/or IL-1α in serum of a subject.

For example, in the method of the present application, the use may basically do not induce a hemolytic reaction, a heart injury, a liver injury, a spleen injury, a lung injury and/or a kidney injury.

In another aspect, the present application provides use of the pharmaceutical combination of the present application, the vaccine of the present application and/or the kit of the present application may for enhancing an innate immunity and/or a specific immune response.

For example, in the use of the present application, compared with no administration with the pharmaceutical combination, administration with the pharmaceutical combination may promote maturation of a dendritic cell and/or may improve cytokine secretion of a lymphocyte.

For example, in the use of the present application, the use may basically do not induce a systemic inflammatory response.

For example, in the use of the present application, the use basically does not induce a systemic inflammatory response, which may include compared with no administration with the pharmaceutical combination, administration with the pharmaceutical combination basically does not increase concentrations of IFN-γ, TNF-α, macrophage chemoattractant protein-1 (MCP-1), IL-12p70, IL-10, IL-23, IL-27, IL-17A, IFN-β, a granulocyte-macrophage colony-stimulating factor (GM-CSF) and/or IL-1α in serum of a subject.

For example, in the use of the present application, the use may basically do not induce a hemolytic reaction, a heart injury, a liver injury, a spleen injury, a lung injury and/or a kidney injury.

In another aspect, the present application provides use of the pharmaceutical combination of the present application, the vaccine of the present application and/or the kit of the present application in preparing a medicine, wherein the medicine may be used for enhancing an innate immunity and/or a specific immune response.

For example, in the use of the present application, compared with no administration with the pharmaceutical combination, administration with the pharmaceutical combination may promote maturation of a dendritic cell and/or may improve cytokine secretion of a lymphocyte.

For example, in the use of the present application, the use may basically do not induce a systemic inflammatory response.

For example, in the use of the present application, the use basically does not induce a systemic inflammatory response, which may include compared with no administration with the pharmaceutical combination, administration with the pharmaceutical combination basically does not increase concentrations of IFN-γ, TNF-α, macrophage chemoattractant protein-1 (MCP-1), IL-12p70, IL-10, IL-23, IL-27, IL-17A, IFN-β, a granulocyte-macrophage colony-stimulating factor (GM-CSF) and/or IL-1α in serum of a subject.

For example, in the use of the present application, the use may basically do not induce a hemolytic reaction, a heart injury, a liver injury, a spleen injury, a lung injury and/or a kidney injury.

In another aspect, the present application further provides a method for preventing and/or treating a tumor, may including administrating a subject in need with the pharmaceutical combination of the present application, the vaccine of the present application and/or the kit of the present application.

For example, in the method of the present application, the prevention and/or treatment of a tumor may include slowing down a rate of volume increase of the tumor and/or reducing a volume of the tumor.

For example, in the method of the present application, the tumor may include the tumor not completely resected after tumor resection. For example, after surgical resection of a tumor of a patient, an incompletely-resected tumor cell and/or tumor tissue may further develop into a tumor again, and such tumor may further be prevented and/or treated by the method of the present application. For example, in the method of the present application, the tumor may include a tumor generated again after the tumor is cleaned. For example, after surgical resection of a tumor of a patient, even if the tumor is completely resected, the patient may produce a tumor again. For example, the patient is easy to produce a tumor, and such new tumor produced again may further be prevented and/or treated by the method of the present application. For example, such new tumor produced again has at least one same antigen as the original tumor.

For example, in the method of the present application, the tumor may include a solid tumor.

For example, in the method of the present application, the tumor may be selected from the following group consisting of: a breast tumor, a colon tumor, a liver tumor, a stomach tumor, a kidney tumor, a pancreatic tumor, an ovarian tumor, lymphoma, osteosarcoma, glioma, prostate cancer, and melanoma, and any combination of the above.

In another aspect, the present application further provides use of the pharmaceutical combination of the present application, the vaccine of the present application and/or the kit of the present application may for preventing and/or treating a tumor.

For example, in the use of the present application, the prevention and/or treatment of a tumor may include slowing down a rate of volume increase of the tumor and/or reducing a volume of the tumor.

For example, in the use of the present application, the tumor may include the tumor not completely resected after tumor resection.

For example, in the use of the present application, inhibiting a tumor may include a tumor generated again after the tumor is cleaned.

For example, in the use of the present application, the tumor may include a solid tumor.

For example, in the use of the present application, the tumor may be selected from the following group consisting of: a breast tumor, a colon tumor, a liver tumor, a stomach tumor, a kidney tumor, a pancreatic tumor, an ovarian tumor, lymphoma, osteosarcoma, glioma, prostate cancer, and melanoma, and any combination of the above.

In another aspect, the present application further provides use of the pharmaceutical combination of the present application, the vaccine of the present application and/or the kit of the present application in preparing a medicine, wherein the medicine may be used for preventing and/or treating a tumor.

For example, in the use of the present application, the prevention and/or treatment of a tumor may include slowing down a rate of volume increase of the tumor and/or reducing a volume of the tumor.

For example, in the use of the present application, the tumor may include the tumor not completely resected after tumor resection.

For example, in the use of the present application, inhibiting a tumor may include a tumor generated again after the tumor is cleaned.

For example, in the use of the present application, the tumor may include a solid tumor.

For example, in the use of the present application, the tumor may be selected from the following group consisting of: a breast tumor, a colon tumor, a liver tumor, a stomach tumor, a kidney tumor, a pancreatic tumor, an ovarian tumor, lymphoma, osteosarcoma, glioma, prostate cancer, and melanoma, and any combination of the above.

### Examples

An Elisa kit involved in the following examples is purchased from Abcam, USA; CD80 and CD86 antibodies are purchased from BioLegend, USA; an ELISPOT kit is purchased from Beijing Dakewe Biotechnology Co., Ltd; and a ProcartaPlex multiplex immunoassay kit is purchased from Thermo Fisher Scientific, USA.

Test animal mice involved in the following examples are BALB/c, 6-8 weeks old, 16-18 g in weight, and female. The mice are purchased from Beijing Vital River Laboratory Animal Technology Co., Ltd., and raised in a pathogen-free animal room of the National Center for Nanoscience and Technology. All animal experiments are conducted in strict accordance with the guidelines approved by the Institutional Animal Care and Use Committee (IACUC).

In the following examples, an inner membrane of *Escherichia coli* (*E.coli DH5α*) is used as a model of a gram-negative bacterial inner membrane, a cell membrane of a cell in a 4T1-luciferase triple-negative breast cancer tissue derived from surgical resection or a cell membrane of a cell in a CT26 colon cancer tissue is used as a research model of a solid tumor cell membrane derived from surgical resection, and PLGA is used as a research model of an inner core material.

The pharmaceutical combination of the present application uses a mixed *E.coli* cytoplasmic membrane (EM) and an autologous tumor membrane (TM) from a resected tumor tissue to generate a hybrid membrane nanoparticle vaccine (HM-NPs) to deliver an antigen and an adjuvant to an antigen-presenting cell (APC). The personalized tumor vaccine is specially designed to safely enhance an innate immune response and maximize an anti-tumor effect while avoiding side effects. In the current study, the present application demonstrates that the presence of the EM can significantly enhance uptake (p < 0.0001) of tumor antigens by DC, activate a Toll-like receptor (TLR) on a plasma membrane, and promote the maturation of the TLR *in vitro.* The present application demonstrates upregulation of stimulatory markers of the DC in lymph nodes (LNs) and subsequent activation of a tumor membrane antigen-specific T cell *in vivo.* In a prevention model of recurrence after a surgery, the HM-NPs obtain a significant anti-tumor immune effect in 4T1-Luc, B16F10, EMT-6, and CT-26 tumor models. In addition, in the CT26 colon tumor model, the HM-NPs can induce a strong tumor specific immune response, thereby prolonging the survival time of animals after a surgery and having a long-term protective effect on tumor re-challenge (up to 3 months). As the effective autologous tumor vaccine, the HM-NPs of the present application can successfully activate the innate and adaptive immune responses, thus significantly inhibit tumor recurrence, and have negligible side effects. The current research shows that the mixed membrane vaccine technology may be transformed into an effective strategy for clinical treatment of cancer after a surgery.

### Example 1

Four membrane nanoparticle systems are prepared in the example, and are (1) a nanoparticle system formed by wrapping PLGA with an inner membrane of *Escherichia coli* (hereinafter denoted by EM-NPs); (2) a nanoparticle system formed by wrapping PLGA with a cell membrane of a cell in a 4T1-luciferase triple-negative breast cancer tissue (hereinafter denoted by TM-NPs); (3) a nanoparticle system obtained by mixing products of (1) and (2) (hereinafter denoted by Mix NPs); and (4) a nanoparticle system formed by wrapping PLGA with a hybrid membrane composed of an inner membrane of *Escherichia coli* and a tumor cell membrane (hereinafter denoted by HM-NPs). The concentration equivalent of the total membrane protein in the four products is kept consistent, and the concentration equivalent of the two membrane proteins in the Mix NPs and HM-NPs are respectively kept consistent.

A method for preparing the HM-NPs included the following steps:
(1) extraction of *Escherichia coli* inner membrane EM: *Escherichia coli* was inoculated in an LB liquid culture medium for culture, cultured overnight, and amplified until the OD600 value is 1.2; a lysozyme with the final concentration of 2 mg/mL was added and co-incubated with the *Escherichia coli* at 37°C for 1 h to lyse a cell wall; the treated *Escherichia coli* was centrifuged at 4°C and 3,000 g for 5 min, and a supernatant was discarded to obtain a protoplast; an extraction buffer was added and the mixture was centrifuged in a density gradient to obtain an *Escherichia coli* inner membrane; and the *Escherichia coli* inner membrane was stored at -80°C for later use after resuspension;
(2) extraction of cell membrane TM of cell in 4T1-luciferase triple negative breast cancer tissue: each BALB/c mouse was subcutaneously inoculated with 200,000 tumor cells, and when the tumor volume reached about 300 mm³, a tumor tissue was aseptically surgically resected; the obtained tumor tissue was cut into pieces with scissors, a GBSS solution containing a collagenase IV (1.0 mg.mL⁻¹), DNase (0.1 mg.mL⁻¹) and a hyaluronidase (0.1 mg.mL⁻¹) was added, and the mixture was placed at 37°C for 15 min to digest the tumor tissue; the digested tissue was ground on a 70-µm filter screen and filtered through the filter screen with a pore diameter of 70 µm, a filtrate was centrifuged and resuspended to obtain a single cell suspension; an extraction buffer containing a mixture of mannitol, sucrose, bovine serum albumin (BSA), Tris hydrochloric acid, EGTA, and a phosphatase-protease inhibitor, the cells were ultrasonically crushed for 5 min by using a cell crusher at 30 W under an ice bath, and sequentially centrifuged at a centrifugal speed of 3,000 g, 10,000 g and 100,000 g to finally obtain a cell membrane derived from a tumor tissue; and the cell membrane was resuspended and frozen at -80°C;
(3) 3 mg of the extracted *Escherichia coli* inner membrane EM and 1 mg of the extracted surgically resected tumor cell membrane TM were mixed in PBS, the mixture was shaken in a shaker at a constant temperature of 37°C for 15 min, and the mixed membrane solution was subjected to a cumulative back-and-forth extrusion for 13 times (one back and one forth are counted as 1 time) through a liposome extruder with a pore diameter of a filter membrane of 400 nm to obtain an HM hybrid membrane microparticle;
(4) 10 mg of PLGA was dissolved in 1 mL of methylene chloride and 0.2 mL of sterile distilled water was added; the mixture was ultrasonically emulsified at 25 W on ice for 3 min; then the emulsion was mixed with 2 mL of 1% of a sodium cholate solution (surfactant) and ultrasonically emulsified at 30 W on ice for 5 min; the emulsion was dropwise added into 10 mL of 0.5% of a sodium cholate solution and stirred at a room temperature for 15 min; the emulsion was subjected a rotary evaporation at 37°C for 10 min by using a rotary evaporator; the emulsion was centrifuged at 11,000 g and a room temperature for 15 min; and the obtained product was washed twice with sterile distilled water and resuspended to obtain a PLGA polymer nanoparticle solution; and
(5) after 50 µL of 2 mg/mL of the HM hybrid membrane microparticle prepared in step (3) was mixed with 500 µL of 1 mg/mL of the PLGA polymer nanoparticle solution prepared in step (4), the mixture was subjected to a cumulative back-and-forth extrusion for 13 times through a liposome extruder with a pore diameter of a filter membrane of 200 nm to obtain an HM-NPs hybrid membrane nanoparticle.

A method for preparing the EM-NPs included the following steps:
(1) 4 mg of the prepared bacterial inner membrane EM solution was shaken in a shaker at 37°C for 10 min and subjected to a cumulative back-and-forth extrusion for 13 times (one back and one forth are counted as 1 time) through a liposome extruder with a pore diameter of a filter membrane of 400 nm;
(2) referring to step (4) in the preparation method of the HM-NPs, the PLGA nanoparticle was prepared by a double emulsification, 50 µL of 2 mg/mL of the prepared bacterial inner membrane EM was mixed with 500 µL of 1 mg/mL of the prepared PLGA polymer nanoparticle solution, and the mixture was subjected to a cumulative back-and-forth extrusion for 13 times (one back and one forth are counted as 1 time) through a liposome extruder with a pore diameter of a filter membrane of 200 nm to finally obtain EM-NPs.

A method for preparing the TM-NPs included the following steps:
(1) 4 mg of the prepared surgically resected tumor cell membrane TM solution was shaken in a shaker at 37°C for 10 min and subjected to a cumulative back-and-forth extrusion for 13 times (one back and one forth are counted as 1 time) through a liposome extruder with a pore diameter of a filter membrane of 400 nm;
(2) referring to step (4) in the preparation method of the HM-NPs, the PLGA nanoparticle was prepared by a double emulsification, 50 µL of 2 mg/mL of the prepared surgically resected tumor cell membrane TM was mixed with 500 µL of 1 mg/mL of the prepared PLGA polymer nanoparticle solution, and the mixture was subjected to a cumulative back-and-forth extrusion for 13 times (one back and one forth are counted as 1 time) through a liposome extruder with a pore diameter of a filter membrane of 200 nm to finally obtain TM-NPs.

A method for preparing the Mix NPs included the following steps:
(1) the prepared EM-NPs and TM-NPs were mixed at 37 °C, wherein the concentrations of the two membrane proteins were respectively consistent with the concentration in the HM-NPs.

The prepared EM-NPs, TM-NPs and HM-NPs hybrid membrane nanoparticles were observed by a transmission electron microscope (US FEI, Tecnai G2 20 S-TWIN, 200 KV). The result was shown in FIG. 1 that the three membrane nanoparticles were regularly round and had uniform sizes. The scale bar in the figure was 100 nm.

The prepared HM-NPs hybrid membrane nanoparticle was characterized by dynamic light scattering (Zetasizer NanoZS). The result is shown in FIG. 2 that the prepared HM-NPs hybrid membrane nanoparticle has a size mainly distributed between 100-300 nm and the size reaches a peak value at 164.2 nm (accounting for 15.8%), which indicate that the prepared hybrid membrane nanoparticle has a good size distribution and is beneficial to entering a cell to play a biological role.

### Example 2

In the present example, the effects of the four products prepared in example 1 on enhancing innate immunity and specific immunity are investigated, specifically including:

### 1. Experiment for promoting BMDCs to secrete proinflammatory cytokines:

(1) bone marrow-derived dendritic cells (BMDCs) of the BALB/c mice were extracted;
(2) the BMDCs were cultured to the 5th day and resuspended by using a 1640 complete culture medium, 40,000 cells were inoculated into each well in a 96-well plate, the EM-NPs, TM-NPs, Mix NPs and HM-NPs solutions with the final concentration of 1 mg/mL were respectively added for co-incubation, 3 parallel wells were set in each group, and the 1640 complete culture medium with the same volume was additionally added as a control (Ctrl) group;
(3) after the co-incubation at 37°C for 24 h, a supernatant of the culture medium in each group was respectively taken out; and
(4) levels of IL-6, TNF-α, and IL-1β in the supernatant were determined using an Elisa kit.

The results are shown in FIGS. 3A-3C that the HM-NPs hybrid membrane nanoparticle has the best potential to enhance the innate immunity of the body compared with the other groups.

### 2. Experiment for promoting maturation of BMDCs:

(1) bone marrow-derived dendritic cells (BMDCs) of the BALB/c mice were extracted;
(2) the BMDCs were cultured to the 5th day and resuspended by using a 1640 complete culture medium, 40,000 cells were inoculated into each well in a 96-well plate, the EM-NPs, TM-NPs, Mix NPs and HM-NPs solutions with the final concentration of 1 mg/mL were respectively added for co-incubation, 3 parallel wells were set in each group, and the 1640 complete culture medium with the same volume was additionally added as a control (Ctrl) group;
(3) after the co-incubation at 37°C for 24 h, the cells were collected and washed 3 times with PBS;
(4) the cells were stained with FITC-CD11c, PE-CD80 and PE-CD86 flow cytometry antibodies in a dark place for 20 min; and
(5) levels of CD80 and CD86 expressed on a cell surface of each group were detected by a BD-C6 flow cytometer, and 10,000 cells were collected in each group.

The result is shown in FIG. 4 that the HM-NPs hybrid membrane nanoparticle have the strongest potential to stimulate maturation of DC cells compared with the other groups.

### 3. Experiment for promoting mouse spleen lymphocytes to secrete IFN-γ:

(1) the BALB/c mice were randomly divided into 5 groups with 3 mice per group;
(2) the right back of the mice in each group were respectively inoculated subcutaneously with the EM-NPs, TM-NPs, Mix-NPs and HM-NPs vaccines (100 µg/mouse) on the 1st, 3rd and 7th day; and a PBS solution with the same volume was used as a control (Ctrl) group;
(3) spleen cells of each group of the mice were respectively ground on the 8th day and the ground cells were filtered by a 70-µm filter screen to obtain a mixed spleen lymphocyte suspension;
(4) the spleen lymphocytes of each group of the mice and a tumor cell membrane (as a tumor antigen) from the same source in the HM-NPs vaccine were co-incubated in a 5%CO₂ incubator at 37°C for 24 h;
(5) the cells in each group were lysed and the secretion amount of IFN-γ in the cells was detected using an ELISPOT (enzyme linked immunosorbent spot) kit; and
(6) the number of spots in each group was counted by using a CTL-ImmunoSpot Plate Reader software and the data were analyzed.

The results are shown in FIG. 5 and 6 that the HM-NPs hybrid membrane nanoparticle has the strongest potential to stimulate the spleen lymphocytes to secrete IFN-γ compared with the other groups.

The results of the three experiments are combined to show that the HM-NPs hybrid membrane nanoparticle has the potential to enhance innate and specific immunity.

### Example 3

In the present example, the effects of the four products prepared in example 1 on stimulating the systemic immune system of the mice to secrete inflammatory factors and chemokines are investigated, specifically including the following steps:
(1) the BALB/c mice were randomly divided into 5 groups with 5 mice per group;
(2) the right back of the mice in each group were respectively inoculated subcutaneously with the EM-NPs, TM-NPs, Mix-NPs and HM-NPs vaccines (100 µg/mouse) on the 1st, 3rd and 7th day; and normal saline with the same volume was used as a control (Ctrl) group;
(3) after the mice were subcutaneously injected with the vaccines for 12 h on the 7th day, serum of each group of the mice was respectively taken, and the secretion levels of the inflammatory factors and the chemokines were detected by a ProcartaPlex multiplex immunoassay kit; and
(4) data analysis was performed using a BD-C6 flow cytometer and data processing was performed using a ProcartaPlex Analysis software.

The results are shown in FIGs. 7A-7M that the HM-NPs hybrid membrane nanoparticle has a certain effect on the secretion of the inflammatory factors and chemokines by the systemic immune system, only obviously increases proinflammatory cytokines IL-6 and IL-1β, and basically keeps other 11 inflammatory factors and chemokines unchanged compared with the blank control group. The results indicate that the HM-NPs hybrid membrane nano-vaccine has good biological safety.

### Example 4

In the present example, the effects of the four products prepared in example 1 on enriching lymph nodes *in vivo* are investigated, specifically including the following steps:
(1) construction of each group of membrane nanoparticles entrapping a fluorescent dye IR780: a hydrophilic fluorescent dye IR780 was entrapped in PLGA by using a double emulsification, specifically: 200 µL of the hydrophilic fluorescent dye IR780 with the concentration of 1 mg/mL was added into 1 mL of a PLGA dichloromethane solution with a concentration of 10 mg/mL, and the mixture was primarily emulsified for 3 min; 1% of a surfactant sodium cholate was added, and the mixture was re-emulsified for 5 min; the emulsion was subjected to rotary evaporation for 10 min in a dark place to remove an organic solvent dichloromethane; and the treated emulsion was centrifuged at 11,000 g for 15 min and washed with tertiary water twice to obtain a PLGA nanoparticle entrapping a fluorescent dye IR780. Then the PLGA nanoparticle was mixed with each group of membrane microparticles prepared in advance. The specific steps and the feeding proportion were the same as those of example 1;
(2) the BALB/c mice were randomly divided into 4 groups with 4 mice per group;
(3) the right back of each group of the mice was subcutaneously injected with the fluorescence-labeled membrane nanoparticles of each group, lymph nodes of each group of the mice were taken after 12 h, fluorescence imaging was performed by using a small animal optical 3D living body imaging system, and analysis was performed by using a LivingImage software.

The results are shown in FIGs. 8 and 9 that compared with other groups, the HM-NPs hybrid membrane nanoparticle has the strongest fluorescence signal in the lymph nodes, which indicates that the HM-NPs hybrid membrane nanoparticle has the stronger potential to enrich lymph nodes.

### Example 5

In the present example, the biological safety of the HM-NPs hybrid membrane nanoparticle prepared in example 1 is investigated, specifically including the following steps:
(1) 1 mL of blood of the BALB/c mice was taken, dissolved in 2 mL of a PBS solution, and centrifuged at 3,000 rpm for 10 min;
(2) the centrifuged blood was washed with PBS for three times and gently pipetted until the blood supernatant was colorless;
(3) the centrifuged red blood cells were diluted with 2 mL of PBS, 0.1 mL of the blood diluent was respectively taken into 0.9 mL of samples to be detected (HM-NPs hybrid membrane nanoparticles with different concentrations, PBS as a negative control, and tertiary water as a positive control);
(4) all the samples were placed on a shaker at 150 rpm and shaken at 37°C for 3 h; and
(5) the shaken mixture was centrifuged at 12,000 rpm for 10 min and placed for photographing; and the absorbance of the supernatant at 541 nm was measured by a microplate reader to calculate the hemolysis rate.

The result is shown in FIG. 10 that no hemolysis occurs after the HM-NPs hybrid membrane nanoparticles of various concentrations are mixed with red blood cells, indicating that the tumor vaccines of the present disclosure have good biological safety.

### Example 6

In the present example, the effect of the four products prepared in example 1 on inhibiting postoperative tumor recurrence of 4T1-luciferase breast cancer-bearing mice subjected to tumor resection is investigated, and a flow chart of experimental operations of the present example is shown in FIG. 11. The specific steps were as follows:
(1) female BALB/c mice were randomly divided into 5 groups with 6 mice per group, and the right back of each mouse was subcutaneously inoculated with 200,000 4T1-luciferase breast cancer cells to construct a mouse model with breast cancer (Day 0);
(2) growth of the tumors of the mice was observed and recorded every other day, and the tumor fluorescence intensity of the tumor-bearing mice was detected by using a small animal fluorescence imaging system every other week as shown in FIG. 12;
(3) when the average tumor volume reached around 300 mm³, all the mice tumors were surgically resected and the wound was sutured (the 7th day);
(4) the cut tumor blocks were ground and crushed, the tumor tissue was digested by using a mixed solution of collagenase, DNase and hyaluronidase at 37°C, the digested cell suspension passed through a 70-µm filter screen and then centrifuged, the supernatant was removed, an extraction buffer was added, and the cells were resuspended to obtain a cell suspension; the cell suspension was ultrasonically crushed on an ice bath by using a cell crusher (35 W, 5 min); the crushed cell suspension was centrifuged (3,000 g, 5 min, 4°C); the supernatant of the previous step was centrifuged again (10,000 g, 10 min, 4°C); the centrifuged cell supernatant was added into an ultracentrifugation tube for ultracentrifugation (100,000 g, 2 h, 4°C); and after the ultracentrifugation, the supernatant was discarded, the obtained residue was resuspended with 1 mL of PBS to obtain a tumor cell membrane fragment TM derived from surgical resection, and the TM was stored at -80°C;
(5) the various membrane nanoparticles were prepared according to the method in example 1;
(6) fluorescein potassium was injected into the abdominal cavity of the mice after the surgery, small animal fluorescence imaging was performed, and the residual tumor volume of the mice after the surgery was observed and counted (the 9th day);
(7) the mice were randomly grouped again according to the result of the small animal fluorescence imaging into 5 groups in total with 6 mice per group;
(8) the mice of each group were subcutaneously injected with the EM-NPs, TM-NPs, Mix NPs, and HM-NPs (100 µg/mouse) on the 10th, 12nd and 16th day, respectively, and normal saline was used as a control (Ctrl) group; and
(9) the growth of the tumors of the mice was observed and recorded every other day, and when the tumor volume in the mice reached about 1,000 mm³, the mice were considered dead. The specific time of death of each group of the mice was recorded.

The results are shown in FIGs. 13-15 that compared with other groups, the HM-NPs hybrid membrane nanoparticle has the potential of more obviously inhibiting the tumor recurrence of the mice after the breast cancer surgery with an inhibitory rate of 83.3%.

### Example 7

In the present example, the effect of the four products prepared in example 1 on inhibiting postoperative tumor recurrence of CT26 colon cancer-bearing mice subjected to tumor resection and a tumor cell re-challenge experiment are investigated, and a flow chart of experimental operations of the present example is shown in FIG. 16. The specific steps were as follows:

### 1. Experiment for preventing postoperative recurrence of colon cancer:

(1) male BALB/c mice were randomly divided into 5 groups with 6 mice per group, and the right back of each mouse was subcutaneously inoculated with 200,000 CT26 colon cancer cells to construct a mouse model of colon cancer (Day 0);
(2) the growth of the tumors of the mice was observed and recorded every other day;
(3) when the average tumor volume reached around 300 mm³, all the mice tumors were surgically resected and the wound was sutured (the 7th day);
(4) the cut tumor blocks were ground and crushed, the tumor tissue was digested by using a mixed solution of collagenase, DNase and hyaluronidase at 37°C, the digested cell suspension passed through a 70-µm filter screen and then centrifuged, the supernatant was removed, an extraction buffer was added, and the cells were resuspended to obtain a cell suspension; the cell suspension was ultrasonically crushed on an ice bath by using a cell crusher (35 W, 5 min); the crushed cell suspension was centrifuged (3,000 g, 5 min, 4°C); the supernatant of the previous step was centrifuged again (10,000 g, 10 min, 4°C); the centrifuged cell supernatant was added into an ultracentrifugation tube for ultracentrifugation (100,000 g, 2 h, 4°C); and after the ultracentrifugation, the supernatant was discarded, the obtained residue was resuspended with 1 mL of PBS to obtain a tumor cell membrane fragment TM derived from surgical resection, and the TM was stored at -80°C;
(5) the various membrane nanoparticles were prepared according to the method in example 1;
(6) the mice were randomly grouped again into 5 groups in total with 15 mice per group;
(7) the mice of each group were subcutaneously injected with the EM-NPs, TM-NPs, Mix NPs, and HM-NPs (100 µg/mouse) on the 10th, 12nd and 16th day, respectively, and normal saline was used as a control (Ctrl) group; and
(8) the growth of the tumors of the mice was observed and recorded every other day, and when the tumor volume in the mice reached about 1,000 mm³, the mice were considered dead. The specific time of death of each group of the mice was recorded.

The results are shown in FIGs. 17-18 that compared with other groups, the HM-NPs hybrid membrane nanoparticle has the potential of more obviously inhibiting the tumor recurrence of the mice after the colon cancer surgery with an inhibitory rate of 100%.

### 2. Re-challenge test of postoperative tumor:

(1) the mice inoculated with the HM-NPs vaccine in 1 were randomly divided into 3 groups again after 60 days with 5 mice per group;
(2) the right back of one group of the mice was inoculated subcutaneously with 200,000 4T1 breast cancer cells, the right back of the other group of the mice was inoculated subcutaneously with 200,000 CT26 colon cancer cells, and the rest group of the mice was not inoculated with tumor cells (normal saline was inoculated as a control, Ctrl);
(3) the growth of the tumors of the mice was observed and recorded every other day, when the tumor volume in the mice reached about 1,000 mm³, the mice were considered dead, and the specific time of death of each group of the mice was recorded.

The result is shown in FIG. 19 that when the CT26 colon cancer cells invade the body of the mice for the second time, the mice of the group inoculated with the hybrid membrane vaccine prepared from the tumor cell membrane derived from the colon cancer tissue are capable of completely resisting the tumor. However, when the 4T1 breast cancer cells "invade" the mice, since the mice are not exposed to the 4T1 breast cancer cells before, the mice do not obtain 4T1 breast cancer cell-related tumor antigens, and thus do not have the immunity to the 4T1 breast cancer cells. IL-6, TNF-α, IL-1β, and IFN-γ in the serums of each group of the mice were determined on the 90th day. The results are shown in FIGs. 20A-20D that the proinflammatory factors (IL-6, TNF-α, and IL-1β ) secreted by the mice inoculated with the CT26 colon cancer cells for the second time are obviously higher than those secreted by the other two groups, and the secreted IFN-γ is also increased to a certain extent compared with the mice inoculated with the 4T1 breast cancer cells.

### Example 8

In the present example, the dosage ratio of the gram-negative bacterial inner membrane to the solid tumor cell membrane derived from surgical resection in the HM-NPs hybrid membrane nanoparticle provided by the present disclosure is investigated, specifically including:
(1) the hybrid membrane nanoparticles with different membrane protein concentration ratios were prepared according to the method of example 1, specifically: the single gram-negative bacterial inner membrane EM, the single solid tumor cell membrane TM derived from surgical resection, the hybrid membrane of the gram-negative bacterial inner membrane EM and the solid tumor cell membrane TM derived from surgical resection at a mass ratio of 1:100, 1:75, 1:50, 1:25, 1:10, 1:5, 1:3, 1:1, 1:0, 0:1, 3:1, 5:1, 10:1, 25:1, 50:1, 75:1, or 100:1, and PBS solution of the same volume as a control group (Control);
(2) the bone marrow-derived dendritic cells (BMDCs) of the BALB/c mice were extracted and cultured according to the method of example 2; the BMDCs were cultured to the 5th day and resuspended by using a 1640 complete culture medium, the cell suspension was uniformly divided 6 parts and inoculated into a 96-well plate with 50,000 cells per well, the hybrid membrane nanoparticles with different membrane protein ratios were added respectively for co-incubation, and 3 parallels were set in each group; and
(3) after the co-incubation at 37°C for 24 h, a supernatant of the culture medium in each group was respectively taken out; and levels of IL-6, TNF-α, and IL-1β in the supernatant were determined using an Elisa kit.

The results are shown in FIGs. 21A-21C that compared to the other groups, when the ratio of EM:TM is 3:1 in the hybrid membrane, the hybrid membrane nanoparticle has the strongest ability of stimulating the proinflammatory cytokines (IL-1β, TNF-α, and IL-6), indicating that the hybrid membrane nanoparticle has the strongest potential of stimulating the innate immunity of the body.

### Example 9

The present example prepares a membrane nanoparticle system. The nanoparticle system (hereinafter denoted by HM-NPs-2) is formed by wrapping MOF (metal organic framework) based on zirconium (Zr) by a hybrid membrane composed of an inner membrane of *Klebsiella pneumoniae* (CG43 strain) and a cell membrane of a cell in a HepG2 liver cancer tissue. The preparation method included the following steps:
(1) specific operations for extracting a *pneumobacillus* inner membrane EM were the same as example 1 and included: bacteria were cultured and amplified; cell walls were broken by adding a lysozyme and the bacteria were centrifuged at a low speed to obtain a protoplast; and an extraction buffer was added and the mixture was finally centrifuged in a density gradient manner to obtain the *pneumobacillus* inner membrane EM;
(2) specific operations for extracting a cell membrane TM of a cell in a HepG2 liver cancer tissue were the same as example 1 and included: BALB/c mice were subcutaneously inoculated with a tumor; when the tumor grew to a certain volume, the tumor was surgically resected; the tumor was cut into pieces with scissors, three enzymes were added to digest and lyse the tumor tissue, and the tumor tissue was ground with a 70-µm filter screen; an extraction buffer was added and the tumor tissue was crushed by using an ultrasonic cell crusher; and finally, a liver cancer cell membrane from the liver cancer tissue is obtained through centrifugation;
(3) 3 mg of the extracted *pneumobacillus* inner membrane EM and 1 mg of the extracted surgically resected tumor cell membrane TM (with the same concentration of membrane proteins) were mixed in PBS, the mixture was shaken in a shaker at a constant temperature of 37°C for 15 min, and the mixed membrane solution was subjected to a cumulative back-and-forth extrusion for 13 times (one back and one forth are counted as 1 time) through a liposome extruder with a pore diameter of a filter membrane of 400 nm to obtain an HM hybrid membrane microparticle;
(4) MOF was synthesized: 300 mg of ZrOCl₂.8H₂O, 100 mg of H₂TCPP, and 2.8 mg of benzoic acid (BA) were dissolved in 100 mL of a dimethylformamide (DMF) solvent, and the materials were stirred at 90°C under the protection of nitrogen for 5 h; after the mixture was cooled to 25°C, the mixture was centrifuged at 10,000 g for 30 min to collect an MOF product, the product was washed three times with DMF, the synthesized MOF was dissolved in DMF and stored at 4°C, and the MOF was washed three times with deionized water before use in the experiment; and
(5) 50 µL of 2 mg/mL of the hybrid membrane microparticle prepared in step (3) was mixed with 500 µL of 1 mg/mL of the MOF prepared in step (4), the mixture was subjected to ultrasonic treatment in a bath ultrasonic instrument (ultrasonic water bath) for 30 min, and the product was collected by centrifugation to obtain an HM-NPs-2 hybrid membrane nanoparticle.

### Example 10

The present example prepares a membrane nanoparticle system. The nanoparticle system (hereinafter denoted by HM-NPs-3) is formed by wrapping MSN (mesoporous silica) by a hybrid membrane composed of an inner membrane of *Bacterium burgeri* PBP₃₉ and a cell membrane of a cell in an SN12-PM6SN12-PM6 kidney cancer tissue. The preparation method included the following steps:
(1) specific operations for extracting a *Bacterium burgeri* inner membrane EM were the same as example 1 and included: bacteria were cultured and amplified; cell walls were broken by adding a lysozyme and the bacteria were centrifuged at a low speed to obtain a protoplast; and an extraction buffer was added and the mixture was finally centrifuged in a density gradient manner to obtain the *Bacterium burgeri* inner membrane EM;
(2) specific operations for extracting a cell membrane TM of a cell in an SN12-PM6 kidney cancer tissue were the same as example 1 and included: BALB/c mice were subcutaneously inoculated with a tumor; when the tumor grew to a certain volume, the tumor was surgically resected; the tumor was cut into pieces with scissors, three enzymes were added to digest and lyse the tumor tissue, and the tumor tissue was ground with a 70-µm filter screen; an extraction buffer was added and the tumor tissue was crushed by using an ultrasonic cell crusher; and finally, a kidney cancer cell membrane from the kidney cancer tissue is obtained through centrifugation;
(3) 3 mg of the extracted *Bacterium burgeri* inner membrane EM and 1 mg of the extracted surgically resected tumor cell membrane TM (with the same concentration of membrane proteins) were mixed in PBS, the mixture was shaken in a shaker at a constant temperature of 37°C for 15 min, and the mixed membrane solution was subjected to a cumulative back-and-forth extrusion for 13 times (one back and one forth are counted as 1 time) through a liposome extruder with a pore diameter of a filter membrane of 400 nm to obtain an HM hybrid membrane microparticle;
(4) MSN was synthesized: 1.5 mL of tetraethyl orthosilicate (TEOS) and 5 mL of acetone were used as an oil phase, and 40 mL of an aqueous solution containing 0.15 g of cetyltrimethylammonium bromide (CTAB); the mixture was stirred and cut at 10,000 rpm for 5min to form an emulsion; 50 µL of ammonia water was added to the emulsion and the emulsion was magnetically stirred at 400 rpm for 2 h to evaporate an organic solvent; a suspension was centrifuged, and the precipitate was washed three times with deionized water and freeze-dried; the tetraethyl orthosilicate (TEOS) was hydrolyzed and condensed at an oil/water interface to form a silica shell; and finally, liquid drops were removed through evaporation (volatilization) in a reaction or drying process to obtain the mesoporous silica MSN; and
(5) 50 µL of 2 mg/mL of the HM hybrid membrane microparticle prepared in step (3) was mixed with 500 µL of 1 mg/mL MSN prepared in step (4), the mixture was subjected to ultrasonic treatment in a bath ultrasonic instrument (ultrasonic water bath) for 30 min, and the product was collected by centrifugation to obtain an HM-NPs-3 hybrid membrane nanoparticle.

### Example 11

In the present example, the effects of the two products prepared in examples 9 and 10 on enhancing innate immunity and specific immunity are investigated, specifically including:

### 1. Experiment for promoting BMDCs to secrete proinflammatory cytokines:

The specific method referred to example 2.

The result shows that the HM-NPs-2 and HM-NPs-3 hybrid membrane nanoparticles promote BMDCs to secrete levels of IL-6, TNF-α, and IL-1β, which is similar to the HM-NPs, and have the potential to significantly enhance the innate immunity of the body.

### 2. Experiment for promoting maturation of BMDCs:

The specific method referred to example 2.

The result shows that the HM-NPs-2 and HM-NPs-3 hybrid membrane nanoparticles promote expressions of CD80 and CD86 on the surfaces of the BMDCs, which is similar to the HM-NPs, and have the potential to significantly stimulate the maturation of DCs.

### 3. Experiment for promoting mouse spleen lymphocytes to secrete IFN-γ:

The specific method referred to example 2.

The result shows that the HM-NPs-2 and HM-NPs-3 hybrid membrane nanoparticles have the number of spots similar to the HM-NPs, and have the potential to significantly stimulate the spleen lymphocytes to secrete IFN-γ.

The results of the three experiments are combined to show that the HM-NPs-2 and HM-NPs-3 hybrid membrane nanoparticles have the potential to significantly enhance innate and specific immunity.

### Example 12

### Study protocol

An *Escherichia coli* inner membrane and a tumor cell membrane of a resected autologous tumor tissue were separated and four batches of EM and TM samples were subjected to whole proteomic analysis using liquid chromatography-mass spectrometry (LC-MS). A PLGA polymer nanoparticle (NP) were prepared using a double emulsion method. Bone marrow-derived dendritic cells (BMDCs) were generated by induced differentiation from mouse bone marrow mesenchymal stem cells freshly extracted from 6-8 week-old female C57BL/6J mice. The application studies an innate immune stimulation response and a specific immune stimulation response of different membrane nanoparticles *in vivo* and *in vitro,* and characterization of physicochemical properties and proteomics of the different membrane nanoparticles. In an animal experiment, a tumor volume was measured using an electronic vernier caliper. When the volume of the tumor reaches about 300 mm³, it was surgically resected. Then the mice were immunized with different vaccine preparations on the 3rd, 5th, and 9th day after the surgery. In a CT-26 tumor re-challenge model, the mice from the HM-NP vaccinated group were randomized into three groups and inoculated with CT-26 colon adenocarcinoma cells or 4T1 breast cancer cells 60 days later. In an immune cell depletion model, one day before the start of the HM-NPs treatment, macrophages (CSF1R antibody), NK cells (ASGM1 antibody), CD8⁺ T cells (CD8 antibody) or CD4⁺ T cells (CD4 antibody) were depleted by intraperitoneal injection of depleting antibodies targeting designated surface markers. All the mice, except in the untreated group, were inoculated with the HM-NPs on the 3rd, 5th, and 9th day after the surgery. In order to evaluate *in-vivo* biosafety, at an end point of a tumor regression experiment of the 4T1-Luc model, major organs of each group of the mice were subjected to hematoxylin-eosin (H&E) staining and sectioning, and liver and kidney function indicators in serums of each group of the mice were detected.

### Detection of BMDC uptake of HM-NPs in vitro

A fluorescently labeled nanoparticle was prepared by adding rhodamine B to a PLGA core during synthesis. The BMDCs were generated from the fresh bone marrow mesenchymal stem cells of the C57BL/6J mice by induced differentiation. The cells were cultured in a RPMI 1640 medium containing 10% FBS and penicillin-streptomycin. The cells were added to 1.5-mL centrifuge tubes (40,000 cells/tube), the different vaccine preparations at a final concentration of 0.4 mg.mL⁻¹ were added, and the cells were co-cultured at 37°C for 8 h. The cells were centrifuged at 500×g for 3 min, collected, and resuspended in an FACS buffer (RPMI 1640 medium supplemented with 2% FBS). A fluorescence signal was assessed using a BD Accuri C6 FACS flow cytometer (BD Biosciences, San Jose, USA) and analyzed using a BD Accuri C6 software.

### Confocal microscopy of HM-NPs

To evaluate an internalization process into dendritic cells, Cy5.5-labeled membrane-coated NPs and BMDCs with fluorescently labeled lysosomes were prepared. The EM-NPs, TM-NPs, and HM-NPs were co-incubated with the BMDCs, and imaged under a confocal laser scanning microscope (Zeiss 710, Zeiss Microsystems, Germany). To further compare the co-localization efficiency of the HM-NPs and Mix NPs, the BMDCs were treated with the HM-NPs or Mix NPs and imaged under a confocal laser scanning microscope. The EM-NPs were labeled with a bacterial inner membrane specific primary anti-FtsZ antibody and an anti-rabbit IgG goat secondary antibody (Alexa Fluor @ 633 goat, Abcam, Cambridge, UK). The TM-NPs were labeled with a tumor cell membrane specific primary anti-Na⁺/K⁺-ATPase antibody and an anti-mouse IgG goat secondary antibody (Alexa Fluor @ 488 goat, Abcam, Cambridge, UK).

### Innate immunity experiment in vitro

To investigate ability of different membrane NPs to stimulate an innate immune response *in vitro,* the BMDCs were added to a 96-well plate (40,000 cells/well) and co-incubated with different nanopreparations at a final concentration of 0.4 mg.mL⁻¹ at 37°C for 24 h. A supernatant was collected and concentrations of IL-6, TNF-α, and IL-1β were analyzed by using corresponding specific ELISA kits.

### In-vitro and in-vivo DC maturation experiment

To investigate ability of different membrane NPs to promote BMDC maturation *in vitro,* the BMDCs were added to 1.5 mL centrifuge tubes (40,000 cells/tube) and co-incubated with different nanopreparations at a final concentration of 0.4 mg.mL⁻¹ at 37°C for 24 h. The cells were centrifuged, collected, and resuspended in an FACS buffer. FITC-CD1 1c, PE-CD80, and PE-CD86 were used for fluorescent labeling and analyzed by using a BD Accuri C6 FACS flow cytometer and a BD Accuri C6 software. To assess DC maturation *in vivo,* the female BALB/c mice were inoculated subcutaneously with 4T1 breast cancer cells. When the volume of the tumor reaches about 300 mm³, it was surgically resected. Then the mice were randomly divided into five groups after the surgery (n = 5 mice in each group). The mice were immunized with different vaccine preparations on the 3rd, 5th, and 9th day after the surgery.

### Measurement of T cell response

To examine whether the HM-NPs could efficiently activate T cells, the female BALB/c mice were subcutaneously inoculated with the 4T1 breast cancer cells. The surgical procedure and inoculation schedule were the same as the study protocol. Splenocytes were collected 12 h after the last vaccination and co-incubated with a 4T1 cell membrane (as an antigen) for 24 h or more. An ELISPOT kit pre-coated with an IFN-γ capture antibody was used to assess release of IFN- γ by the T cells induced by different membrane NPs. Plates were scanned using a CTL-ImmunoSpot plate reader and data was analyzed using a CTL ImmunoSpot software.

### Determination of serum cytokines

To assess a degree of a systemic inflammatory response after treatment with different vaccine preparations, the female BALB/c mice were inoculated subcutaneously with the 4T1 breast cancer cells. Surgical and vaccination procedures were as described above. According to the manufacturer's instructions, the serum inflammatory cytokines and chemokines were detected with ELISA kits based on Luminex magnetic beads. The amounts of capturing magnetic beads and detecting antibodies were 1/5 of the recommended amounts.

### Tumor regression experiment

The female BALB/c or C57BL/6 mice were subcutaneously inoculated with 4T1-Luc, CT-26, B16F10 or EMT-6 tumor cells (2×10⁵ cells per mouse). Surgical and vaccination procedures were as described above. IVIS (Spectrum CT, Perkin Elmer, UK) was used to monitor a bioluminescent signal generated by the 4T1-Luc cells. The volume of all the tumors was measured with an electronic caliper every other day. The tumor volume was calculated according to the following formula:
$V = \left(L\times W\times W\right) / 2$(L, longest size; and W, shortest size)

### CT-26 tumor and re-challenge models

To establish a CT-26 tumor re-challenge model, the post-operative mice treated with the HM-NPs were randomly divided into three groups 60 days later and inoculated with CT-26 colon adenocarcinoma cells or 4T1 breast tumor cells, respectively (n = 12). The volume of all the tumors was measured with an electronic caliper every other day. The secretion of the cytokines in the serums of the re-challenged mice was detected with specific ELISA kits.

### Depletion detection of in-vivo macrophages, NK cells, CD8⁺ T cells or CD4⁺ T cells

The female BALB/c mice are subcutaneously inoculated with CT-26 colon adenocarcinoma cells (2×10⁵ cells per mouse). The surgical procedure and inoculation schedule were the same as described above. Intraperitoneal injection of antibodies depletes an immune cell subset, the day before the start of the HM-NP vaccine treatment. The types of immune cell-depleting antibodies and the mode of use were as follows: macrophages with anti-mouse CSF1R (CD115, BioXCell; 300 µg injected every other day), NK cells with anti-ASGM1 (anti-mouse Asialo-GM1, BioLegend; 50 µL/injection twice weekly), CD8⁺ T cells with anti-mouse CD8 (Lyt 2.1, BioXCell; 400 µg/injection twice weekly), and CD4⁺ T cells with anti-mouse CD4 (twice weekly, GK1.5, BioXCell; 200 µg/injection once weekly). The volume of all the tumors was measured with an electronic caliper every other day. Depletion of the macrophages, NK cells, CD8⁺ T cells, and CD4⁺ T cells was confirmed by flow cytometry of PBMCs.

### Statistical analysis

GraphPad Prism software version 5.0 is used for statistical analysis. Differences between the two experimental groups are assessed by using a Bonferroni multiple comparison test through an one-way analysis of variance or a two-way analysis of variance Heat maps of ProcartaPlex multiple immunoassay based on Luminex are grouped and analyzed by a two-way analysis of variance and a Bonferroni multiple comparison test, A log-rank test (Mantel-Cox) is used to compare survival curves. The data are expressed as mean ± standard deviation as the legend in the figure. Statistical significance is set as follows: **p* < 0.05, ***p* < 0.01, ****p* < 0.001, ***p* < 0.0001, and *ns* means no significant difference.

### Sources of materials

Poly(lactide-co-glycolic acid)-OH (75:25, relative molecular mass [Mr] 20,000 Da) is purchased from Jinandaigan Biotechnology Company (Beijing, China). Phosphate buffered saline (PBS), DMEM, a RPMI 1640 medium, and fetal bovine serum (FBS) are purchased from Wisent Bio-Products (Montreal, Canada). A BCA protein assay kit is purchased from Thermo Fisher Scientific (Waltham, Massachusetts). A precoated ELISA kit for LPS detection is purchased from eBioscience (San Diego, California). An anti-FtsZ rabbit antibody is purchased from Agriera (Vännäs, Sweden). Mouse anti-Na⁺/K⁺-ATPase, ATP5A (IM CVa), panthenol-cytochrome C reductase core protein I (IM Core I), VDAC1/Porin (OM Porin), matrix cyclophilin D (matrix CypD), and antibody Cytc to IMS cytochrome C (IMS) are purchased from Abcam (Cambridge, UK). An anti-rabbit IgG-gold conjugate (5-nm gold nanoparticle) and an anti-mouse IgG-gold conjugate (10-nm gold nanoparticle) are purchased from Abcam (Cambridge, UK). Mouse IL-4 and GM-CSF are purchased from Beyotime Biotechnology (Shanghai, China). ELISA kits for analyzing IL-6, TNF-α, IL-1β, and IFN-γ are purchased from eBioscience (San Diego, California). Mouse antibodies to FITC-CD11c, PE-CD80, and PE-CD86 are purchased from BioLegend (San Diego, USA). A precoated ELISPOT kit for IFN-γ is purchased from Dakewei Biotechnology, Co., Ltd (Beijing, China). An ELISA kit based on Luminex beads is purchased from Thermo Fisher Scientific (Waltham, Massachusetts). D-fluorescein potassium is purchased from bide Pharmatech Co. Ltd (Shanghai, China). Depletion antibodies for macrophages (CD115, anti-mouse CSF1R), CD8⁺ T cells (Lyt 2.1, anti-mouse CD 8), and CD4⁺ T cells (GK1.5, anti-mouse CD4) are purchased from Bio X Cell company, USA. A depletion antibody for NK cells (anti-mouse Asialo-GM1) is purchased from BioLegend (San Diego, USA). Unless otherwise stated, other chemicals are purchased from Hualide Technology Co., Ltd (Beijing, China).

### Cell lines and animals

Mouse 4T1 breast cancer cells, 4T1-luciferase breast cancer cells, CT-26 colon tumor cells, EMT-6 breast cancer cells, and B16F10 melanoma cells are all purchased from the American Type Culture Collection (ATCC, Manassas, USA). The 4T1, 4T1-Luc, EMT-6 and B16F10 cells were cultured in a RPMI 1640 medium containing 10% FBS, 2.5 g.L⁻¹ of glucose, and 0.11 g.L⁻¹ of sodium pyruvate. CT-26 cells were maintained in a DMEM medium supplemented with 10% FBS. All the cells were incubated at 37°C under a 5%CO₂ condition.

Female BALB/c or C57BL/6 mice are purchased from Beijing Vital River Laboratory Animal Technology Co., Ltd. and maintained under a pathogen-free condition. All animal experiments were conducted under the guidence of approval of the Institutional Animal Care and Use Committee (IACUC) of the National Center for Nanoscience and Technology, Chinese Academy of Sciences (Beijing, China).

### Generation of bone marrow-derived dendritic cells (BMDCs)

The BMDCs were generated from BMSCs of female BALB/c mice (6 to 8 weeks old) by induced differentiation. Briefly, after the mice were euthanized, bones were collected and placed in 70% ethanol, and washed with PBS. Both distal ends of the bones were dissected and bone marrow was collected by gentle flushing with a RPMI 1640 medium. Erythrocytes in the collected cells were lysed with an ACK buffer and centrifuged at 300×g for 5 min, and the remaining cells were collected and then cultured in the RPMI 1640 medium in a 6-well plate (1×10⁶ cells/well). 10% FBS, 2 mM L-glutamine, 100 µg.mL⁻¹ penicillin, 100 µg.mL⁻¹ streptomycin, 0.05 mM β-mercaptoethanol (β-ME), 10 ng.mL⁻¹ mouse IL-4, and 20 ng.mL⁻¹ of mouse GM-CSF. A fresh medium was added on the 2nd and 4th day. On the 6th day, non-adherent cells were aspirated and incubated with a fresh medium in a 6-well plate for a further experiment.

### Isolation of Escherichia coli-derived cell plasma membrane (EM)

A cell plasma membrane was isolated from an *Escherichia coli* strain DH5α. Briefly, the cryopreserved *Escherichia coli* DH5α cells were cultured overnight in a liquid LB medium at 37°C under shaking at 200 rpm. When OD600 was 1.2, the bacteria were collected by centrifugation (3,000×g, 20 min, 4°C) and washed 3 times with PBS. The cell precipitate was resuspended in 10 mL of buffer A (1 M sucrose, 0.2 M Tris-HCl, pH 8.0). Then lysozyme was added to a final concentration of 2 mg.mL⁻¹ and the cells were incubated at 37°C for 1 h under shaking at 120 rpm. Sterile water (90 mL) supplemented with DNase (10 µg.mL⁻¹) was added and the materials were mixed gently in tubes for 20 times. Protoplasts (Spheroplasts) were collected by centrifugation at 3,000×g and 4°C for 20 min and resuspended in 10 mL of ice-cold buffer B (20 mM Tris-HCl, pH 7.2, 50 mM NaCl, 5 mM EDTA) containing 20% w/v. Sucrose was used to lyse the cells. Cell debris from the lysate was clarified by centrifugation at 10,000×g and 4°C for 30 min. A sample of the supernatant (5 mL) was placed on a discontinuous sucrose gradient (bottom to top: 10 mL at 50%, 5 mL at 46%, 10 mL at 42%, 10 mL at 36%, 5 mL at 32%, and 10 mL at 27% of a solution) and centrifuged at 113,000×g and 4°C for 2 h in a Beckman Optima XPN-100 ultracentrifuge (Brea, CA, USA) using a 70Ti rotor. Subsequently, 1.5 mL of the plasma membrane-containing fraction was extracted and diluted with ice-cold buffer B to reduce the sucrose concentration to 10% (w/v). A membrane was collected by centrifugation at 113,000×g and 4°C for 1 h, resuspended in PBS, and stored at -80°C for a subsequent experiment. The protein concentration of the final membrane product was quantified using a BCA protein assay kit. In order to analyze the protein composition of the EM, a proteomics analysis (H.Wayen Biotechnology Co., Ltd, Shanghai, China) was performed, and a total of 2,302 proteins were detected.

### Isolation of tumor cell membrane (TM) from resected tumor tissue

To obtain a cell membrane from a tumor tissue, female BALB/c mice were subcutaneously inoculated with mouse 4T1 breast cancer cells, 4T1-Luc breast cancer cells or CT-26 colon cancer cells. On the 7th day after tumor inoculation, the tumor (approximately 300 mm³) was resected and cut into small blocks, and the tumor blocks were then treated with a GBSS buffer (2 mL) containing a collagenase IV (1.0 mg.mL⁻¹), DNase (0.1 mg.mL⁻¹) and hyaluronidase (0.1 mg.mL⁻¹) to be digested at 37°C. The cells were scraped and collected by centrifugation at 1,000×g for 5 min. The precipitate was homogenized in 2 mL of a separation buffer (225 mM mannitol, 75 mM sucrose, 0.5% BSA, 0.5 mM EGTA, and 30 mM of Tris and a mixture of phosphatase and protease inhibitors) and then dispensed by ultrasonic treatment (30 W) for 3 min in an ice bath to sufficiently disrupt the cells. The cell homogenate was centrifuged at 3,000×g and 4°C for 5 min and the supernatant was collected. The supernatant was further centrifuged at 10,000×g and 4°C for 10 min and the obtained supernatant was ultracentrifuged at 100,000×g and 4°C for 2 h. Finally, the cell membrane derived from the tumor tissue was resuspended in 5 mM of a Bis-Tris buffer (pH 6.0) and stored at -80°C. The protein concentration of the membrane extract was determined using a BCA protein assay kit. In order to analyze the protein composition of the TM, a proteomics analysis (H.Wayen Biotechnology Co., Ltd, Shanghai, China) was performed, and a total of 5,353 proteins were detected.

### Generation and physicochemical properties of HM-NPs

To prepare a hybrid membrane (HM) vesicle, the mixed EM and TM were gently shaken at 37°C for 15 min using a dry bath incubator before a membrane fusion. The HM vesicle was formed by a repeated physical extrusion through a 400 nm cut-off extruder (Hamilton Company, Reno, Nevada, USA). A poly(lactide-co-glycolic acid)-OH (PLGA) nanoparticle was prepared using a double emulsion method. Briefly, the PLGA was dissolved in methylene chloride at a concentration of 10 mg.mL⁻¹. Then, 1 mL of the PLGA solution was mixed with 0.2 mL of sterile water. The mixture was emulsified by an ultrasonic treatment (25 W) in an ice bath for 3 min, then 2 mL of 1% sodium cholate was added, and the mixture was emulsified by an ultrasonic treatment (30 W) in an ice bath for 5 min. The emulsion was dropwise added into 10 mL of a 0.5% sodium cholate solution and the materials were stirred at a room temperature for 30 min. After 15 min of rotary evaporation, the emulsion was centrifuged at 10,000×g for 15 min and washed twice in sterile water to obtain PLGA NPs. To engineer HM-NPs, the PLGA NPs and HM vesicles were repeatedly co-extruded at 200:1 using a cut-off extruder (Hamilton Company) at a mass ratio of a polymer to a membrane protein of 5:1.

To examine morphologies of the nanoparticles and vesicles, 10 µ L of the sample was deposited onto a carbon-coated copper mesh and incubated for 15 min. The remaining liquid was absorbed using filter paper. The sample on a grid was negative-stained with 1% (v/v) uranyl acetate for 8 min, then air-dried, and observed using a transmission electron microscope (TEM; HT7700, HITACHI, Tokyo, Japan). A hydrodynamic particle size distribution, zeta potential, and polydispersity index (PDI) were measured using a Zetasizer Nano ZS dynamic light scattering (DLS) instrument (Malvern, UK). The EM-NPs and TM-NPs were generated by using a similar preparation method.

### Protein characterization of HM-NPs

To study the protein profile of the HM-NPs, SDS-PAGE was used and the obtained gel was stained with Coomassie brilliant blue (Solarbio, Beijing, China). All samples were prepared in a loading buffer (Invitrogen, Carlsbad, California, USA) and the equal amount of protein (10 µg) was loaded on NuPAGE Novex 12% separation gel (Invitrogen) in an MOPAGE running buffer (Invitrogen). The presence of specific protein markers for each component was identified by a western blot analysis. The proteins were decomposed and transferred to a nitrocellulose membrane (Thermo Fisher Scientific, Waltham, Massachusetts, USA), and detection was performed with a primary antibody specific for FtsZ for characterization of plasma membranes of bacteria. Mouse anti-Na⁺/K⁺-ATPase, ATP5A (IM CVa), panthenol-cytochrome C reductase core protein I (IM Core I), VDAC1/Porin (OM Porin), matrix cyclophilin D (matrix CypD), and antibody Cytc to IMS cytochrome C (IMS) were also used for detection. These are markers of tumor-derived membranes. For FtsZ, a secondary antibody was an anti-rabbit IgG HRP conjugated antibody, For other primary antibodies, the secondary antibody was anti-mouse IgG HRP conjugated antibody. To further characterize the HM-NPs, immunogold staining was performed. The sample was deposited onto a carbon-coated copper grid. The grid was blocked with 1wt% bovine serum albumin (BSA) in PBS. The EM-NP grid was stained with 10 µL anti-rabbit FtsZ (0.5 mg.mL⁻¹) and the TM-NP grid was stained with 10 µL anti-mouse Na⁺/K⁺-ATPase (0.5 mg.mL⁻¹). After one hour incubation, the sample was washed 6 times in 1% BSA. The EM-NPs grid was then stained with 10 µL of an anti-rabbit IgG-gold conjugate (5-nm gold nanoparticle) and diluted 1:20 with 1% BSA. The TM-NPs grid was stained with 10 µL of an anti-mouse IgG-gold conjugate (10-nm gold nanoparticle) and diluted 1:20 with 1% BSA. After one hour incubation at a dark place, the grid was washed 8 times with PBS. The sample was then fixed in 50 µL of 1% glutaraldehyde in PBS and washed 8 times with sterile water for 2 min each time. Finally, the sample grid was stained with 10 µL of 1% uranyl acetate and examined using TEM (HT7700, HITACHI, Japan). The HM-NPs grid was prepared following the same procedure. A mixture of antibodies against FtsZ and Na⁺/K⁺-ATPase was used as a primary antibody. An anti-rabbit IgG-gold conjugate (5-nm gold nanoparticle) and an anti-mouse IgG-gold conjugate (10-nm gold nanoparticle) were conjugated to label the HM-NPs.

### Design and characterization of HM-NPs

The engineering design of the HM-NPs included three steps: 1) an EM-TM hybrid membrane (HM) vesicle was prepared; 2) lactide-glycolic acid polymer nanoparticles (PLGA NPs) were synthesized; and 3) the HM was coated onto the PLGA NPs (FIG. 29A). EM was isolated from an *Escherichia coli* strain DH5α and TM was isolated from an autologous tumor cell membrane. The protein expression profiles of the *Escherichia coli* inner membrane (Tables 1A-1B) and the surgically resected 4T1 tumor cell plasma membrane (Tables 2A-2B) were identified by a liquid chromatography-mass spectrometry (LC-MS). The Venn diagrams in FIG. 22 show that there is little difference in membrane proteins between EMs (left in FIG. 22; E1, E2, E3, and E4) and TMs (right in FIG. 22; T1, T2, T3, and T4) in four batches of different samples. The membranes with different ratios were fused at protein mass ratios of 1:100, 1:75, 1:50, 1:25, 1:10, 1:5, 1:3, 1:1, 1:0, 0:1, 3:1, 5:1, 10:1, 25:1, 50:1, 75:1 or 100:1 between the EM and TM. The HM was prepared by repeatedly passing through a liposome extruder with a pore diameter of a filter membrane of 400 nm. In order to determine the optimal membrane ratio to activate DC, the HM with different membrane ratios was incubated with the BMDCs for 24 h and the released proinflammatory cytokines were measured. The results show that the releases of the proinflammatory cytokines in the BMDCs increase with the increase of the EM (FIGs. 23A-23C). When the protein mass ratio of the EM to TM is 3:1, the highest levels of the proinflammatory cytokines are induced.

**Table 1A Identification of protein expression profile of cell plasma membrane of Escherichia coli DH5α by LC-MS**

| Name of protein | Protein accession number | Scoring of database search of protein | Molecular weight [KDa] | Abundance [Mean ± standard deviation] ×10⁹ | Functional description |
|---|---|---|---|---|---|
| FtsZ | P0A9A6 | 991 | 40.3 | 2.48 ± 0.40 | Cell division protein |
| YhcB | P0ADW3 | 125 | 15.0 | 1.96 ± 0.21 | Inner membrane protein |
| YidC | P25714 | 109 | 61.5 | 0.31 ± 0.11 | Inner membrane protein transposase |
| NlpI | P0AFB1 | 71 | 33.6 | 0.09 ± 0.03 | Cell division protein |
| MsbA | P60752 | 69 | 64.4 | 5.66 ± 3.74 | ABC transport protein |
| TatA | P69428 | 58 | 9.7 | 0.13 ± 0.05 | Inner membrane transporter |
| MlaA | P76506 | 43 | 28.0 | 1.25 ± 0.39 | Intermembrane phospholipid transport system lipoprotein |
| TolQ | P0ABU9 | 26 | 25.6 | 0.05 ± 0.01 | Inner membrane protein |
| YebE | P33218 | 24 | 23.7 | 0.07 ± 0.01 | Inner membrane protein |

**Table 1B Protein expression profile in gram-negative bacterial inner membrane identified by LC-MS**

| Name of protein | Protein accession number | Scoring of database search of protein | Molecular weight [KDa] | Type |
|---|---|---|---|---|
| FtsZ | P0A9A6 | 991 | 40.3 | Cell division protein |
| YhcB | P0ADW3 | 125 | 15.0 | Inner membrane protein |
| YidC | P25714 | 109 | 61.5 | Inner membrane protein transposase |
| MsbA | P60752 | 69 | 64.4 | ABC transport protein |
| TatA | P69428 | 58 | 9.7 | Inner membrane transporter |
| MlaA | P76506 | 43 | 28.0 | Intermembrane phospholipid transport system lipoprotein |
| TolQ | P0ABU9 | 26 | 25.6 | Inner membrane protein |
| YebE | P33218 | 24 | 23.7 | Inner membrane protein |
| SecB | C4ZXK1 | 63 | 17.3 | Protein-export protein |
| FtsY | P10121 | 103 | 54.5 | Signal recognition granulin receptor |
| Ffh | P0AGD8 | 174 | 49.8 | Signal recognition granulin |
| YajC | P0ADZ8 | 70 | 11.9 | Sec translocon accessory complex subunit |
| PqiC | P0AB10 | 46.28 | 20.6 | Intermembrane transport lipoprotein |
| TolC | P02930 | 313.125 | 53.7 | Outer membrane protein |
| FimD | P30130 | 235.099 | 96.4 | Outer membrane usher protein |
| OmpC | P06996 | 202.498 | 40.3 | Outer membrane porin |
| PqiB | P43671 | 181.753 | 60.5 | Intermembrane transport protein |
| Lpp | P69778 | 128.985 | 8.3 | Major outer membrane lipoprotein |
| MltB | P41052 | 109.193 | 40.2 | Membrane-bound lytic murein transglycosylase |
| YbhG | B7MGQ3 | 101.826 | 36.4 | UPF0194 membrane protein |
| IgaA | P58720 | 99.794 | 79.4 | Putative membrane protein IgaA homolog |
| YejM | P0AD29 | 67.313 | 67.3 | Inner membrane protein |
| MltA | P0A935 | 64.13 | 40.4 | Membrane-bound lytic murein transglycosylase |
| MlaC | P0ADV7 | 42.361 | 23.9 | Intermembrane phospholipid transport system binding protein |
| YlaC | P0AAS0 | 40.683 | 18.3 | Inner membrane protein |
| YebT | P76272 | 39.947 | 94.9 | Intermembrane transport protein |
| MltC | B6I7A0 | 38.718 | 40.1 | Membrane-bound lytic murein transglycosylase |
| MlaF | P63388 | 36.693 | 29.1 | Intermembrane phospholipid transport system ATP-binding protein |
| MlaD | P64604 | 35.836 | 19.6 | Intermembrane phospholipid transport system binding protein |
| YqjE | P64585 | 30.275 | 15.1 | Inner membrane protein |
| YfjD | P37908 | 28.593 | 48 | Inner membrane protein |
| YoaE | P0AEC1 | 28.151 | 56.5 | Inner membrane protein |
| mltE | B5YXL7 | 24.052 | 22.2 | Inner membrane lysis murein glycosylase A |
| YedE | P31064 | 23.894 | 44.4 | Inner membrane protein |
| MlaB | P64602 | 21.602 | 10.7 | Intermembrane phospholipid transport system binding protein |
| YccF | P0AB12 | 21.108 | 16.3 | Inner membrane protein |
| MalE | P0AEX9 | 205.154 | 396 | Intermembrane phospholipid transport system permease protein |
| YedI | P46125 | 18.288 | 32.2 | Inner membrane protein |
| YgaP | P55734 | 13.461 | 18.6 | Inner membrane protein |
| PqiA | P0AFL9 | 13.238 | 46.4 | Intermembrane transport protein |
| YhcE | P25743 | 11.954 | 23.5 | UPF0056 membrane protein |
| YbhL | P0AAC5 | 11.854 | 25.9 | Inner membrane protein |
| YhjD | P37642 | 11.814 | 37.9 | Inner membrane protein |
| YbaT | P77400 | 11.769 | 45.6 | Inner membrane transporter |
| YjjP | P0ADD5 | 11.727 | 28 | Inner membrane protein |
| YhaH | P64591 | 10.057 | 14.3 | Inner membrane protein |
| YbjJ | P75810 | 8.525 | 41.8 | Inner membrane protein |
| YqeG | P63340 | 7.918 | 45.1 | Inner membrane transporter |
| YtfL | P0AE45 | 7.761 | 49.7 | UPF0053 inner membrane protein |
| ArsB | P0AB93 | 7.161 | 45.5 | Arsenical pump membrane protein |
| YpjD | P64434 | 6.673 | 29.2 | Inner membrane protein |
| MliC | P28224 | 6.576 | 12.6 | Membrane-bound lysozyme inhibitor of C-type lysozyme |
| YcjF | B5Z0P2 | 6.421 | 39.4 | UPF0283 membrane protein |
| YciC | B7ML08 | 6.389 | 26.4 | UPF0259 membrane protein |
| YgfX | Q46824 | 6.321 | 16.1 | Inner membrane protein |
| YbbJ | P0AAS3 | 6.27 | 16.9 | Inner membrane protein |
| LolE | P75958 | 5.4 | 45.3 | Lipoprotein-releasing system transmembrane protein |
| YjcH | P0AF54 | 5.289 | 11.7 | Inner membrane protein |
| SecG | P0AGA0 | 4.973 | 11.4 | Protein-export membrane protein |
| YfdC | P37327 | 4.703 | 34.5 | Inner membrane protein |
| YeiH | P62724 | 4.352 | 36.9 | UPF0324 inner membrane protein |
| YobD | B1XH87 | 4.039 | 17.6 | UPF0266 membrane protein |
| YdjZ | P76221 | 3.91 | 26.1 | TVP38/TMEM64 family inner membrane protein |
| UidC | Q47706 | 3.757 | 46.6 | Membrane-associated protein |
| YbiR | P75788 | 3.283 | 41.1 | Inner membrane protein |
| YhiM | P37630 | 2.542 | 37.6 | Inner membrane protein |
| YfcA | P0AD32 | 2.499 | 28.6 | Membrane transport protein |
| YdgK | P76180 | 1.92 | 16.3 | Inner membrane protein |
| MltF | Q0TET0 | 1.522 | 58.3 | Membrane-bound lytic murein transglycosylase F |
| MdtP | Q8CVH8 | 1.348 | 53.4 | Multidrug resistance outer membrane protein |
| YfbV | B7NNX4 | 1.164 | 17.2 | UPF0208 membrane protein |
| ECs0776 | P0A913 | 132.962 | 18.8 | Peptidoglycan-associated lipoprotein |
| YiaD | P37665 | 115.848 | 22.2 | Lipoprotein |
| YeaY | P0AA91 | 83.997 | 20.9 | Lipoprotein |
| INT | Q8FJY4 | 80.529 | 57 | Apolipoprotein N-acyltransferase |
| MetQ | Q8X8V9 | 80.04 | 29.4 | D-methionine-binding lipoprotein |
| GfcD | P75882 | 78.791 | 78.6 | Lipoprotein |
| YbjP | P75818 | 77.229 | 19 | Lipoprotein |
| YgeR | Q46798 | 73.906 | 26.5 | Lipoprotein |
| LolD | Q1RD37 | 49.359 | 25.4 | Lipoprotein-releasing system ATP-binding protein |
| OsmE | P0ADB1 | 47.972 | 12 | Osmotically-inducible lipoprotein |
| OsmB | P0ADA7 | 47.236 | 6.9 | Osmotically-inducible lipoprotein |
| YdcL | P64451 | 43.182 | 24.4 | Lipoprotein |
| YajG | P0ADA6 | 39.01 | 20.9 | Lipoprotein |
| NlpE | P40710 | 30.969 | 25.8 | Lipoprotein |
| LGT | B1LR25 | 26.132 | 33.1 | Phosphatidylglycerol:prolipoprotein diacylglyceryl transferase |
| YghJ | E3PJ90 | 24.412 | 167.9 | Lipoprotein |
| YedD | P31063 | 17.663 | 15 | Lipoprotein |
| YifL | P0ADN7 | 14.649 | 7.2 | Lipoprotein |
| YgdI | P65292 | 13.66 | 8.2 | Lipoprotein |
| YbaY | P77717 | 9.259 | 19.4 | Lipoprotein |
| LspA | Q0TLW4 | 3.978 | 18.1 | Lipoprotein signal peptidase |
| YceB | P0AB26 | 3.97 | 20.5 | Lipoprotein |
| YajI | P46122 | 2.083 | 19.5 | Lipoprotein |

A Proteome Discoverer 2.4 software (Thermo Fisher Scientific, USA) is used to analyze the proteins in EM. Accession number (protein accession number) displays default a unique identifier assigned to the protein by an FASTA database used to generate a report. Abundance displays an abundance value of a sample before scaling and normalization. The data are expressed as mean value ± standard deviation of 4 biologically independent plasma membrane samples of *Escherichia coli* DH5α.

Through mass spectrometry analysis, some characteristic proteins are also identified. These characteristic proteins can be used to confirm a component derived from a bacterial inner membrane in the pharmaceutical combination. For example, these characteristic proteins of a bacterial inner membrane may include a protein or a functionally active fragment thereof selected from the following group consisting of: a cell division protein FtsZ, an inner membrane protein YhcB, an inner membrane protein transposase YidC, a cell division protein NlpI, an ABC transporter MsbA, an inner membrane transporter TatA, an intermembrane phospholipid transport system lipoprotein MlaA, an inner membrane protein TolQ, an intermembrane transport lipoprotein PqiC, an outer membrane protein TolC, an outer membrane usher protein FimD, an outer membrane porin OmpC, a transmembrane transport protein PqiB, a major outer membrane lipoprotein Lpp, a membrane-bound lytic murein transglycosylase MltB, a UPF0194 membrane protein YbhG, a putative membrane protein IgaA homolog, an intermembrane phospholipid transport system lipoprotein MlaA, an inner membrane protein YejM, a membrane-bound lytic murein transglycosylase MltA, an intermembrane phospholipid transport system binding protein MlaC, an inner membrane protein YlaC, an intermembrane transport protein YebT, a membrane-bound lytic murein transglycosylase MltC, an intermembrane phospholipid transport system ATP-binding protein MlaF, an intermembrane phospholipid transport system binding protein MlaD, an inner membrane protein YqjE, a UPF0053 inner membrane protein YfjD, a UPF0053 inner membrane protein YoaE, an inner membrane lysis murein glycosylase A, a UPF0394 inner membrane protein YedE, an intermembrane phospholipid transport system binding protein MlaB, an inner membrane protein YccF, an intermembrane phospholipid transport system permease protein MlaE, an inner membrane protein YedI, an inner membrane protein YgaP, a transmembrane transport protein PqiA, a UPF0056 membrane protein YhcE, an inner membrane protein YbhL, an inner membrane protein YhjD, an inner membrane transporter YbaT, an inner membrane protein YjjP, an inner membrane protein YhaH, an inner membrane protein YbjJ, an inner membrane transporter YqeG, a UPF0053 inner membrane protein YtfL, an arsenical pump membrane protein ArsB, an inner membrane protein YpjD, a membrane-bound lysozyme inhibitor of C-type lysozyme MliC, a UPF0283 membrane protein YcjF, a UPF0259 membrane protein YciC, an inner membrane protein YgfX, an inner membrane protein YbbJ, a lipoprotein-releasing system transmembrane protein LolE, an inner membrane protein YjcH, a protein-export membrane protein SecG, an inner membrane protein YfdC, a UPF0324 inner membrane protein YeiH, a UPF0266 membrane protein YobD, a TVP38/TMEM64 family inner membrane protein YdjZ, a membrane-associated protein UidC, an inner membrane protein YbiR, an inner membrane protein YhiM, a membrane transporter protein YfcA, an inner membrane protein YdgK, a membrane-bound lytic murein transglycosylase F, a multidrug resistance outer membrane protein MdtP, a UPF0208 membrane protein YfbV, a peptidoglycan-associated lipoprotein, a lipoprotein YiaD, a lipoprotein YeaY, an apolipoprotein N-acyltransferase, a D-methionine-binding lipoprotein MetQ, a lipoprotein GfcD, a lipoprotein YbjP, a lipoprotein YgeR, a lipoprotein-releasing system ATP-binding protein LolD, an osmotically-inducible lipoprotein OsmE, an osmotically-inducible lipoprotein OsmB, a lipoprotein YdcL, a lipoprotein YajG, a lipoprotein NlpE, a phosphatidylglycerol:prolipoprotein diacylglyceryl transferase, a lipoprotein YghJ, a lipoprotein YedD, a lipoprotein YifL, a lipoprotein YgdI, a lipoprotein YbaY, a lipoprotein signal peptidase, a lipoprotein YceB, a lipoprotein YajI, and an inner membrane protein YebE, and any combination of the above.

**Table 2A Protein expression profile of tumor membrane derived from surgically resected tumor identified by LC-MS**

| Name of protein | Protein accession number | Scoring of database search of protein | Molecular weight [KDa] | Abundance [Mean ± standard deviation] ×10⁸ | Functional description |
|---|---|---|---|---|---|
| Antigen peptide transporter 1 | P21958 | 39.322 | 78.863 | 9.15 ± 1.96 | Antigen transport associated with class I MHC molecules |
| H-2 class II histocompatibility antigen γ chain | P04441 | 8.7133 | 31.557 | 0.50 ± 0.12 | Formation and transport of MHC class II proteins |
| Protein tyrosine kinase SYK | P48025 | 10.157 | 71.376 | 0.05 ± 0.01 | Innate immune recognition |
| High affinity immunoglobulin ε receptor subunit γ | P20491 | 4.2233 | 9.6523 | 1.02 ± 0.27 | Cell adhesion translocatase |
| Ras-related C3 botulinum toxin substrate 2 | Q05144 | 10.34 | 21.441 | 5.31 ± 1.42 | IgE binding, IgE receptor activity, and IgG binding |
| Protein tyrosine kinase BTK | P35991 | 3.7038 | 76.437 | 0.09 ± 0.05 | Regulation of secretion, phagocytosis, and cell polarization |
| Protein tyrosine phosphatase receptor type C | P06800 | 51.282 | 144.84 | 2.52 ± 0.39 | Development and maturation of B cells |

**Table 2B Protein expression profile in cell membrane derived from tumor tissue identified by LC-MS**

| Name of protein | Protein accession number | Scoring of database search of protein | Molecular weight [KDa] | Functional description |
|---|---|---|---|---|
| Antigen peptide transporter 1 | P21958 | 39.322 | 78.863 | Transport of MHC class I molecule-related antigens |
| H-2 class histocompatibility antigen chain | P04441 | 8.7133 | 31.557 | Formation and transport of MHC class II proteins |
| Protein tyrosine kinase SYK | P48025 | 10.157 | 71.376 | Innate immune recognition and cell adhesion translocatase |
| High affinity immunoglobulin receptor subunit γ | P20491 | 4.2233 | 9.6523 | IgE binding, IgE receptor activity, and IgG binding |
| Ras-related C3 botulinum toxin substrate 2 | Q05144 | 10.34 | 21.441 | Regulation of secretion, phagocytosis, and cell polarization |
| Protein tyrosine kinase BTK | P35991 | 3.7038 | 76.437 | Development and maturation of B cells |
| Protein tyrosine phosphatase receptor type C | P06800 | 51.282 | 144.84 | Lymphocyte development and antigen signal transduction |
| ATP1a4 | Q9WV27 | 83.264 | 114.8 | Na⁺/K⁺-ATPase |
| ATP5f1a | Q03265 | 274.62 | 59.7 | ATP synthase subunit α |
| IM Core I | Q9D2R6 | 7.529 | 12 | Ubiquinol-cytochrome C reductase core protein I |
| VDAC | Q60932 | 184.465 | 32.3 | Voltage-dependent anion-selective channel |
| CypD | P49070 | 6.584 | 32.5 | Matrix cyclophilin D |
| IMS Cytc | P62897 | 67.671 | 11.6 | Intermembrane space cytochrome C |
| HSPG2 | Q05793 | 374.926 | 398 | Basement membrane-specific heparan sulfate proteoglycan core protein |
| ATP2b 1 | G5E829 | 303.293 | 134.7 | Plasma membrane calcium-transporting ATPase 1 |
| EMC1 | Q8C7X2 | 212.771 | 111.5 | Endoplasmic reticulum membrane protein complex subunit 1 |
| TMEM43 | Q9DBS1 | 211.564 | 44.8 | Transmembrane protein 43 |
| LMAN2 | Q9DBH5 | 127.866 | 40.4 | Vesicular integral-membrane protein VIP36 |
| VAPA | Q9WV55 | 122.943 | 27.8 | Vesicle-associated membrane protein-associated protein A |
| TM9SF2 | P58021 | 118.99 | 75.3 | Transmembrane 9 superfamily member 2 |
| TMED10 | Q9D1D4 | 111.544 | 24.9 | Transmembrane emp24 domain-containing protein 10 |
| APAMP | Q9D7N9 | 108.978 | 46.4 | Adipocyte plasma membrane associated protein |
| VAT1 | Q62465 | 108.935 | 43.1 | Synaptic vesicle membrane protein VAT |
| NUP210 | Q9QY81 | 104.791 | 204 | Nuclear pore membrane glycoprotein 210 |
| ATP2b4 | Q6Q477 | 98.016 | 133 | Plasma membrane calcium-transporting ATPase 4 |
| ATP2b2 | Q9R0K7 | 89.389 | 132.5 | Plasma membrane calcium-transporting ATPase 2 |
| STOM | P54116 | 87.004 | 31.4 | Erythrocyte band 7 integral membrane protein |
| TMED9 | Q99KF 1 | 80.956 | 27.1 | Transmembrane emp24 domain-containing protein 9 |
| TIMM44 | O35857 | 79.741 | 51.1 | Mitochondrial import inner membrane translocase subunit |
| PGRMC1 | O55022 | 78.46 | 21.7 | Progesterone receptor membrane component 1 |
| EMC3 | Q99KI3 | 78.122 | 30 | Endoplasmic reticulum membrane protein complex subunit 3 |
| VAPB | Q9QY76 | 77.666 | 26.9 | Vesicle-associated membrane protein-associated protein B |
| AOC3 | O70423 | 72.941 | 84.5 | Membrane primary amine oxidase |
| VAMP7 | P70280 | 72.08 | 25 | Vesicle-associated membrane protein 7 |
| GOLIM4 | Q8BXA1 | 71.893 | 76.7 | Golgi membrane protein 4 |
| PGRMC2 | Q80UU9 | 69.416 | 23.3 | Progesterone receptor membrane component 2 |
| TM9SF3 | Q9ET30 | 69.336 | 67.5 | Transmembrane 9 superfamily member 3 |
| TM9SF4 | Q8BH24 | 69.087 | 74.6 | Transmembrane 9 superfamily member 4 |
| EMC2 | Q9CRD2 | 67.817 | 34.9 | Endoplasmic reticulum membrane protein complex subunit 2 |
| TIMM50 | Q9D880 | 61.606 | 39.8 | Mitochondrial import inner membrane translocase subunit |
| PEX11b | Q9Z210 | 60.486 | 28.7 | Peroxisomal membrane protein 11B |
| TMED2 | Q9R0Q3 | 55.931 | 22.7 | Transmembrane emp24 domain-containing protein 2 |
| SCAMP3 | O35609 | 55.533 | 38.4 | Secretory carrier membrane protein 3 |
| TMX4 | Q8C0L0 | 52.095 | 37.1 | Thioredoxin-related transmembrane protein 4 |
| SLC25a17 | O70579 | 50.515 | 34.4 | Peroxisomal membrane protein PMP34 |
| PEX14 | Q9R0A0 | 49.56 | 41.2 | Peroxisomal membrane protein |
| EMC8 | O70378 | 46.138 | 23.3 | Endoplasmic reticulum membrane protein complex subunit 8 |
| IFITM3 | Q9CQW9 | 45.018 | 14.9 | Interferon-induced transmembrane protein 3 |
| LAMP2 | P17047 | 44.35 | 45.7 | Lysosome-associated membrane glycoprotein 2 |
| TMX2 | Q9D710 | 44.093 | 33.9 | Thioredoxin-related transmembrane protein 2 |
| VAMP3 | P63024 | 43.89 | 11.5 | Vesicle-associated membrane protein 3 |
| LAMP1 | P11438 | 43.082 | 43.8 | Lysosome-associated membrane glycoprotein 1 |
| TIMM8a1 | Q9WVA2 | 42.77 | 11 | Mitochondrial import inner membrane translocase subunit TIM8A |
| SCARB2 | 035114 | 42.475 | 54 | Lysosomal integral membrane protein-2 |
| IGHG2a | P01865 | 41.548 | 43.9 | Ig gamma-2A chain C region |
| TM9SF1 | Q9DBU0 | 37.836 | 68.9 | Transmembrane 9 superfamily member 1 |
| LEMD3 | Q9WU40 | 37.67 | 100.2 | Inner nuclear membrane protein Man1 |
| TMED4 | Q8R1V4 | 36.989 | 26 | Transmembrane emp24 domain-containing protein 4 |
| Thy1 | P01831 | 36.555 | 18.1 | Thy-1 membrane glycoprotein |
| TIMM23 | Q9WTQ8 | 34.772 | 22 | Mitochondrial import inner membrane translocase subunit |
| TIMM9 | Q9WV98 | 34.714 | 10.3 | Mitochondrial import inner membrane translocase subunit |
| TIMM13 | P62075 | 33.548 | 10.5 | Mitochondrial import inner membrane translocase subunit |
| SCAMP2 | Q9ERN0 | 32.888 | 36.4 | Secretory carrier membrane protein 2 |
| SCAMP1 | Q8K021 | 31.93 | 38 | Secretory carrier membrane protein 1 |
| ITM2b | 089051 | 29.051 | 30.2 | Integrin membrane protein 2B |
| TIMM10 | P62073 | 27.821 | 10.3 | Mitochondrial import inner membrane translocase subunit |
| VMP1 | Q99KU0 | 26.248 | 45.9 | Vacuole membrane protein 1 |
| GHITM | Q91VC9 | 24.089 | 37.3 | Growth hormone-inducible transmembrane protein |
| NRADD | Q8CJ26 | 21.981 | 24.7 | Membrane protein with a death domain |
| VAMP8 | O70404 | 20.787 | 11.4 | Vesicle-associated membrane protein 8 |
| TAPT1 | Q4VBD2 | 18.023 | 63.9 | Transmembrane anterior posterior transformation 1 |
| CHCHD4 | Q8VEA4 | 17.254 | 15.5 | Mitochondrial intermembrane space import and assembly protein 40 |
| GLMP | Q9JHJ3 | 17.173 | 43.8 | Glycosylated lysosomal membrane protein |
| MS4A6D | Q99N07 | 16.842 | 26.4 | Membrane-spanning 4-domains, subfamily A, member 6D |
| TRAM1 | Q91V04 | 15.724 | 43 | Translocation associated membrane protein 1 |
| IFITM2 | Q99J93 | 13.927 | 15.7 | Interferon-induced transmembrane protein 2 |
| SLMAP | Q3URD3 | 12.682 | 96.9 | Sarcolemma associated protein |
| MMGT1 | Q8K273 | 12.632 | 14.7 | Membrane magnesium transporter 1 |
| POM121 | Q8K3Z9 | 11.531 | 120.9 | Nuclear envelope pore membrane protein |
| AI467606 | Q8C708 | 11.34 | 24.5 | Transmembrane protein C16orf54 homolog |
| VMA21 | Q78T54 | 11.239 | 11.4 | Vacuolar ATPase assembly integral membrane protein |
| EMP1 | P47801 | 10.171 | 17.8 | Epithelial membrane protein 1 |
| PITPNM1 | O35954 | 8.909 | 134.9 | Membrane-associated phosphatidylinositol transfer protein 1 |
| SMIM15 | Q3UTD9 | 7.82 | 8.6 | Small integral membrane protein 15 |
| NEMP 1 | Q6ZQE4 | 6.452 | 49.8 | Nuclear envelope integral membrane protein 1 |
| GPR180 | Q8BPS4 | 6.36 | 48.9 | Integral membrane protein |
| SCAMP4 | Q9JKV5 | 6.017 | 25.3 | Secretory carrier membrane protein 4 |
| TRAM2 | Q924Z5 | 5.947 | 43.2 | Translocation associated membrane protein 2 |
| TMCC3 | Q8R310 | 5.754 | 53.7 | Transmembrane and coiled-coil domain family 3 |
| SMAP1 | Q91VZ6 | 5.472 | 47.6 | Stromal membrane-associated protein 1 |
| GPM6b | P35803 | 4.989 | 36.2 | Neuronal membrane glycoprotein M6-b |
| EMP2 | O88662 | 4.97 | 19.7 | Epithelial membrane protein 2 |
| GOLM1 | Q91XA2 | 4.955 | 44.3 | Golgi membrane protein 1 |
| SIDT2 | Q8CIF6 | 4.685 | 94.4 | SID1 transmembrane family member 2 |
| TGOLN2 | Q62314 | 3.821 | 38.8 | Trans-Golgi network integral membrane protein 2 |
| FAM18b | Q9D8T4 | 3.373 | 23.3 | Golgi apparatus membrane protein TVP23 homolog B |
| OXA1L | Q8BGA9 | 3.362 | 48.2 | Mitochondrial inner membrane protein |
| OSTM1 | Q8BGT0 | 3.116 | 38 | Osteopetrosis-associated transmembrane protein 1 |
| PEX13 | Q9D0K1 | 2.875 | 44.6 | Peroxisomal membrane protein |
| SMCO1 | Q8CEZ1 | 1.498 | 24.5 | Single-pass membrane and coiled-coil domain-containing protein 1 |
| DMAC1 | Q9CQ00 | 1.478 | 11.3 | Distal membrane arm assembly complex 1 |
| Scamp5 | Q9JKD3 | 1.431 | 26.1 | Secretory carrier membrane protein 5 |
| CFTR | P26361 | 1.347 | 167.8 | Cystic fibrosis transmembrane conductance regulator |
| OCSTAMP | Q9D611 | 1.236 | 54.5 | Osteoclast stimulatory transmembrane protein |
| FITM2 | P59266 | 1.166 | 30 | Fat storage-inducing transmembrane protein 2 |
| TMC5 | Q32NZ6 | 1.148 | 111 | Transmembrane channel-like protein 5 |

A Proteome Discoverer 2.4 software (Thermo Fisher Scientific, USA) is used to analyze the proteins in TM. Protein accession number displays by default a unique identifier assigned to the protein by an FASTA database used to generate a report. Abundance displays an abundance value of a sample before scaling and normalization. The data are expressed as mean value ± standard deviation of 4 biologically independent tumor membrane samples derived from surgical resection.

Through mass spectrometry analysis, some characteristic proteins are also identified. These characteristic proteins can be used to confirm a component derived from other organism in the pharmaceutical combination. For example, these characteristic proteins of the other organism may include a protein or a functionally active fragment thereof selected from the following group consisting of: an antigen peptide transporter 1, an H-2 class II histocompatibility antigen γ chain, a protein tyrosine kinase SYK, a high affinity immunoglobulin epsilon receptor subunit γ, a Ras-related C3 botulinum toxin substrate 2, a protein tyrosine kinase BTK, a protein tyrosine phosphatase receptor type C, a Na⁺/K⁺-ATPase, ATP5A (IM CVa), a ubiquinol-cytochrome C reductase core protein I (IM Core I), a VDAC1/porin (OM Porin), a matrix cyclophilin D (Matrix CypD), an intermembrane space cytochrome C (IMS Cytc), a basement membrane-specific heparan sulfate proteoglycan core protein (HSPG2), a plasma membrane calcium-transporting ATPase 1 (ATP2b1), an endoplasmic reticulum membrane protein complex subunit 1 (EMC1), a transmembrane protein 43 (TMEM43), a vesicular integral-membrane protein VIP36 (LMAN2), a vesicle-associated membrane protein-associated protein A (VAPA), a transmembrane 9 superfamily member 2 (TM9SF2), a transmembrane emp24 domain-containing protein 10 (TMED10), an adipocyte plasma membrane associated protein (APAMP), a synaptic vesicle membrane protein VAT (VAT1), a nuclear pore membrane glycoprotein 210 (NUP210), a plasma membrane calcium-transporting ATPase 4 (ATP2b4), a plasma membrane calcium-transporting ATPase 2 (ATP2b2), an erythrocyte band 7 integral membrane protein (STOM), a transmembrane emp24 domain-containing protein 9 (TMED9), a mitochondrial import inner membrane translocase subunit TIM44 (TIMM44), a progesterone receptor membrane component 1 (PGRMC1), an endoplasmic reticulum membrane protein complex subunit 3 (EMC3), a vesicle-associated membrane-protein-associated protein B (VAPB), a membrane primary amine oxidase (AOC3), a vesicle-associated membrane protein 7 (VAMP7), a Golgi membrane protein 4 (GOLIM4), a progesterone receptor membrane component 2 (PGRMC2), a transmembrane 9 superfamily member 3 (TM9SF3), a transmembrane 9 superfamily member 4 (TM9SF4), an endoplasmic reticulum membrane protein complex subunit 2 (EMC2), a mitochondrial import inner membrane translocase subunit TIM50 (TIMM50), a peroxisomal membrane protein 11B (PEX11b), a transmembrane emp24 domain-containing protein 2 (TMED2), a secretory carrier membrane protein 3 (SCAMP3), a thioredoxin-related transmembrane protein 4 (TMX4), a peroxisomal membrane protein PMP34 (SLC25a17), a peroxisomal membrane protein PEX14 (PEX14), an endoplasmic reticulum membrane protein complex subunit 8 (EMC8), an interferon-induced transmembrane protein 3 (IFITM3), a lysosome-associated membrane glycoprotein 2 (LAMP2), a thioredoxin-related transmembrane protein 2 (TMX2), a vesicle-associated membrane protein 3 (VAMP3), a lysosome-associated membrane glycoprotein 1 (LAMP1), a mitochondrial import inner membrane translocase subunit TIM8 A (TIMM8a1), a lysosomal integral membrane protein-2 (SCARB2), an Ig gamma-2A chain C region (IGHG2a), a transmembrane 9 superfamily member 1 (TM9SF1), an inner nuclear membrane protein Man1 (LEMD3), a transmembrane emp24 domain-containing protein 4 (TMED4), a Thy-1 membrane glycoprotein (Thy1), a mitochondrial import inner membrane translocase subunit TIM23 (TIMM23), a mitochondrial import inner membrane translocase subunit TIM9 (TIMM9), a mitochondrial import inner membrane translocase subunit TIM13 (TIMM13), a secretory carrier membrane protein 2 (SCAMP2), a secretory carrier membrane protein 1 (SCAMP1), an integrin membrane protein 2B (ITM2b), a mitochondrial import inner membrane translocase subunit TIM10 (TIMM10), a vacuole membrane protein 1 (VMP1), a growth hormone-inducible transmembrane protein (GHITM), a membrane protein with a death domain (NRADD), a vesicle-associated membrane protein 8 (VAMP8), a transmembrane anterior posterior transformation 1 (TAPT1), a mitochondrial intermembrane space import and assembly protein 40 (CHCHD4), a glycosylated lysosomal membrane protein (GLMP), a membrane-spanning 4-domains, subfamily A, member 6D (MS4A6D), a translocation associated membrane protein 1 (TRAM1), an interferon-induced transmembrane protein 2 (IFITM2), a sarcolemma associated protein (SLMAP), a membrane magnesium transporter 1 (MMGT1), a nuclear envelope pore membrane protein POM 121 (POM121), a transmembrane protein C16orf54 homolog (AI467606), a vacuolar ATPase assembly integral membrane protein VMA21 (VMA21), an epithelial membrane protein 1 (EMP1), a membrane-associated phosphatidylinositol transfer protein 1 (PITPNM1), a small integral membrane protein 15 (SMIM15), a nuclear envelope integral membrane protein 1 (NEMP1), an integral membrane protein GPR180 (GPR180), a secretory carrier membrane protein 4 (SCAMP4), a translocation associated membrane protein 2 (TRAM2), a transmembrane and coiled-coil domain family 3 (TMCC3), a stromal membrane-associated protein 1 (SMAP1), a neuronal membrane glycoprotein M6-b (GPM6b), an epithelial membrane protein 2 (EMP2), a Golgi membrane protein 1 (GOLM1), an SID1 transmembrane family member 2 (SIDT2), a trans-Golgi network integral membrane protein 2 (TGOLN2), a Golgi apparatus membrane protein TVP23 homolog B (FAM18b), a mitochondrial inner membrane protein OXA1L (OXA1L), an osteopetrosis-associated transmembrane protein 1 (OSTM1), a peroxisomal membrane protein PEX13 (PEX13), a single-pass membrane and coiled-coil domain-containing protein 1 (SMCO1), a distal membrane arm assembly complex 1 (DMAC1), a secretory carrier membrane protein 5 (SCAMP5), a cystic fibrosis transmembrane conductance regulator (CFTR), an osteoclast stimulatory transmembrane protein (OCSTAMP), a fat storage-inducing transmembrane protein 2 (FITM2), and a transmembrane channel-like protein 5 (TMC5), and any combination of the above.

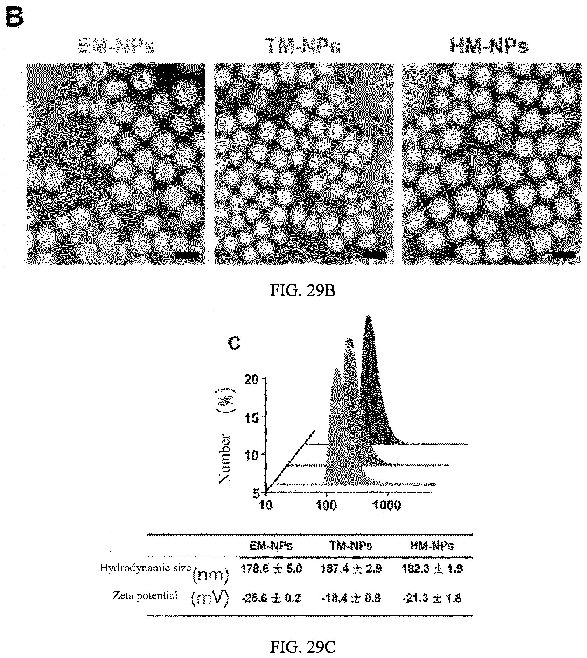

Transmission electron microscope (TEM) images show that all the three membrane vesicle preparations (EM, TM, and HM) have a similar size and a diameter of about 100 nm. Compared with the TM and HM vesicles, the EM vesicle has a Zeta potential more negative (FIGs. 24A-24C). The present application evaluates the content of lipopolysaccharide (LPS) in all the three membrane NPs (EM-NPs, TM-NPs, and HM-NPs). It is found that there is no significant difference between the HM-NPs group and the LPS-free control group (p = 0.2977) (FIG. 25), indicating that most LPS is removed from the *Escherichia coli* inner membrane during the preparation process. PLGA NPs are prepared by a double emulsion method. The HM-NPs were obtained by repeatedly co-extruding the PLGA core and the HM vesicle through a liposome extruder with a pore diameter of a filter membrane of 200 nm. Similarly, the EM-coated PLGA NPs (EM-NPs) and TM-coated PLGA NPs (TM-NPs) can be produced by repeatedly co-extruding the PLGA core with the EM vesicle or the TM vesicle through a liposome extruder with a pore diameter of a filter membrane of 200 nm. TEM images show that the HM-NPs have a uniform spherical nanostructure with an inner core and an outer shell (FIGs. 29B and 26A-26B). More specifically, the diameters of the PLGA inner core and the membrane outer shell of the HM-NPs are 86.83±7.41 nm and 14.16±2.07 nm, respectively (FIGs. 26A-26B). Moreover, when the mass ratio of the nanoparticle to the hybrid membrane is 5:1, the surface charge of the HM-NPs is -21.3 mV (FIG. 29C), which is basically unchanged compared with the charge of the HM vesicle (FIG. 24C), indicating that the HM surface is complete and saturated at the ratio. Dynamic light scattering (DLS) analysis shows that the HM-NPs have a hydrodynamic size of about 180 nm (FIG. 29C), which is similar to the EM-NPs and TM-NPs. The particle size difference between the TEM and DLS results is attributed to the following reasons: the TEM measures the diameter of the sample in the dry state, but the DLS measures the hydrodynamic size of the sample and takes into account the hydration layer on the particle surface at the same time. In a stability study, the size and Zeta potential of the HM-NPs remain almost unchanged before the freeze-drying and after resuspension in phosphate buffered saline (PBS; FIGs. 27A-27B). Furthermore, these physical properties of the HM-NPs remain stable after they are suspended in PBS at 4°C for more than 3 weeks (FIGs. 28A-28B).

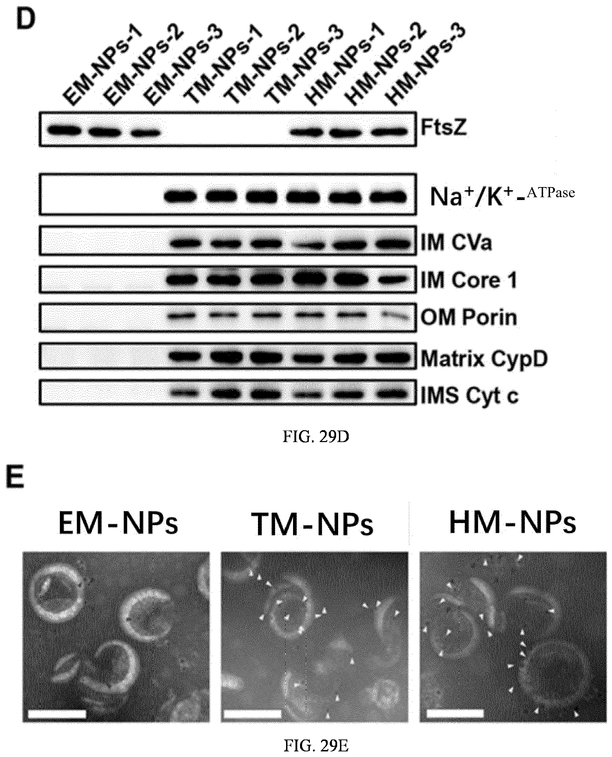

To identify the proteins contained in the membrane-coated NPs, the proteins are resolved by sodium dodecyl sulfate-polyacrylamide gel electrophoresis (SDS-PAGE) and the obtained gel is stained with Coomassie brilliant blue. The protein characteristics of the HM-NPs are similar to the protein combination of the two separate membrane NPs (FIG. 30, EM-NPs and TM-NPs). To further characterize the HM-NPs, the present application examine the specific proteins of the EM-NPs, TM-NPs, and HM-NPs (FIG. 29D). FtsZ is a bacterial cell division protein present in both the EM-NPs and HM-NPs (FIG. 29D, above). However, a series of mammalian cellular proteins including Na⁺/K⁺-ATPase, ATP5A (IM CVa), panthenol-cytochrome C reductase core protein I (IM Core I), VDAC1/Porin (OM Porin), matrix cyclophilin D (matrix CypD), and IMS cytochrome C (IMS Cytc) are obviously present in both the TM-NPs and HM-NPs (FIG. 29D, below). Furthermore, the three membrane nanoparticles were subjected to immunogold staining and observed by TEM which provided a direct visual evidence of the presence of the FtsZ and Na⁺/K⁺-ATPase (specific markers for the EM and TM, respectively) on the HM-NPs, respectively (FIG. 29E).

The abundance of 5-nm gold particles (bound to FtsZ, labeled EM) on the surface of the HM-NPs is greater than that of 10-nm gold particles (bound to Na⁺/K⁺-ATPase, labeled TM) in the HM-NPs (FIGs. 31A-31B), corresponding to the fact that a feeding ratio of the EM is greater than that of the TM. In conclusion, the present application has successfully constructed a hybrid membrane-coated nanoparticle that inherits key components from a bacterial inner membrane and a tumor cell membrane.

### Example 13

Detection of HM-NPs to enhance uptake of tumor antigens, activate expressions of TLRs on surface of BMDCs, and simultaneously deliver antigens and adjuvants to BMDCs

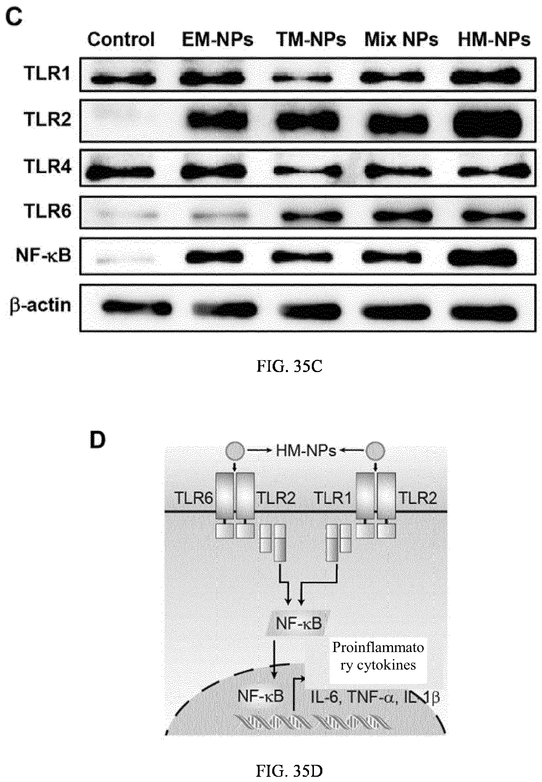

Internalization into DCs is a first step in processing and presentation of tumor-associated antigens (TAAs). Thus, the present application investigates the internalization of the three membrane NPs by the BMDCs. After incubation of rhodamine B-loaded membrane preparations with the BMDCs for one hour, all the three membrane nanoparticles labeled with rhodamine B are found to internalize into the BMDCs and localize within a lysosomal structure (FIGs. 35A and 32). Since most nanoparticles are trapped in a lysosomal compartment, there is no statistical difference in the co-localization efficiency of the three membrane NPs with the lysosome (FIG. 33). We also study the ability of mouse BMDCs to uptake each group of the membrane nanoparticles using a flow cytometry analyzer and synthesize the NPs (Mix NPs, i.e., physically mixed EM-NPs and TM-NPs) or the HM-NPs combined rhodamine B-loaded membrane samples. The samples are incubated with the BMDCs for 8 h. Then, the present application evaluate the fluorescence intensity of rhodamine B in each group of cells by a flow cytometry (FIG. 35B). The present application observe that the BMDCs only uptake a small fraction of the TM-NPs (5.16%), suggesting that a vaccine derived from the TM alone is not sufficient to effectively present antigens to APCs. In contrast, the BMDCs efficiently uptake both bacterial inner membrane-derived preparations. The EM-containing samples (EM-NPs, Mix NPs, and HM-NPs) show 20 times higher cell-associated fluorescence than the TM-NPs group. This may due to the fact that the DCs surface exhibits a large number of PRRs, which help to identify PAMPs on the EM surface and contribute to increase uptake levels of the DCs. In addition, the absorption rate of the HM-NPs is almost the same as that of the EM-NPs (FIG. 35B). These results indicate that the introduction of the bacterial inner membrane can facilitate the uptake of the nanoparticles by the BMDCs and grant the HM-NPs with the characteristic of stimulating DC uptake of tumor antigens by a membrane fusion technology. Studies show that the TLRs can recognize the bacterial surface PAMPs and initiate an innate immune response. To investigate the expression of the TLRs on the surface of the DCs stimulated by an EM adjuvant, we co-incubate different groups of the membrane nanoparticles (EM-NPs, TM-NPs, Mix NPs, and HM-NPs) with the BMDCs at 37°C for 24 h. Then whole proteins on the surfaces of the cells in each group are extracted and the expression levels of the proteins in each group are detected by using a series of specific membrane surface TLRs protein antibodies TLR1, TLR2, TLR4, and TLR6. The present application observes that the expressions of the TLR1, TLR2, and TLR6 in the BMDCs of the HM-NPs treatment group are higher than those of the control group (FIG. 35C), proving that the HM-NPs can stimulate the expressions of a plurality of membrane TLRs on the surface of the APCs and show the good potential to stimulate innate immunity. In the present application, it is found that there is no significant difference in the TLR4 expression among all the groups, probably due to the fact that a majority of LPS is removed during the preparation process of the HM-NPs and thus the expressions of downstream TLR4 receptors are not obviously changed (FIG. 25). TLR stimulation regulates the production of proinflammatory cytokines through downstream proinflammatory signal transduction. The expression of the NF-κB molecule is obviously upregulated in the HM-NPs group compared to the control group (FIG. 35C). A possible known signal transduction mechanism is shown in FIG. 35D.

To assess the stimulatory level of the EM on the innate immunity of the BMDCs, the present application first examines the ability and changes of the EM-NPs alone to stimulate the secretion of the proinflammatory cytokines after incubation with the BMDCs for a certain period of time. The EM-NPs with a series of concentration gradients (0.12 mg/mL, 0.25 mg/mL, 0.50 mg/mL, and 1.00 mg/mL) are co-incubated with the BMDCs, supernatants of each group of cells are collected at different time points (0 h, 3 h, 8 h, 24 h, and 48 h). The secretion level of the proinflammatory cytokine IL-6 in the supernatant is detected by using a kit. The observed secretion level of the IL-6 presents an obvious concentration and time dependence, indicating that a bacterial membrane vesicle can stimulate the BMDC activation and activate an innate immune response (FIG. 34). To further investigate the ability of different groups of the membrane nanoparticles to stimulate the secretion of proinflammatory cytokines by the BMDCs, different membrane NPs were incubated with the BMDCs for 24 h, and then specific cytokines are measured (FIGs. 35E-35G). Compared to the TM-NP group, the present application observe a large release of IL-6, TNF-α, and IL-1β in the BMDCs treated with the HM-NPs. The dramatic cytokine release curve in the HM-NPs is attributed to the adjuvanticity of the bacterial plasma membrane component, as the TM-NPs lack the ability to stimulate the BMDCs. Furthermore, the HM-NPs are more effective than the Mix NPs in stimulating the IL-6 and IL-1β secretion (p < 0.0001). Tumor self-antigens and immune adjuvants have the potential to increase the intensity of immune stimulation more strongly on the same nanoparticle compared with that the individual components are delivered together in admixture.

Next, the present application investigates the ability of the HM-NPs to promote BMDC maturation. The BMDCs are treated with different samples for 24 h and then stained with flow cytometry antibodies CD11c (marker for DCs), CD80 (stimulatory marker for mature DCs), and CD86 (stimulatory marker for mature DCs). The fluorescence intensity of the cells is measured by a flow cytometry (FIG. 35H-35I). Consistent with the BMDC activation result (FIGs. 35E-35G), the percentages of mature BMDCs in all the EM-containing nanoparticles (EM-NPs, Mix NPs, and HM-NPs) are all significantly increased (all *p* < 0.0001), as indicated by the enhanced expressions of the CD80 and CD86. It is also observed in the present application that the BMDCs fail to achieve efficient maturation when incubated with the TM-coated nanoparticle (FIGs. 35H-35I). These results indicate that the bacterial inner membrane confers the ability of the HM-NPs to promote DC maturation. Again, consistent with the DC activation result (FIGs. 35E-35G), compared to the Mix NPs group, the proportion of maturation of the BMDCs treated with the HM-NPs is greater (*p* < 0.0001), further confirming the advantages of the HM-NPs in enhancing an innate immune response (FIGs. 35H-35I). Notably, there is no significant difference between BMDC internalization rates of the HM-NPs and Mix NPs (p > 0.9999), but differences in stimulatory capacity of innate immunity are significant (FIG. 35B). To explain the phenomenon, we suppose that compared with the physical mixing of the single antigen and adjuvant, the nano system coated by the same membrane can realize a higher level of antigen-adjuvant co-delivery and generate a stronger synergistic stimulation immune effect.

To verify the above hypothesis, the present application further investigates the co-localization efficiency of the EM and TM components in the BMDCs treated by the HM-NPs and Mix NPs using an immunofluorescence experiment. The BMDCs are incubated with the HM-NPs or the Mix NPs for 8 h. The red fluorescently labeled FtsZ antibody and green fluorescently labeled Na⁺/K⁺-ATPase antibody are used to identify the EM and TM, respectively (FIG. 35J). The immunofluorescent image shows that co-localization of the HM-NPs group is significantly greater than the Mix NPs group (p < 0.0001) (mean 81.53% vs 52.39%; FIG. 35K) when the BMDCs are treated with the HM-NPs. These results indicate that the HM-NPs have enhanced ability to deliver the EM and TM to the same DC compared to the mixed sample. The co-delivery of the antigen and adjuvant to the DCs can enhance the immunogenicity of tumor antigens and the efficacy of cancer vaccines in immunotherapy. The results of the present application demonstrate that the HM-NPs can facilitate the co-presentation.

### Example 14

### Detection of HM-NPs in promoting DC maturation in LNs and activating tumor membrane antigen specific T cells

To assess DC maturation *in vivo,* the female BALB/c mice were inoculated subcutaneously with mouse 4T1 breast cancer cells. When the volume of the tumor reaches about 300 mm³, it was surgically resected. Most tumor tissues are resected to prepare a vaccine preparation, and the remaining about 1% is used to simulate the presence of residual micro-tumor in an operating table of the clinic. The mice were imaged on the 2nd day after the surgery, the tumors were measured, and the mice were randomly divided into five groups. The mice were immunized with different vaccine preparations on the 3rd, 5th, and 9th day after the surgery. 12 h after the last vaccination, the present application evaluated the presence of stimulatory molecules on DCs in inguinal lymph nodes. As shown in FIGs. 37A-37B, the expressions of both stimulatory markers (CD80 and CD86) are significantly increased after DC stimulation. The mice were treated with all the three membrane-coated nanoparticles (including TM-NPs) with the highest expressions in the HM-NPs group (the expressions of CD80 are 14.38%, 12.36%, and 13.82% and the expressions of CD86 are 14.46%, 9.76%, and 11.92% in the HM-NPs, EM-NPs, and Mix groups respectively). The efficacy of the TM-NPs *in vivo* (FIGs. 37A-37B) is enhanced compared to *in vitro* (FIGs. 35H-35I). This may due to the fact that after the TM-NPs enter the mouse body, the TM-NPs, as damage-associated molecular patterns (DAMPs), are recognized by sensitized immune cells (e.g., DCs) in the immune system, and stimulate DCs maturation to some extent. The HM-NPs treatment group shows the highest expressions of co-stimulatory markers, consistent with the *in-vitro* maturation result (FIGs. 35H-35I).

To present tumor membrane-associated antigens to naive T cells via activated DCs, the antigens must migrate to lymph nodes (LNs). To determine whether the HM-NPs can accumulate in the LNs, in the present application, the BALB/c mice are subcutaneously injected with the membrane NPs labeled with a fluorescent dye IR-780. After 12 h, the inguinal LNs were resected and imaged using an *in-vivo* imaging system (IVIS; FIG. 36A). Fluorescence signals are observed in all the groups. The fluorescence intensity of the HM-NPs group is 3.3 times that of the EM-NPs group and 12.4 times that of the TM-NPs group (FIG. 36B), indicating that the HM-NPs migrate to the LNs most efficiently for tumor antigen presentation.

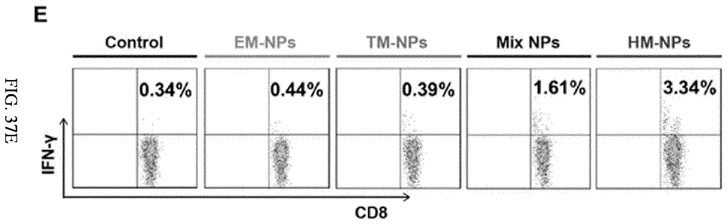

To further verify whether the HM-NPs could effectively activate naive T cells, in the present application, the female BALB/c mice were subcutaneously inoculated with the mouse 4T1 breast cancer cells. Surgical and vaccination procedures were as described above. To evaluate antigen specific CD8⁺ T cells, splenocytes were collected 12 h after the last vaccination and co-incubated with a 4T1 cell membrane (as an antigen) together for 24 h. The results show that almost no positive spots are observed in the TM-NPs treatment group, indicating that only the tumor membrane preparation has limited activation of T cells (FIGs. 37C-37D). At the same time, an appropriate amount of the T cells are activated in the EM-NPs treatment group. The weak immune response may be due to the lack of tumor membrane antigens. The Mix NPs and HM-NPs groups, containing both the antigens and adjuvants, show more positive spots than the other groups. These results reflect the weak immunogenicity of the tumor membrane, but the presence of the EMs can enhance the immunogenicity. Compared to the Mix NPs treatment group, the co-delivery of the antigens and adjuvant strategy induce an approximately two-fold increase in IFN-γ release from T cells. In addition, the production of tumor membrane antigen-specific cytokines in the T cells confirms that the ability of the HM-NPs to enhance a specific T cell response is much stronger than other preparations as assessed by flow cytometry (FIGs. 37E-37F). These results indicate that the HM-NPs can achieve DC maturation and splenic T cell activation through co-transport of tumor antigens and adjuvants.

### Detection of low systemic inflammatory response by HM-NPs

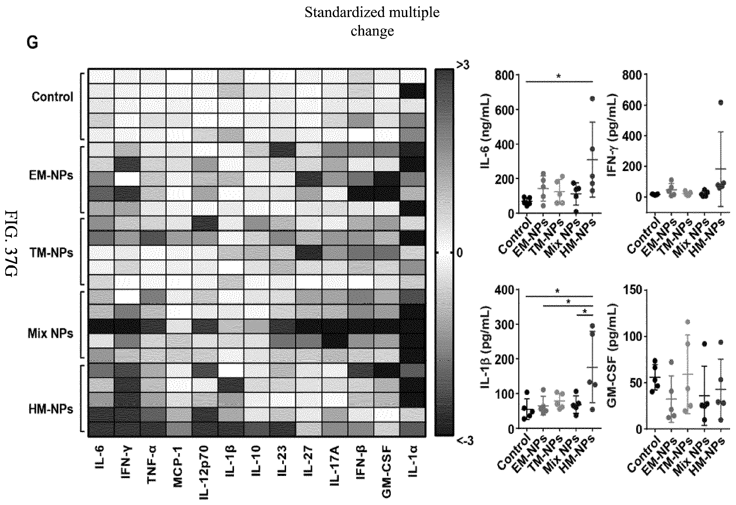

The stimulation to the patient's immune system is enhanced during cancer immunotherapy. The integration of the bacterial membrane component in the preparation can lead to life-threatening side effects such as cytokine storm. To assess the intensity of a systemic inflammatory response stimulated by the vaccine preparations, the female BALB/c mice are inoculated subcutaneously with the mouse 4T1 breast cancer cells. Surgical and vaccination procedures were as described above. In the ProcartaPlex multiple immunoassay, the concentrations of inflammatory cytokines and chemokines in serums are detected by using an ELISA kit based on Luminex magnetic beads. In general, the serum concentrations of IL-6, IFN-γ, TNF-α, MCP-1, IL-12p70, IL-1β, IL-23, IL-27, and IL-17A in the HM-NPs treated mice all increase (FIG. 37G and FIGs. 38A-38I). However, only two proinflammatory cytokines IL-6 and IL-1β increase statistically significantly in the HM-NP treated mice compared to the control mice (*p* = 0.0269; *p* = 0.0139). The remaining 11 cytokines or chemokines are expressed similarly in all the groups, indicating that the inflammatory response induced by the HM-NPs is controllable (FIG. 37G and FIGs. 38A-38I). Taken together, the HM-NPs can enhance the innate immunity, and have the adaptive immunity and low stimulation to the systemic inflammatory response, which indicates that the preparation has the potential of safely initiating a curative effect of antitumor immunotherapy.

### Example 15

### Detection of HM-NPs vaccination inducing tumor regression in mouse model of 4T1-Luc tumor

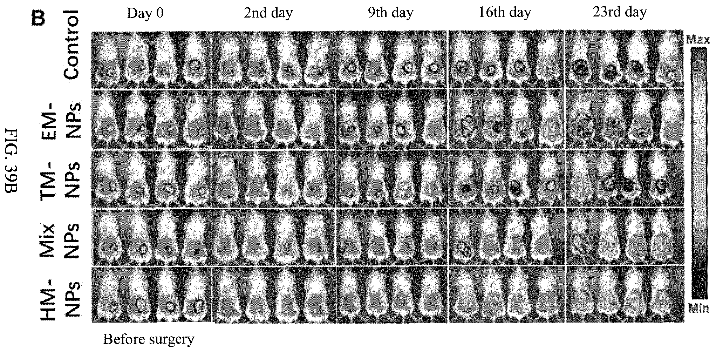

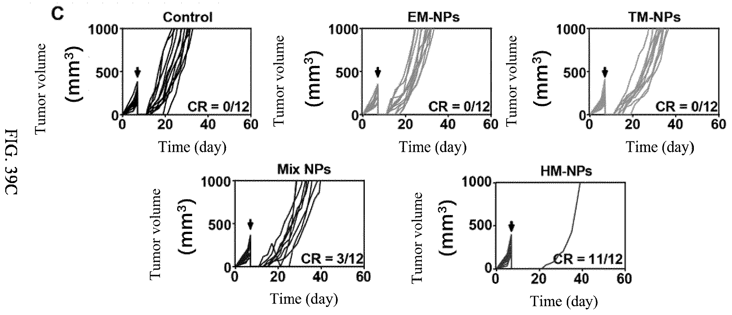

After confirming that the HM-NPs can activate DCs and T cells *in vivo*, the present application proceeds to evaluate the ability of the HM-NPs vaccine to inhibit tumor recurrence (FIG. 39A). To this end, the female BALB/c mice were subcutaneously injected with mouse 4T1-Luc breast cancer cells following surgical and vaccination procedures. The subcutaneous tumors of the mice were monitored using IVIS before and after the surgery (FIG. 39B). IVIS images show a gradual increase in tumor volume in all the non-HM-NPs vaccinated groups. As shown in FIGs. 39C-39E, tumor growth is not affected by the EM-NPs or TM-NPs vaccination. Although the Mix NPs preparation delays tumor recurrence, the antitumor effect is still mild with a survival rate of 25% within 60 days (FIG. 39E, *n* = 12). In contrast, the mice treated with the HM-NPs vaccine show a greater tumor inhibitory ability with a survival rate as high as about 92%, further confirming the benefit of the co-delivery of tumor antigens and adjuvants using the hybrid membrane nano-platform. Importantly, no tumor recurrence is observed in the mice surviving to the experimental endpoint (the 60th day), indicating that animal survival rate in the HM-NPs vaccinated group is significantly higher than other groups.

### Example 16

### Detection of HM-NP vaccine for inhibiting tumor regression in multiple mouse tumor models

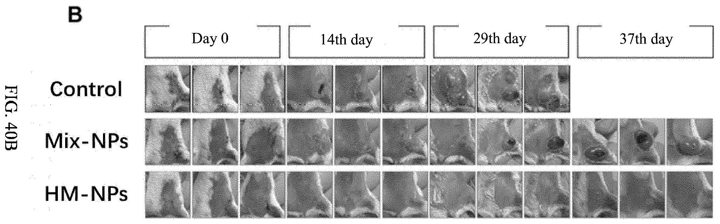

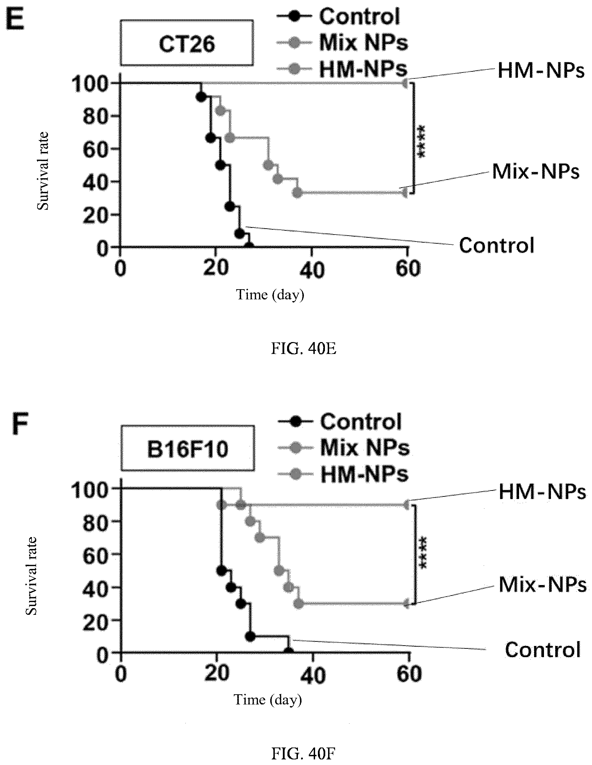

To demonstrate the general applicability of the HM-NPs in post-operative immunotherapy, in the present application, the female BALB/c mice were subcutaneously inoculated with CT26 colon tumor cells. The surgical procedure and inoculation schedule (FIG. 40A) were the same as described above. The tumor volume was measured using an electronic caliper and the survival of the mice in the three different groups was monitored within 60 days. As shown in FIGs. 40B-40D, in the HM-NPs group, vaccination after surgical treatment effectively inhibits the recurrence of the CT26 tumor at the surgical site. Similar to the results of the 4T 1-Luc model described above (FIGs. 39A-39E), the Mix NP preparation delays the tumor recurrence by only eight days compared to the untreated group. None of the mice in the Mix NP group survives to the time point of the 60th day. The mice vaccinated with the HM-NPs do not develop tumor recurrence within 60 days, whereas the median survival time of the mice in the Mix NPs group is approximately 37 days (FIG. 40E). It is worth noting that all the mice immunized with the HM-NPs in the experiment achieve effective tumor recurrence inhibition with an inhibitory rate as high as 100%.

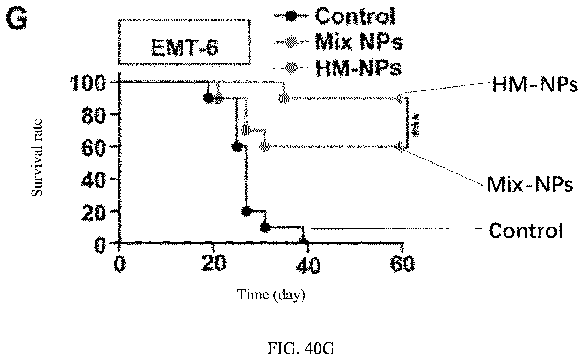

The CT26 and 4T1-luc models are more immunogenic and more responsive to immunotherapy. To further demonstrate the therapeutic potential of the HM-based vaccine of the present application, in the present application, the preparation is tested in two less immunogenic tumor models, B16F10 melanoma and EMT-6 breast tumor models. Similar to the results of the present application in the CT26 and 4T1-Luc models, over 90% of the mice vaccinated with the HM-NPs do not develop tumor recurrence within 60 days, but 40%-100% of tumor recurrence is observed in the Mix NPs and control groups. B16F10 melanoma (FIG. 40F) and EMT-6 breast tumor (FIG. 40G) models. Similarly, the results of the present application indicate that the vaccine based on the hybrid membrane strategy can be used in a variety of solid tumor models with significant therapeutic efficacy.

In the present application, the effect of the bacterial inner membrane as a vaccine adjuvant is also compared with the commercially available adjuvant monophosphoryl lipid A (MPLA) in the mouse model of CT-26 tumor (FIGs. 41A-41B). Meanwhile, the present application also studies the anti-tumor efficacy of the hybrid membrane vesicle without the PLGA core. The EM-based cancer vaccine preparation always shows higher therapeutic efficacy than other tumor membrane antigen-containing preparations including HM vesicles and three MPLA-containing groups (TM-NPs+free MPLA, TM-MPLA-NPs, and TM-NPs) (FIGs. 41A-41B). In the present application, it is observed that the complete response rate (CR) of the mice is 100% in the HM-NPs group, 62.5% in the HM vesicle group, and 50.0%-62.5% in the three MPLA-containing groups (FIGs. 41A-41B), indicating that the vaccine preparation with hybrid membrane-coated PLGA NPs is the best preparation in the experimental group.

### Example 17

### Detection of tumor re-regulation in CT26 tumor model protected by HM-NPs vaccination

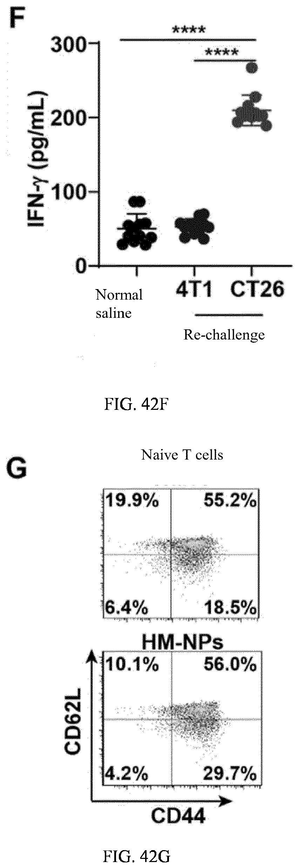

The present application further investigates whether the HM-NPs could provide long-term protection for more than 60 days in a CT-26 tumor re-challenge model. In the model, all the mice from the HM-NPs vaccinated group of the CT-26 tumor model were randomly divided into three groups and vaccinated with normal saline, CT-26 colon adenocarcinoma cells or 4T1 breast cancer cells, respectively. As shown in FIGs. 42A-42B, the mice inoculated with the CT-26 tumor cells show complete tumor elimination and a tumor inhibitory rate of 100%. In addition, the mice in the normal saline group have no tumor recurrence within 90 days. However, the tumor volume of the mice inoculated with the 4T1 cells reached 1,000 cm³ after 3 weeks, indicating that the protective effect provided by the HM-NPs is a CT-26 specific immune response. In addition, in the present application, it is found that compared with the 4T1 inoculation group, the serum concentrations of the proinflammatory cytokines (including IL-6, IL-1β, and TNF-α) in the mice inoculated with the CT-26 cells are increased. CT26 tumor re-challenge model (FIGs. 42C-42E). In addition, the secretion of the specific immune cytokine IFN-γ in the CT-26 vaccination group also increases (FIG. 42F). In T cell subgroups, the HM-NPs also increase effector memory T cells compared with initial T cells (FIGs. 42G-42H). Due to the presence of the same tumor antigens, these results are consistent with a stronger immune response. Therefore, the HM-NPs vaccine provides immunity against tumor recurrence and a specific long-term protection as well.

### Analysis of need for innate and adaptive immunity for tumor inhibition after HM-NPs vaccination

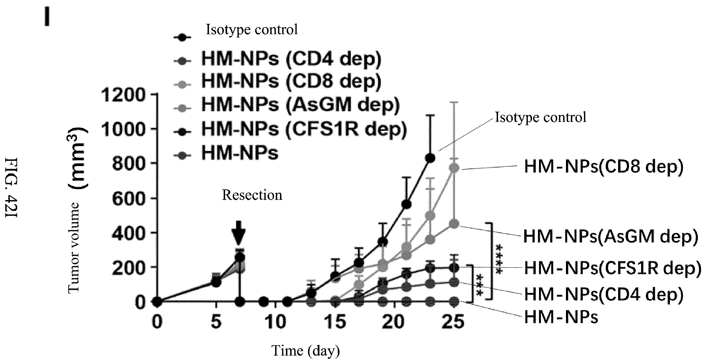

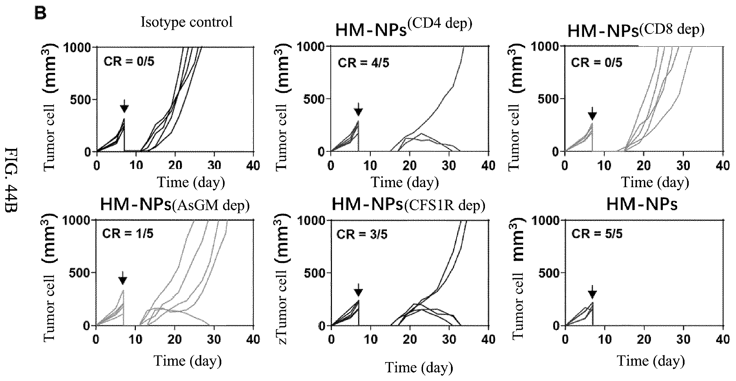

NK cells and macrophages play an important role in innate immunity, while CD4⁺T cells and CD8⁺T cells are crucial for an adaptive immune response. In order to explore the potential mechanism of enhancing an anti-tumor response induced by the HM-NPs, in the present application, the mice were inoculated with the CT-26 mouse colon adenocarcinoma cells and the tumor was resected as described above. The female BALB/c mice were subcutaneously inoculated with CT 26 cells. When the volume of the tumor reaches about 300 mm³, it was surgically resected. Then, in the present application, the mice were randomly divided into six different groups. The mice were intraperitoneally injected with depleting antibodies against surface markers of immune cells (Table 3) one day before the start of the HM-NPs treatment to deplete macrophages (CSF1R antibody), NK cells (ASGM1 antibody), CD8⁺ T cells (CD8 antibody) or CD4⁺ T cells (CD4 antibody). The depletion of the specific cell types is confirmed by flow cytometry of peripheral blood mononuclear cells (PBMCs; FIG. 43). Then the mice were inoculated with the HM-NPs or normal saline (as control) 3 times on the 3rd, 5th and 9th days after the surgery. As shown in FIG. 42I and FIG. 44A, when the immune system of the mice inoculated with the HM-NPs remains intact, the tumor remission rate reaches 100%. However, despite the inoculation of the HM-NPs, all the CD8-depleted mice will still develop a tumor, which reveals the main role of the CD8⁺ T cells in tumor regression (FIG. 42I and FIG. 44B). NK cell depletion also leads to a significant reduction in overall survival rate, but CD4⁺ T cell blockade does not significantly affect (p = 0.3173) the effect of the HM-NPs vaccination (FIG. 44B). The restriction of the macrophages leads to tumor recurrence to a certain extent in the mice inoculated with the HM-NPs, and the tumor recurrence rate is as high as 40% (FIG. 42I and FIG. 44B). In conclusion, the antibody-depleting study shows that both CD8⁺ T cells and NK cells are critical for tumor inhibition, and other immune cell subsets also play a role in tumor regression.

**Table 3 Selective cell type depletion during HM-NPs treatment**

| Cell type | Depleting antibody | Dose |
|---|---|---|
| Macrophages | InVivoMAb anti-mouse CSF1R (CD115), BE0213 | Intraperitoneal injection of 300 µg of depleting antibody every other day |
| NK cells | Ultra-LEAF^{™} purified anti-mouse Asialo-GM1, 146002, BioLegend | Injection of 50 µL each time from one day before treatment, twice a week |
| CD8⁺ T cells | InVivoMab anti-mouse CD8 (Lyt 2.1), BE0118-5 mg, BioXcell | Intraperitoneal injection of 400 µg of depleting antibody from one day before treatment, twice a week |
| CD4⁺ T cells | InVivoPlus, anti-mouse CD4 (GK1.5), BP0003-5 mg, BioXcell | Injection of 200 µg each time, once a week |

The peripheral blood of the mice is analyzed by flow cytometry to confirm the depletion of the macrophages (CSF1R antibody), the NK cells (ASGM1 antibody), the CD8⁺ T cells (CD8 antibody) or the CD4⁺ T cells (CD4 antibody) (FIG. 43).

### Example 18

### Safety detection of HM-NPs vaccine

Since a bacterial ingredient exists in the vaccine preparation, the present application evaluates the biological safety. Many bacteria are pathogenic due to the production of hemolysin. Therefore, in the present application, a hemolysis test is performed. Red blood cells are treated with a certain range of HM-NPs concentrations. No obvious hemolysis is observed in the HM-NPs at any test concentrations (FIG. 45). Then at the end point of a tumor regression experiment of the 4T1-Luc model, the present application harvests the main organs of the mice in each group subjected to hematoxylin-eosin (H&E) staining. No obvious histological changes are observed in any organs of the examined mice (FIG. 46). In addition, studies show that pathogen-derived molecules in the bacterial membrane can cause liver and kidney damages through a variety of pathogenesis. Therefore, the liver and kidney functions of the mice are evaluated. In the HM-NPs treated mice, there is no statistical difference in the serum concentrations of liver enzymes (ALT and AST) (FIGs. 47A-47B). The serum concentrations of kidney function markers (BUN and CREA) are approximately the same in all the groups (FIGs. 47C-47D). To sum up, these data indicate that the HM-NPs have good biological safety, can be used as an efficient and safe post-operative therapeutic vaccine for patients with solid tumors, and have the potential for clinical transformation.

### Example 19

Differential analysis of bacterial outer membrane vesicle (OMV) and bacterial inner membrane

### OMV proteomic analysis:

(1) *Escherichia coli* outer membrane vesicle was extracted, and frozen and stored;
(2) the protein concentration was determined by a BCA assay;
(3) the protein was subjected to an enzymolysis by a filter aided proteome preparation (FASP);
(4) the centrifuged, concentrated and dried polypeptide was desalted on a ziptip C18 column and prepared for analysis by mass spectrometry after drying; and
(5) 30 µL of loading buffer (acetonitrile:water:formic acid = 2:98:0.1) was added into the dried sample, and the sample was shaken to be dissolved, transferred to a sample bottle, and analyzed by mass spectrometry.

Database search and results: the experiment uses the basic process of proteome identification based on mass spectrometry, that is, the similarity between MS/MS mass spectrometry data after a series of optimization processing and the database is compared and scored to identify proteins. Each original data file and corresponding database are uploaded to a Maxquant 1.5.8.3 software respectively. After the database search, Reverse and Potential Contaminant proteins are removed, and the database search results are counted: 218 total proteins (non-redundant), 441 total peptide segments (include redundant), 430 specific peptide segments (razor+unique), and 1,230 total spectrograms (MS/MS Identified) are detected in the mass spectrometry database search. In the present application, the cell locations of the proteins are counted. It is found that the proteins in the cytosol account for the largest proportion, up to 34.63%. The specific results are shown in Table 4 and Table 5:

**Table 4 Distribution of proteins in outer membrane vesicle of Escherichia coli**

| Protein | % |
|---|---|
| Cytosol | 34.63 |
| Cytoplasmic large ribosomal subunit | 10.51 |
| Cytoplasm | 10.12 |
| Periplasmic space at outer membrane boundary | 7.39 |
| Cytoplasmic small ribosomal subunit | 7.00 |
| Membrane | 6.23 |
| Plasma membrane | 5.06 |
| Periplasmic space | 3.89 |
| Cell outer membrane | 3.89 |
| Component of membrane | 3.50 |
| Component of plasma membrane | 1.56 |
| Component of cell outer membrane | 1.17 |
| Pore complex | 1.17 |
| GroEL-GroES complex | 0.78 |
| Exodeoxyribonuclease VII complex | 0.78 |
| Outer membrane | 0.78 |
| Nucleoside | 0.78 |
| ATP-binding cassette (ABC) transporter complex | 0.78 |

In addition, the present application also selects 10 representative key proteins, and describes their molecular weights and functions. The results are shown in the following table:

**Table 5 Expressions of main proteins in outer membrane vesicle of Escherichia coli**

| Name of protein associated with peptide | Name of gene associated with peptide | Highest scoring in relevant MS/MS spectrum | Molecular weight of protein (Unit: kilodalton) |
|---|---|---|---|
| Outer membrane protein A | ompA | 163.79 | 37.702 |
| Outer membrane protein C | ompC | 232.16 | 40.508 |
| Outer membrane protein X | ompX | 160.44 | 18.602 |
| Osmotically-inducible lipoprotein E | osmE | 18.262 | 12.021 |
| Maltoporin | lamB | 19.912 | 49.851 |
| MltA-interacting protein | mipA | 48.552 | 27.831 |
| Outer membrane lipoprotein Lpp | lpp | 323.31 | 8.3234 |
| Outer membrane Lipoprotein SlyB | slyB | 5.7073 | 15.601 |
| Outer membrane protein W | ompW | 5.6974 | 22.928 |
| FhuE receptor | fhuE | 5.6276 | 81.232 |

### Functional description:

OmpA: outer membrane protein A generally exists on surfaces of gram-negative bacteria, has a β-barrel structure, can functionally provide permeability for an outer membrane, maintains the stability of the outer membrane structure, plays a crucial role in bacterial life activities, can be used as bacterial adhesin and invasin, participates in the formation of biofilm, can also be used as a receptor of a variety of bacteriophages, and is an important target of an innate immune system.

OmpC: outer membrane protein C, as an outer membrane pore protein of gram-negative bacteria, allows passive diffusion of small molecules on the outer membrane.

OmpX: outer membrane protein X, an important bacterial outer membrane protein, belongs to the Ail protein family. The protein is involved in the adhesion and invasion of bacteria to cells and the immune defense against hosts in other bacteria, and is closely related to the virulence of the bacteria. Therefore, the protein is considered as a potential candidate target for vaccine development.

OsmE: osmotically-inducible lipoprotein E is related to an osmotic pressure response.

LamB: maltoporin involves the transport of maltose and maltodextrin, and is essential for the transport of dextrin containing more than three glucosyl moieties. A hydrophobic ("greasy") path of aromatic residues is used to guide and select sugars for transport through channels. The protein can also be used as receptors for several bacteriophages (including λ).

MipA: MltA-interacting protein can be used as a scaffold protein for a complex formed by MrcB/PonB and MltA. The complex may play a role in the expansion and separation of wall protein vesicles.

Lpp: outer membrane lipoprotein Lpp interacts covalently and noncovalently with peptidoglycan. The interaction helps to maintain the structural and functional integrity of the cell envelope.

SlyB: outer membrane lipoprotein SlyB directly regulated by a *PhoP* gene which is mainly involved in regulating expressions of bacterial virulence genes.

OmpW: outer membrane protein W, as an outer membrane pore protein of gram-negative bacteria, is a receptor of colicin S4 and can be used as a target for drug screening.

FhuE: FhuE receptor is a receptor protein on an outer membrane of gram-negative bacteria and a receptor molecule necessary for absorption of Fe³⁺ via coprogen, ferrioxamine B, and rhodotorulic acid.

### Escherichia coli inner membrane proteomic analysis:

(1) Four batches of *Escherichia coli* inner membranes were extracted, and frozen and stored;
(2) the protein concentration was determined by a BCA assay;
(3) the protein was subjected to reductive alkylation;
(4) the protein was subjected to an enzymolysis by a filter aided proteome preparation (FASP);
(5) the centrifuged, concentrated and dried polypeptide was desalted on a ziptip C18 column and prepared for analysis by mass spectrometry after drying; and
(6) the centrifuged, concentrated and dried polypeptide was desalted on a ziptip C18 column and prepared for analysis by mass spectrometry after drying.

Database search and results: the experiment uses the basic process of proteome identification based on mass spectrometry, that is, the similarity between MS/MS mass spectrometry data after a series of optimization processing and the database is compared and scored to identify proteins. Proteome Discoverer 2.4 is used to search the database. After the database search, Reverse and Potential Contaminant proteins are removed, and the database search results are counted: 2,302 total proteins (non-redundant), 20,454 total peptide segments (include redundant), 19,006 specific peptide segments (razor+unique), and 86,026 total spectrograms (MS/MS Identified) are detected in the mass spectrometry database search. In the present application, the cell locations of the proteins are counted. It is found that the intracellular proteins account for the largest proportion. The specific results are shown in FIG. 48 and Table 6:

In addition, the present application also selects 12 representative proteins, and describes their protein scoring, molecular weights and functions. The results are shown in the following table:

**Table 6 Expressions of main proteins in different batches of Escherichia coli inner membrane**

| Name of protein | Protein accession number | Scoring of database search of protein | Molecular weight [KDa] | Type |
|---|---|---|---|---|
| FtsZ | P0A9A6 | 991 | 40.3 | Cell division protein |
| YhcB | P0ADW3 | 125 | 15.0 | Inner membrane protein |
| YidC | P25714 | 109 | 61.5 | Inner membrane protein transposase |
| MsbA | P60752 | 69 | 64.4 | ABC transport protein |
| TatA | P69428 | 58 | 9.7 | Inner membrane transporter |
| MlaA | P76506 | 43 | 28.0 | Intermembrane phospholipid transport system lipoprotein |
| TolQ | P0ABU9 | 26 | 25.6 | Inner membrane protein |
| YebE | P33218 | 24 | 23.7 | Inner membrane protein |
| SecB | C4ZXK1 | 63 | 17.3 | Protein-export protein |
| FtsY | P10121 | 103 | 54.5 | Signal recognition granulin receptor |
| Ffh | P0AGD8 | 174 | 49.8 | Signal recognition granulin |
| YajC | P0ADZ8 | 70 | 11.9 | Sec translocon accessory complex subunit |

### Functional description:

FtsZ: a cell division GTPase, a cell division protein, and a protein with clear characteristics in a bacterial cell division organ. It accumulates in middle and early stages of bacterial cell division and plays a crucial role in the septum formation of most bacteria. It has also been considered as a bacterial cytoskeleton counterpart of an eukaryotic microtubule.

YhcB: a bacterial inner membrane protein that participates in the biosynthesis of cytoskeleton and peptidoglycan.

YidC: an inner membrane protein transposase is related to Sec translocon, is a cofactor for integration, folding, and assembly of inner membrane proteins, and can transport small molecular inner membrane proteins (especially the inner membrane proteins with small molecular periplasmic domain) into the inner membrane.

MsbA: an ABC transporter and a lipid A export ATP-binding/permease protein.

TatA: an inner membrane transporter and a Sec-independent protein translocase protein, and belongs to the Tat family. Tat is a system in *Escherichia coli* capable of transporting folding proteins across the membranes. Its signal peptide contains a highly conserved twin-arginine motif. The members of the Tat family include 4 proteins of TatA, TatB, TatC, and TatE. Their complexes form transport channels on the plasma membrane of *Escherichia coli.* Most substrate proteins transported by the *Escherichia coli* Tat system are respiratory electron transfer chain components and related to many life activities of *Escherichia coli.*

MlaA: an intermembrane phospholipid transport system lipoprotein is responsible for the transport and exchange of phospholipids between internal and external membranes of bacteria.

TolQ: an inner membrane protein belongs to the Tol-Pal system. The Tol-Pal inner membrane system is one of the inner membrane protein systems of gram-negative bacteria. The designation of Tol proteins in the system is related to the resistance to colicin. Tol-Pal consists of five proteins: TolQ, TolA, TolB, TolR, and Pal. Their genes are located on the same operon. TolB is a periplasmic protein. TolA, Q and R are three inner membrane proteins which interact to form an inner membrane protein complex. TolQ protein is related to bacterial cell division.

YebE: an inner membrane protein belongs to a molecular tag for heat shock response of *Escherichia coli.*

SecB is a protein-export protein. A signal recognition particle (SRP) targets an inner membrane protein to an inner membrane in the manner of co-translation. However, the SecB pathway targets secretory proteins via late-stage co-translation or post translation process.

FtsY: a signal recognition granulin receptor. *Escherichia coli* SRP receptor only contains one subunit FtsY, namely a homolog of mammalian SRα. *Escherichia coli* FtsY is evenly distributed between cytoplasm and inner membranes. The binding of the FtsY to the membrane involves lipid and protein membrane factors, which may be SecY, a component of Sec-translocon. The interaction of the FtsY with phospholipid stimulates the activity of FtsY GTPase.

Ffh: a signal recognition granulin receptor is homologous to mammalian SRP54. Ffh is necessary for the growth of *Escherichia coli.* It has been proposed that SRP54 (Ffh), binding to a ribosome-targeting signal in the activated and GTP-binding form, can be used to interact with SR (FtsY). The GTPases of the Ffh and the FtsY stimulate each other in the formation of the complex. The Ffh and the FtsY are proposed to activate the GTPases, and act as GTPase-activating proteins for each other.

YajC: a Sec translocon accessory complex subunit is related to the biosynthesis of bacterial inner membrane proteins.

### Example 20

The ability of five bacteria, *Escherichia* (gram-negative), *Staphylococcus* (gram-positive), *Bacillus* (gram-positive), *Lactobacillus* (gram-positive), and *Pseudomonas* (gram-negative) in inhibiting tumor recurrence after combined with a cell membrane derived from an autologous tumor tissue is investigated.

In the example, nanoparticles HM-NPs formed by wrapping PLGA with hybrid membranes composed of a tumor cell membrane and inner membranes of five different bacteria, including *Escherichia, Staphylococcus, Bacillus, Lactobacillus,* and *Pseudomonas.* The concentration equivalents of total membrane proteins in the five products were consistent. At the same time, a single tumor membrane group and a normal saline group were used as controls. A mouse model of colon cancer was used to demonstrate the ability of different bacterial inner membranes in inhibiting postoperative tumor recurrence. The specific steps were as follows:
(1) extraction of different bacterial inner membranes specifically as follows: bacteria were amplified and respectively cultured in a culture medium until the OD600 value is 1.2; a lysozyme (lyse cell walls) was added to the final concentration of 2 mg/mL and co-incubated with the bacteria at 37°C for 2-3 h; the treated bacteria were centrifuged and a supernatant was discarded to obtain a protoplast; an extraction buffer was added and the mixture was centrifuged in a density gradient to obtain a bacterial inner membrane; and the bacterial inner membrane was stored at -80°C for later use after resuspension;
(2) male BALB/c mice were randomly divided into 7 groups with 8-10 mice per group, and the right back of each mouse was subcutaneously inoculated with 200,000 CT26 colon cancer cells to construct a mouse model of colon cancer (Day 0);
(3) the tumor growth of the mice was observed and recorded every other day, when the average tumor volume reached around 300 mm³, all the mice tumors were surgically resected, and the wound was sutured (the 7th day);
(4) extraction of cell membrane of tumor cell in CT26 colorectal cancer tissue: the obtained tumor tissue in (3) was primarily cut into pieces with scissors, a GBSS solution containing a collagenase IV (1.0 mg.mL⁻¹), DNase (0.1 mg.mL⁻¹) and a hyaluronidase (0.1 mg.mL⁻¹) was added, and the mixture was placed at 37°C for 15 min to digest the tumor tissue; the digested tissue was ground and filtered through a filter screen with a pore diameter of 0.22 µm, and a filtrate was centrifuged; and the centrifuged cells were resuspended, an extraction buffer containing mannitol, sucrose, and EGTA was added, and the cells were crushed by using a cell crusher under an ice bath and finally centrifuged to obtain a tumor cell membrane derived from a tumor tissue; and the cell membrane was resuspended, frozen and stored at -80°C;
(5) 3 mg of different bacterial inner membranes were respectively mixed with 1 mg of the extracted surgically resected tumor cell membrane TM in PBS, the mixture was shaken in a shaker at a constant temperature of 37°C for 15 min, and the mixed membrane solution was subjected to a cumulative back-and-forth extrusion for 13 times (one back and one forth are counted as 1 time) through a liposome extruder with a pore diameter of a filter membrane of 400 nm to obtain an HM hybrid membrane microparticle;
(6) 10 mg of PLGA was dissolved in 1 mL of methylene chloride and 0.2 mL of sterile distilled water was added; the mixture was ultrasonically emulsified at 25 W on ice for 3 min; then the emulsion was mixed with 2 mL of 1% of a sodium cholate solution (surfactant) and ultrasonically emulsified at 30 W on ice for 5 min; the emulsion was dropwise added into 10 mL of 0.5% of a sodium cholate solution and stirred at 25°C for 15 min; the emulsion was subjected a rotary evaporation at 37°C for 10 min; and the emulsion was centrifuged at 10,000 g for 15 min; and the obtained product was washed twice with sterile distilled water and resuspended to obtain a PLGA polymer solution;
(7) after 50 µL of 2 mg/mL of the HM hybrid membrane microparticle prepared in step (5) was mixed with 500 µL of 1 mg/mL of the PLGA prepared in step (6), the mixture was subjected to a cumulative back-and-forth extrusion for 13 times (one back and one forth are counted as 1 time) through a liposome extruder with a pore diameter of a filter membrane of 200 nm to obtain an HM-NPs hybrid membrane nanoparticle;
(8) the mice of each group were subcutaneously injected with SM-NPs, TM-NPs, Mix NPs, and HM-NPs (100 µg/mouse) on the 10th, 12nd and 16th day, respectively, and normal saline was used as a control (Control) group; and
(9) the growth of the tumors of the mice was observed and recorded every other day, when the tumor volume in the mice reached about 1,000 mm³, the mice were considered dead, and the specific time of death of each group of the mice was recorded.

The results show that the HM-NPs hybrid membrane nanoparticles prepared from different bacterial inner membranes in the present application have a uniform particle size (FIG. 49A), and have the potential of inhibiting the tumor recurrence of the mice after colon cancer surgery with a significantly improved inhibitory rate (FIG. 49B).

### Example 21

In the present application, an inhibition experiment of a liver cancer, a stomach cancer, a kidney cancer, a pancreatic cancer, an ovarian cancer, lymphoma, osteosarcoma, glioma, a prostate cancer, and melanoma was performed.

The experiment process is shown in FIG. 50A:
(1) 6-8 week-old C57BL/6J or Balb/C mice were randomly divided into 2 groups with 8-10 mice per group, and the right back of each mouse was subcutaneously inoculated with different tumor cells; the type and number of the tumor cells inoculated and the corresponding mouse strains were shown in Table 7; and different mouse models of postoperative tumor recurrence were constructed to evaluate an inhibitory effect of the present application on various tumors;
(2) when the average tumor volume reached around 300 mm³, 99% tumors were surgically resected, 1% tumor was remained, and the wound was sutured to simulate postoperative tumor recurrence (Day 0);
(3) the cut tumor blocks were ground and crushed, the tumor tissue was digested by using a mixed solution of collagenase, DNase and hyaluronidase at 37°C, the digested cell suspension passed through a filter screen and then centrifuged, the supernatant was removed, an extraction buffer was added, and the cells were resuspended to obtain a cell suspension; the cell suspension was ultrasonically crushed on an ice bath by using a cell crusher (35 W, 5 min); the crushed cell suspension was centrifuged (3,000 g, 5 min, 4°C); the supernatant of the previous step was centrifuged again (10,000 g, 10 min, 4°C); the centrifuged cell supernatant was added into an ultracentrifugation tube for ultracentrifugation (100,000 g, 2 h, 4°C); and after the ultracentrifugation, the supernatant was discarded, the obtained residue was resuspended with 1 mL of PBS to obtain a tumor cell membrane fragment TM derived from surgical resection, and the TM was stored at -80°C;
(5) various membrane nanoparticles were prepared according to the method in the example of the present application, pictures were taken under an electron microscope, and the hybrid membrane nanoparticles prepared from different tumor cells were characterized;
(6) the mice were immunized with the HM-NPs (100 µg/mouse) prepared from corresponding tumor membranes three times on the 3rd, 5th and 9th day after the surgery, respectively, and normal saline was as a control group (Control); and
(7) the growth of the tumors of the mice was observed and recorded, when the tumor volume in the mice reached about 1,000 mm³, the mice were considered dead, and the specific time of death of each group of the mice was recorded. A survival curve was drawn.

**Table 7 Cells and mice used for constructing different tumor models**

| Name of model | Name of cell | Inoculation number (cell/mouse) | Mouse strain |
|---|---|---|---|
| Liver cancer | Hepa 1-6 | 1 × 10⁵ | C57BL/6 female |
| Stomach cancer | MFC | 1 × 10⁷ | 615 female |
| Kidney cancer | RenCa | 1 × 10⁶ | BALB/c female |
| Pancreatic cancer | Panc02 | 1 × 10⁵ | C57BL/6 female |
| Ovarian cancer | ID8 | 1 × 10⁷ | C57BL/6 female |
| Lymphoma | EL4 | 1 × 10⁶ | C57BL/6 female |
| Osteosarcoma | LM8 | 1 × 10⁵ | C3h male |
| Glioma | GL261 | 1 × 10⁵ | C57BL/6 female |
| Prostate cancer | RM-1 | 1 × 10⁶ | C57BL/6 male |

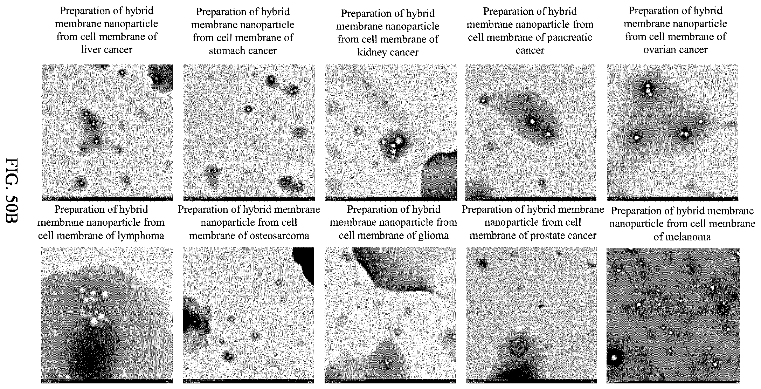

As shown in FIG. 50B, different tumor cells can be prepared with the *Escherichia coli* inner membrane into core-shell hybrid membrane nanoparticles with a uniform size. This proves that the present application can be used to prepare the hybrid membrane nanoparticles containing a liver cancer cell, a stomach cancer cell, a kidney cancer cell, a pancreatic cancer cell, an ovarian cancer cell, a lymphoma cell, an osteosarcoma cell, a glioma cell, a prostate cancer cell, and a melanoma cell.

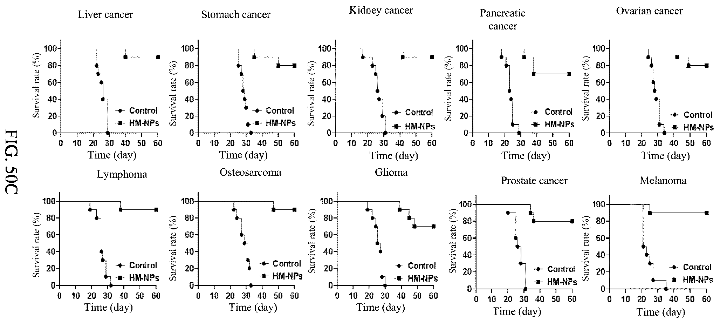

As shown in FIG. 50C, in the corresponding tumor models, compared with the control group (Control) without any treatment, the hybrid membrane nanoparticles vaccines (HM-NPs) all significantly prolong the survival period of different tumor-bearing mice.

### Example 22

In the present application, the hybrid membrane is used to wrap the PLGA polymer. The surface potential of the outer shell of the hybrid membrane can reach -50 mV after the mixture passes through a liposome extruder with a pore diameter of 200 nm. But the surface potential of the outer shell of the hybrid membrane can reach +50 mV after a cationic liposome is added into the hybrid membrane. The result is shown in FIG. 51.

## Claims

1. A pharmaceutical combination, wherein the pharmaceutical combination comprises a first membrane component and a second membrane component, the first membrane component comprises a membrane derived from an inner membrane of a bacterium, the second membrane component comprises a membrane derived from a tumor cell, and the first membrane component and the second membrane component are fused to form an outer shell.

2. The pharmaceutical combination according to claim 1, wherein the tumor cell is a solid tumor cell, preferably is selected from the group consisting of: a breast cancer cell, a colon cancer cell, a liver cancer cell, a stomach cancer cell, a kidney cancer cell, a pancreatic cancer cell, an ovarian cancer cell, a lymphoma cell, an osteosarcoma cell, a glioma cell, a prostate cancer cell, and a melanoma cell.

3. The pharmaceutical combination according to claim 1, wherein the second membrane component comprises a component with immunogenicity; optionally, wherein the second membrane component further comprises a protein or a functionally active fragment thereof selected from the following group consisting of: an antigen peptide transporter 1, an H-2 class II histocompatibility antigen γ chain, a protein tyrosine kinase SYK, a high affinity immunoglobulin epsilon receptor subunit γ, a Ras-related C3 botulinum toxin substrate 2, a protein tyrosine kinase BTK, a protein tyrosine phosphatase receptor type C, a Na⁺/K⁺-ATPase, ATP5A (IM CVa), a ubiquinol-cytochrome C reductase core protein I (IM Core I), a VDAC1/porin (OM Porin), a matrix cyclophilin D (Matrix CypD), an intermembrane space cytochrome C (IMS Cytc), a basement membrane-specific heparan sulfate proteoglycan core protein (HSPG2), a plasma membrane calcium-transporting ATPase 1 (ATP2b1), an endoplasmic reticulum membrane protein complex subunit 1 (EMC1), a transmembrane protein 43 (TMEM43), a vesicular integral-membrane protein VIP36 (LMAN2), a vesicle-associated membrane protein-associated protein A (VAPA), a transmembrane 9 superfamily member 2 (TM9SF2), a transmembrane emp24 domain-containing protein 10 (TMED10), an adipocyte plasma membrane associated protein (APAMP), a synaptic vesicle membrane protein VAT (VAT1), a nuclear pore membrane glycoprotein 210 (NUP210), a plasma membrane calcium-transporting ATPase 4 (ATP2b4), a plasma membrane calcium-transporting ATPase 2 (ATP2b2), an erythrocyte band 7 integral membrane protein (STOM), a transmembrane emp24 domain-containing protein 9 (TMED9), a mitochondrial import inner membrane translocase subunit TIM44 (TIMM44), a progesterone receptor membrane component 1 (PGRMC1), an endoplasmic reticulum membrane protein complex subunit 3 (EMC3), a vesicle-associated membrane-protein-associated protein B (VAPB), a membrane primary amine oxidase (AOC3), a vesicle-associated membrane protein 7 (VAMP7), a Golgi membrane protein 4 (GOLIM4), a progesterone receptor membrane component 2 (PGRMC2), a transmembrane 9 superfamily member 3 (TM9SF3), a transmembrane 9 superfamily member 4 (TM9SF4), an endoplasmic reticulum membrane protein complex subunit 2 (EMC2), a mitochondrial import inner membrane translocase subunit TIM50 (TIMM50), a peroxisomal membrane protein 11B (PEX11b), a transmembrane emp24 domain-containing protein 2 (TMED2), a secretory carrier membrane protein 3 (SCAMP3), a thioredoxin-related transmembrane protein 4 (TMX4), a peroxisomal membrane protein PMP34 (SLC25a17), a peroxisomal membrane protein PEX14 (PEX14), an endoplasmic reticulum membrane protein complex subunit 8 (EMC8), an interferon-induced transmembrane protein 3 (IFITM3), a lysosome-associated membrane glycoprotein 2 (LAMP2), a thioredoxin-related transmembrane protein 2 (TMX2), a vesicle-associated membrane protein 3 (VAMP3), a lysosome-associated membrane glycoprotein 1 (LAMP1), a mitochondrial import inner membrane translocase subunit TIM8 A (TIMM8a1), a lysosomal integral membrane protein-2 (SCARB2), an Ig gamma-2A chain C region (IGHG2a), a transmembrane 9 superfamily member 1 (TM9SF1), an inner nuclear membrane protein Man1 (LEMD3), a transmembrane emp24 domain-containing protein 4 (TMED4), a Thy-1 membrane glycoprotein (Thy1), a mitochondrial import inner membrane translocase subunit TIM23 (TIMM23), a mitochondrial import inner membrane translocase subunit TIM9 (TIMM9), a mitochondrial import inner membrane translocase subunit TIM13 (TIMM13), a secretory carrier membrane protein 2 (SCAMP2), a secretory carrier membrane protein 1 (SCAMP1), an integrin membrane protein 2B (ITM2b), a mitochondrial import inner membrane translocase subunit TIM10 (TIMM10), a vacuole membrane protein 1 (VMP1), a growth hormone-inducible transmembrane protein (GHITM), a membrane protein with a death domain (NRADD), a vesicle-associated membrane protein 8 (VAMP8), a transmembrane anterior posterior transformation 1 (TAPT1), a mitochondrial intermembrane space import and assembly protein 40 (CHCHD4), a glycosylated lysosomal membrane protein (GLMP), a membrane-spanning 4-domains, subfamily A, member 6D (MS4A6D), a translocation associated membrane protein 1 (TRAM1), an interferon-induced transmembrane protein 2 (IFITM2), a sarcolemma associated protein (SLMAP), a membrane magnesium transporter 1 (MMGT1), a nuclear envelope pore membrane protein POM 121 (POM121), a transmembrane protein C16orf54 homolog (AI467606), a vacuolar ATPase assembly integral membrane protein VMA21 (VMA21), an epithelial membrane protein 1 (EMP1), a membrane-associated phosphatidylinositol transfer protein 1 (PITPNM1), a small integral membrane protein 15 (SMIM15), a nuclear envelope integral membrane protein 1 (NEMP1), an integral membrane protein GPR180 (GPR180), a secretory carrier membrane protein 4 (SCAMP4), a translocation associated membrane protein 2 (TRAM2), a transmembrane and coiled-coil domain family 3 (TMCC3), a stromal membrane-associated protein 1 (SMAP1), a neuronal membrane glycoprotein M6-b (GPM6b), an epithelial membrane protein 2 (EMP2), a Golgi membrane protein 1 (GOLM1), an SID1 transmembrane family member 2 (SIDT2), a trans-Golgi network integral membrane protein 2 (TGOLN2), a Golgi apparatus membrane protein TVP23 homolog B (FAM18b), a mitochondrial inner membrane protein OXA1L (OXA1L), an osteopetrosis-associated transmembrane protein 1 (OSTM1), a peroxisomal membrane protein PEX13 (PEX13), a single-pass membrane and coiled-coil domain-containing protein 1 (SMCO1), a distal membrane arm assembly complex 1 (DMAC1), a secretory carrier membrane protein 5 (SCAMP5), a cystic fibrosis transmembrane conductance regulator (CFTR), an osteoclast stimulatory transmembrane protein (OCSTAMP), a fat storage-inducing transmembrane protein 2 (FITM2), and a transmembrane channel-like protein 5 (TMC5), and any combination of the above.

4. The pharmaceutical combination according to claim 1, wherein the bacterium comprises a gram-negative bacterium and/or a gram-positive bacterium.

5. The pharmaceutical combination according to claim 1, wherein the the bacterium is selected from the following group consisting of: *Escherichia, Staphylococcus, Bacillus, Lactobacillus, Klebsiella, Brucella, Proteus, Acinetobacter,* and *Pseudomonas.*

6. The pharmaceutical combination according to claim 1, wherein the inner membrane of the bacterium comprises a protein or a functionally active fragment thereof selected from the following group consisting of: a cell division protein FtsZ, an inner membrane protein YhcB, an inner membrane protein transposase YidC, a cell division protein NlpI, an ABC transporter MsbA, an inner membrane transporter TatA, an intermembrane phospholipid transport system lipoprotein MlaA, an inner membrane protein TolQ, an intermembrane transport lipoprotein PqiC, an outer membrane protein TolC, an outer membrane usher protein FimD, an outer membrane porin OmpC, a transmembrane transport protein PqiB, a major outer membrane lipoprotein Lpp, a membrane-bound lytic murein transglycosylase MltB, a UPF0194 membrane protein YbhG, a putative membrane protein IgaA homolog, an intermembrane phospholipid transport system lipoprotein MlaA, an inner membrane protein YejM, a membrane-bound lytic murein transglycosylase MltA, an intermembrane phospholipid transport system binding protein MlaC, an inner membrane protein YlaC, an intermembrane transport protein YebT, a membrane-bound lytic murein transglycosylase MltC, an intermembrane phospholipid transport system ATP-binding protein MlaF, an intermembrane phospholipid transport system binding protein MlaD, an inner membrane protein YqjE, a UPF0053 inner membrane protein YfjD, a UPF0053 inner membrane protein YoaE, an inner membrane lysis murein transglycosylase A, a UPF0394 inner membrane protein YedE, an intermembrane phospholipid transport system binding protein MlaB, an inner membrane protein YccF, an intermembrane phospholipid transport system permease protein MlaE, an inner membrane protein YedI, an inner membrane protein YgaP, a transmembrane transport protein PqiA, a UPF0056 membrane protein YhcE, an inner membrane protein YbhL, an inner membrane protein YhjD, an inner membrane transporter YbaT, an inner membrane protein YjjP, an inner membrane protein YhaH, an inner membrane protein YbjJ, an inner membrane transporter YqeG, a UPF0053 inner membrane protein YtfL, an arsenical pump membrane protein ArsB, an inner membrane protein YpjD, a membrane-bound lysozyme inhibitor of C-type lysozyme MliC, a UPF0283 membrane protein YcjF, a UPF0259 membrane protein YciC, an inner membrane protein YgfX, an inner membrane protein YbbJ, a lipoprotein-releasing system transmembrane protein LolE, an inner membrane protein YjcH, a protein-export membrane protein SecG, an inner membrane protein YfdC, a UPF0324 inner membrane protein YeiH, a UPF0266 membrane protein YobD, a TVP38/TMEM64 family inner membrane protein YdjZ, a membrane-associated protein UidC, an inner membrane protein YbiR, an inner membrane protein YhiM, a membrane transporter protein YfcA, an inner membrane protein YdgK, a membrane-bound lytic murein transglycosylase F, a multidrug resistance outer membrane protein MdtP, a UPF0208 membrane protein YfbV, a peptidoglycan-associated lipoprotein, a lipoprotein YiaD, a lipoprotein YeaY, an apolipoprotein N-acyltransferase, a D-methionine-binding lipoprotein MetQ, a lipoprotein GfcD, a lipoprotein YbjP, a lipoprotein YgeR, a lipoprotein-releasing system ATP-binding protein LolD, an osmotically-inducible lipoprotein OsmE, an osmotically-inducible lipoprotein OsmB, a lipoprotein YdcL, a lipoprotein YajG, a lipoprotein NlpE, a phosphatidylglycerol:prolipoprotein diacylglyceryl transferase, a lipoprotein YghJ, a lipoprotein YedD, a lipoprotein YifL, a lipoprotein YgdI, a lipoprotein YbaY, a lipoprotein signal peptidase, a lipoprotein YceB, a lipoprotein YajI, and an inner membrane protein YebE, and any combination of the above.

7. The pharmaceutical combination according to claim 1, wherein the pharmaceutical combination further comprises an inner core; optionally, wherein the inner core comprises a substance selected from the following group consisting of: a poly(lactic-co-glycolic acid) (PLGA), a metal-organic framework (MOF), polycaprolactone (PCL), polyamide-amine (PAMAM), a carbon nanotube, graphene, a gold nanoparticle, a mesoporous silica nanoparticle, an iron oxide nanoparticle, a silver nanoparticle, a tungsten nanoparticle, a manganese nanoparticle, a platinum nanoparticle, a quantum dot, an alumina nanoparticle, a hydroxyapatite nanoparticle, a lipid nanoparticle (LNP), a DNA nanostructure, a nano hydrogel, a rare earth fluoride nanocrystal, and a NaYF₄ nanoparticle, and any combination of the above; optionally, wherein the inner core comprises a substance selected from the following group consisting of: an immune adjuvant, an immune checkpoint inhibitor, a nucleic acid molecule, and a chemotherapeutic agent, and any combination of the above.

8. The pharmaceutical combination according to claim 1, wherein the mass ratio of the first membrane component to the second membrane component in the pharmaceutical combination is 1:100, 1:75, 1:50, 1:25, 1:10, 1:5, 1:3, 1:1, 1:0, 0:1, 3:1, 5:1, 10:1, 25:1, 50:1, 75:1, or 100:1.

9. The pharmaceutical combination according to claim 1, wherein the pharmaceutical combination further comprises a substance selected from the following group consisting of: an immune adjuvant, an immune checkpoint inhibitor, a nucleic acid molecule, a chemotherapeutic agent, and a photosensitizer, and any combination of the above.

10. A vaccine, comprising the pharmaceutical combination according to any one of claims 1-9.

11. A kit, comprising the pharmaceutical combination according to any one of claims 1-9 and/or the vaccine according to claim 10.

12. The pharmaceutical combination according to any one of claims 1-9, the vaccine according to claim 10 and/or the kit according to claim 11, for use in activating an immune cell.

13. The pharmaceutical combination according to any one of claims 1-9, the vaccine according to claim 10 and/or the kit according to claim 11, for use in enhancing an innate immunity and/or a specific immune response, and/or preventing and/or treating a tumor.

14. The pharmaceutical combination for use according to claim 13 wherein the tumor is a solid tumor, preferably is selected from the following group consisting of: a breast tumor, a colon tumor, a liver tumor, a stomach tumor, a kidney tumor, a pancreatic tumor, an ovarian tumor, lymphoma, osteosarcoma, glioma, prostate cancer, and melanoma.

15. A tumor vaccine suitable for use in preventing postoperative recurrence of cancer, wherein the tumor vaccine comprises a hybrid cell membrane outer shell and an inner core material, and the hybrid cell membrane outer shell comprises a gram-negative bacterial inner membrane and a solid tumor cell membrane derived from surgical resection.

16. A method for preparing the tumor vaccine for preventing postoperative recurrence of cancer according to claim 15, wherein the preparation method comprises the following steps:
(1) respectively extracting a gram-negative bacterial inner membrane and a solid tumor cell membrane derived from surgical resection;
(2) mixing the gram-negative bacterial inner membrane and the solid tumor cell membrane derived from surgical resection extracted in step (1), and extruding the mixture with a liposome extruder to obtain a hybrid membrane nanoparticle; and
(3) mixing the hybrid membrane microparticle obtained in step (2) with a nanoparticle inner core and extruding the mixture with the liposome extruder to obtain the tumor vaccine for preventing postoperative recurrence of cancer.

17. The tumor vaccine suitable for preventing postoperative recurrence of cancer according to claim 15, for use in enhancing innate and specific immune responses of a body.

## Patentansprüche

1. Pharmazeutische Kombination, wobei die pharmazeutische Kombination eine erste Membrankomponente und eine zweite Membrankomponente umfasst, die erste Membrankomponente eine Membran umfasst, die von einer Innenmembran eines Bakteriums stammt, die zweite Membrankomponente eine Membran umfasst, die aus einer Tumorzelle stammt, und die erste Membrankomponente und die zweite Membrankomponente fusioniert sind, um eine Außenhülle auszubilden.

2. Pharmazeutische Kombination nach Anspruch 1, wobei die Tumorzelle eine Zelle eines soliden Tumors ist, die vorzugsweise aus der Gruppe ausgewählt ist, bestehend aus: einer Brustkrebszelle, einer Dickdarmkrebszelle, einer Leberkrebszelle, einer Magenkrebszelle, einer Nierenkrebszelle, einer Bauchspeicheldrüsenkrebszelle, einer Eierstockkrebszelle, einer Lymphomzelle, einer Osteosarkomzelle, einer Gliomzelle, einer Prostatakrebszelle und einer Melanomzelle.

3. Pharmazeutische Kombination nach Anspruch 1, wobei die zweite Membrankomponente eine Komponente mit Immunogenität umfasst; optional, wobei die zweite Membrankomponente ferner ein Protein oder ein funktionell aktives Fragment davon umfasst, das aus der folgenden Gruppe ausgewählt ist, bestehend aus: einem Antigen-Peptid-Transporter 1, einer H-2-Klasse-II-Histokompatibilitätsantigen-γ-Kette, einer Protein-Tyrosin-Kinase SYK, einer hochaffinen Immunglobulin-Epsilon-Rezeptor-Untereinheit γ, einem Rasverwandten C3-Botulinumtoxin-Substrat 2, einer Protein-Tyrosin-Kinase BTK, einem Protein-Tyrosin-Phosphatase-Rezeptor Typ C, einer Na⁺/K⁺-ATPase, ATP5A (IM CVa), einem Ubichinol-Cytochrom-C-Reduktase-Kernprotein I (IM Core I), einem VDAC1/Porin (OM Porin), einem Matrix-Cyclophilin D (Matrix CypD), einem Intermembranraum-Cytochrom C (IMS Cytc), einem Basalmembran-spezifischen Heparansulfat-Proteoglykan-Kernprotein (HSPG2), einer Plasmamembran-Calcium-transportierenden ATPase 1 (ATP2b1), einer endoplasmatisches-Retikulum-Membranproteinkomplex-Untereinheit 1 (EMC1), einem Transmembranprotein 43 (TMEM43), einem vesikulären integralen Membranprotein VIP36 (LMAN2), einem Vesikel-assoziierten Membranprotein-assoziierten Protein A (VAPA), einem Transmembran-9-Superfamilien-Mitglied 2 (TM9SF2), einem Transmembran-emp24-Domänen-haltigen Protein 10 (TMED10), einem Adipozyten-Plasmamembran-assoziierten Protein (APAMP), einem synaptischen Vesikelmembranprotein (VAT1), einem nuklearen Porenmembranglykoprotein 210 (NUP210), einer Plasmamembran-Calcium-transportierenden ATPase 4 (ATP2b4), einer Plasmamembran-Calcium-transportierenden ATPase 2 (ATP2b2), einem integralen Erythrozyten-Bande-7-Membranprotein (STOM), einem Transmembran-emp24-Domänen-haltigen Protein 9 (TMED9), einem Mitochondrialimport-Innenmembran-Translokase-Untereinheit TIM44 (TIMM44), einer Progesteronrezeptor-Membrankomponente 1 (PGRMC1), einem endoplasmatisches-Retikulum-Membranproteinkomplex-Untereinheit 3 (EMC3), einem Vesikel-assoziierten Membranprotein-assoziierten Protein B (VAPB), einer primären Membran-Aminoxidase (AOC3), einem Vesikel-assoziierten Membranprotein 7 (VAMP7), einem Golgi-Membranprotein 4 (GOLIM4), einer Progesteronrezeptor-Membrankomponente 2 (PGRMC2), einem Transmembran-9-Superfamilienmitglied 3 (TM9SF3), einem Transmembran-9-Superfamilienmitglied 4 (TM9SF4), einer endoplasmatisches-Retikulum-Membranproteinkomplex-Untereinheit 2 (EMC2), einer Mitochondrialimport-Innenmembran-Translokase-Untereinheit TIM50 (TIMM50), einem peroxisomalen Membranprotein 11B (PEX11b), einem Transmembran-emp24-Domänen-haltigen Protein 2 (TMED2), einem sekretorischer-Carrier-Membranprotein 3 (SCAMP3), einem Thioredoxin-verwandten Transmembranprotein 4 (TMX4), einem peroxisomalen Membranprotein PMP34 (SLC25a17), einem peroxisomalen Membranprotein PEX14 (PEX14), einer endoplasmatisches-Retikulum-Membranproteinkomplex-Untereinheit 8 (EMC8), einem Interferon-induzierten Transmembranprotein 3 (IFITM3), einem Lysosom-assoziierten Membranglykoprotein 2 (LAMP2), einem Thioredoxin-verwandten Transmembranprotein 2 (TMX2), einem Vesikel-assoziierten Membranprotein 3 (VAMP3), einem Lysosom-assoziierten Membranglykoprotein 1 (LAMP1), einer Mitochondriaimport-Innenmembran-Translokase-Untereinheit TIM8 A (TIMM8a1), einem lysosomalen integralen Membranprotein-2 (SCARB2), einer Ig-gamma-2A-Ketten-C-Region (IGHG2a), einem Transmembran-9-Superfamilien-Mitglied 1 (TM9SF1), einem Innenkernmembranprotein Man1 (LEMD3), einem Transmembran-emp24-Domänen-haltigen Protein 4 (TMED4), einem Thy-1-Membranglykoprotein (Thy1), einer Mitochondrialimport-Innenmembran-Translokase-Untereinheit TIM23 (TIMM23), einer Mitochondrialimport-Innenmembran-Translokase-Untereinheit TIM9 (TIMM9), einer Mitochondrialimport-Innenmembran-Translokase-Untereinheit TIM13 (TIMM13), einem sekretorischen Carrier-Membranprotein 2 (SCAMP2), einem sekretorischen Carrier-Membranprotein 1 (SCAMP1), einem Integrin-Membranprotein 2B (ITM2b), einer Mitochondrialimport-Innenmembran-Translokase-Untereinheit TIM10 (TIMM10), einem Vakuolen-Membranprotein 1 (VMP1), einem Wachstumshormon-induzierbaren Transmembranprotein (GHITM), einem Membranprotein mit einer Todesdomäne (NRADD), einem Vesikel-assoziierten Membranprotein 8 (VAMP8), einer Transmembran-Anterior-Posterior-Transformation-1 (TAPT1), einem Mitochondrialintermembranraum-Import- und Assemblierungsprotein 40 (CHCHD4), einem glykosylierten lysosomalen Membranprotein (GLMP), einem membranspannenden 4-Domänen-Protein, Unterfamilie A, Mitglied 6D (MS4A6D), einem Translokations-assoziierten Membranprotein 1 (TRAM1), einem Interferon-induzierten Transmembranprotein 2 (IFITM2), einem Sarkolemm-assoziierten Protein (SLMAP), einem Membran-Magnesiumtransporter 1 (MMGT1), einem Zellkernhüllen-Porenmembranprotein POM 121 (POM121), einem Transmembranprotein-C16orf54-Homolog (AI467606), einem integralem Membranprotein der vakuolären ATPase-Assemblierung VMA21 (VMA21), einem epithelialen Membranprotein 1 (EMP1), einem Membran-assoziierten Phosphatidylinositol-Transferprotein 1 (PITPNM1), einem kleinen integralen Membranprotein 15 (SMIM15), einem integralen Zellkernhüllen-Membranprotein 1 (NEMP1), einem integralen Membranprotein GPR180 (GPR180), einem sekretorischen Carrier-Membranprotein 4 (SCAMP4), einem Translokation-assoziierten Membranprotein 2 (TRAM2), einer Transmembran- und Coiled-Coil-Domänen-Familie 3 (TMCC3), einem stromalen Membran-assoziierten Protein 1 (SMAP1), einem neuronalen Membranglykoprotein M6-b (GPM6b), einem epithelialen Membranprotein 2 (EMP2), einem Golgi-Membranprotein1 (GOLM1), einem SID1-Transmembranfamilienmitglied 2 (SIDT2), einem integralen Membranprotein 2 des Trans-Golgi-Netzwerks (TGOLN2), einem Golgi-Apparat-Membranprotein-TVP23-Homolog B (FAM18b), einem Mitochondrien-Innenmembranprotein OXA1L (OXA1L), einem Osteopetrose-assoziierten Transmembranprotein 1 (OSTM1), einem peroxisomalen Membranprotein PEX13 (PEX13), einem Single-Pass-Membran- und Coiled-Coil-Domänen-haltigen Protein 1 (SMCO1), einem distalen Membranarm-Assemblierungskomplex 1 (DMAC1), einem sekretorischen Carrier-Membranprotein 5 (SCAMP5), einem Cystic-Fibrosis-Transmembran-Leitfähigkeitsregulator (CFTR), einem Osteoklasten-stimulierenden Transmembranprotein (OCSTAMP), einem Fettspeicher-induzierenden Transmembranprotein 2 (FITM2) und einem Transmembrankanal-ähnlichen Protein 5 (TMC5) und einer beliebigen Kombination der Vorstehenden.

4. Pharmazeutische Kombination nach Anspruch 1, wobei das Bakterium ein gramnegatives Bakterium und/oder ein grampositives Bakterium umfasst.

5. Pharmazeutische Kombination nach Anspruch 1, wobei das Bakterium aus der folgenden Gruppe ausgewählt ist, bestehend aus: *Escherichia, Staphylococcus, Bacillus, Lactobacillus, Klebsiella, Brucella, Proteus, Acinetobacter und Pseudomonas.*

6. Pharmazeutische Kombination nach Anspruch 1, wobei die Innenmembran des Bakteriums ein Protein oder ein funktionell aktives Fragment davon umfasst, das aus der folgenden Gruppe ausgewählt ist, bestehend aus: einem Zellteilungsprotein FtsZ, einem Innenmembranprotein YhcB, einer Innenmembranprotein-Transposase YidC, einem Zellteilungsprotein Nlpl, einem ABC-Transporter MsbA, einem Innenmembrantransporter TatA, einem Intermembran-Phospholipid-Transportsystem-Lipoprotein MlaA, einem Innenmembranprotein TolQ, einem Intermembran-Transport-Lipoprotein PqiC, einem Außenmembranprotein TolC, einem Außenmembran-Usher-Protein FimD, einem Außenmembran-Porin OmpC, einem Transmembran-Transportprotein PqiB, einem Haupt-Außenmembran-Lipoprotein Lpp, einer membrangebundenen lytischen Murein-Transglycosylase MltB, einem UPF0194-Membranprotein YbhG, einem putativen Membranprotein IgaAHomolog, einem Intermembran-Phospholipid-Transportsystem-Lipoprotein MlaA, einem Innenmembranprotein YejM, einer membrangebundenen lytischen Murein-Transglycosylase MltA, einem Intermembran-Phospholipid-Transportsystem-Bindungsprotein MlaC, einem Innenmembranprotein YlaC, einem Intermembran-Transportprotein YebT, einer membrangebundenen lytischen Murein-Transglycosylase MltC, einem Intermembran-Phospholipid-Transportsystem-ATP-Bindungsprotein MlaF, einem Intermembran-Phospholipid-Transportsystem-Bindungsprotein MlaD, einem Innenmembranprotein YqjE, einem UPF0053-Innenmembranprotein YfjD, einem UPF0053-Innenmembranprotein YoaE, einem Innenmembran-Lyse-Murein-Transglycosylase A, einem UPF0394-Innenmembranprotein YedE, einem Intermembran-Phospholipid-Transportsystem-Bindungsprotein MlaB, einem Innenmembranprotein YccF, einem Intermembran-Phospholipid-Transportsystem-Permease-Protein MlaE, einem Innenmembranprotein Yedl, einem Innenmembranprotein YgaP, einem Transmembrantransportprotein PqiA, einem UPF0056-Membranprotein YhcE, einem Innenmembranprotein YbhL, einem Innenmembranprotein YhjD, einem Innenmembrantransporter YbaT, einem Innenmembranprotein YjjP, einem Innenmembranprotein YhaH, einem Innenmembranprotein YbjJ, einem Innenmembrantransporter YqeG, einem UPF0053-Innenmembranprotein YtfL, einem Arsenpumpen-Membranprotein ArsB, einem Innenmembranprotein YpjD, einem Membran-gebunden Lysozyminhibitor von C-Typ-Lysozym MIiC, einem UPF0283-Membranprotein YcjF, einem UPF0259-Membranprotein YciC, einem Innenmembranprotein YgfX, einem Innenmembranprotein YbbJ, einem Transmembranprotein des Lipoprotein-Freisetzungssystems LolE, einem Innenmembranprotein YjcH, einem Proteinexport-Membranprotein SecG, einem Innenmembranprotein YfdC, einem UPF0324-Innenmembranprotein YeiH, einem UPF0266-Membranprotein YobD, einem Innenmembranprotein der TVP38/TMEM64-Familie YdjZ, einem Membran-assoziierten Protein UidC, einem Innenmembranprotein YbiR, einem Innenmembranprotein YhiM, einem Membrantransporterprotein YfcA, einem Innenmembranprotein YdgK, einer membrangebundenen lytischen Mureintransglycosylase F, einem Multidrug-Resistenz-Außenmembranprotein MdtP, einem UPF0208-Membranprotein YfbV, einem Peptidoglycan-assoziierten Lipoprotein, einem Lipoprotein YiaD, einem Lipoprotein YeaY, einer Apolipoprotein-N-Acyltransferase, einem D-Methionin-bindenden Lipoprotein MetQ, einem Lipoprotein GfcD, einem Lipoprotein YbjP, einem Lipoprotein YgeR, einem ATP-bindenden Protein des Lipoprotein-freisetzenden Systems LolD, einem osmotisch induzierbaren Lipoprotein OsmE, einem osmotisch induzierbaren Lipoprotein OsmB, einem Lipoprotein YdcL, einem Lipoprotein YajG, einem Lipoprotein NlpE, einer Phosphatidylglycerol:Prolipoprotein-Diacylglyceryltransferase, einem Lipoprotein YghJ, einem Lipoprotein YedD, einem Lipoprotein YifL, einem Lipoprotein Ygdl, einem Lipoprotein YbaY, einer Lipoprotein-Signalpeptidase, einem Lipoprotein YceB, einen Lipoprotein Yajl und einem Innenmembranprotein YebE und jeder beliebigen Kombination der Vorstehenden.

7. Pharmazeutische Kombination nach Anspruch 1, wobei die pharmazeutische Kombination ferner einen Innenkern umfasst; optional, wobei der Innenkern eine Substanz umfasst, die aus der folgenden Gruppe ausgewählt ist, bestehend aus: einer Poly(milch-co-glykolsäure) (PLGA), einem metallorganischen Gerüst (MOF), Polycaprolacton (PCL), Polyamidamin (PAMAM), einer Kohlenstoffnanoröhre, Graphen, einem Goldnanopartikel, einem mesoporöses-Siliciumdioxid-Nanopartikel, einem Eisenoxidnanopartikel, einem Silbernanopartikel, einem Wolframnanopartikel, einem Mangannanopartikel, einem Platinnanopartikel, einem Quantenpunkt, einem Tonerdenanopartikel, einem Hydroxylapatitnanopartikel, einem Lipidnanopartikel (LNP), einer DNA-Nanostruktur, einem Nanohydrogel, einem Seltenerdfluoridnanokristall und einem NaYF₄-Nanopartikel und einer beliebigen Kombination der Vorstehenden; optional, wobei der Innenkern eine Substanz umfasst, die aus der folgenden Gruppe ausgewählt ist, bestehend aus: einem Immunadjuvans, einem Immun-Checkpoint-Inhibitor, einem Nukleinsäuremolekül und einem Chemotherapeutikum und einer beliebigen Kombination der Vorstehenden.

8. Pharmazeutische Kombination nach Anspruch 1, wobei das Massenverhältnis der ersten Membrankomponente zu der zweiten Membrankomponente in der pharmazeutischen Kombination 1 : 100, 1 : 75, 1 : 50, 1 : 25, 1 : 10, 1 : 5, 1 : 3, 1 : 1, 1 : 0, 0 : 1, 3 : 1, 5 : 1, 10 : 1, 25 : 1, 50 : 1, 75 : 1 oder 100 : 1 beträgt.

9. Pharmazeutische Kombination nach Anspruch 1, wobei die pharmazeutische Kombination ferner eine Substanz umfasst, die aus der folgenden Gruppe ausgewählt ist, bestehend aus: einem Immunadjuvans, einem Immun-Checkpoint-Inhibitor, einem Nukleinsäuremolekül, einem Chemotherapeutikum und einem Photosensibilisator und einer beliebigen Kombination der Vorstehenden.

10. Impfstoff, umfassend die pharmazeutische Kombination nach einem der Ansprüche 1 bis 9.

11. Kit, umfassend die pharmazeutische Kombination nach einem der Ansprüche 1 bis 9 und/oder den Impfstoff nach Anspruch 10.

12. Pharmazeutische Kombination nach einem der Ansprüche 1 bis 9, Impfstoff nach Anspruch 10 und/oder Kit nach Anspruch 11 zur Verwendung bei einem Aktivieren einer Immunzelle.

13. Pharmazeutische Kombination nach einem der Ansprüche 1 bis 9, Impfstoff nach Anspruch 10 und/oder Kit nach Anspruch 11 zur Verwendung bei einem Verstärken einer angeborenen Immunität und/oder einer spezifischen Immunantwort und/oder einem Vorbeugen und/oder Behandeln eines Tumors.

14. Pharmazeutische Kombination zur Verwendung nach Anspruch 13, wobei der Tumor ein solider Tumor ist, der vorzugsweise aus der folgenden Gruppe ausgewählt ist, bestehend aus: einem Brusttumor, einem Dickdarmtumor, einem Lebertumor, einem Magentumor, einem Nierentumor, einem Bauchspeicheldrüsentumor, einem Eierstocktumor, Lymphom, Osteosarkom, Gliom, Prostatakrebs und Melanom.

15. Tumorimpfstoff, der zur Verwendung bei dem Vorbeugen eines postoperativen Krebsrezidivs geeignet ist, wobei der Tumorimpfstoff eine Außenhybridzellmembranhülle und ein Innenkernmaterial umfasst, und die Außenhybridzellmembranhülle eine gramnegative bakterielle Innenmembran und eine Zellmembran des soliden Tumors, die aus einer chirurgischen Resektion stammt, umfasst.

16. Verfahren zum Herstellen des Tumorimpfstoffs zum Vorbeugen des postoperativen Krebsrezidivs nach Anspruch 15, wobei das Herstellungsverfahren die folgenden Schritte umfasst:
(1) jeweils Extrahieren einer gramnegativen bakteriellen Innenmembran und einer Zellmembran des soliden Tumors, die aus einer chirurgischen Resektion stammt;
(2) Mischen der gramnegativen bakteriellen Innenmembran und der Zellmembran des soliden Tumors, die aus einer chirurgischen Resektion stammt, die in Schritt (1) extrahiert wird, und Extrudieren der Mischung mit einem Liposomenextruder, um ein Hybridmembrannanopartikel zu erhalten; und
(3) Mischen des Hybridmembranmikropartikels, das in Schritt (2) erhalten wird, mit einem Nanopartikelinnenkern und Extrudieren der Mischung mit dem Liposomenextruder, um den Tumorimpfstoff zum Vorbeugen des postoperativen Krebsrezidivs zu erhalten.

17. Tumorimpfstoff, der zum Vorbeugen des postoperativen Krebsrezidivs nach Anspruch 15 geeignet ist, zur Verwendung bei dem Verstärken angeborener und spezifischer Immunantworten eines Körpers.

## Revendications

1. Combinaison pharmaceutique, dans laquelle la combinaison pharmaceutique comprend un premier composant membranaire et un second composant membranaire, le premier composant membranaire comprend une membrane dérivée d'une membrane interne d'une bactérie, le second composant membranaire comprend une membrane dérivée d'une cellule tumorale, et le premier composant membranaire et le second composant membranaire sont fusionnés pour former une enveloppe externe.

2. Combinaison pharmaceutique selon la revendication 1, dans laquelle la cellule tumorale est une cellule de tumeur solide, de préférence est choisie dans le groupe constitué de : cellule du cancer du sein, cellule du cancer du côlon, cellule du cancer du foie, cellule du cancer de l'estomac, cellule du cancer du rein, cellule du cancer du pancréas, cellule du cancer de l'ovaire, cellule de lymphome, cellule d'ostéosarcome, cellule de gliome, cellule du cancer de la prostate et cellule de mélanome.

3. Combinaison pharmaceutique selon la revendication 1, dans laquelle le second composant membranaire comprend un composant avec une immunogénicité ; éventuellement, dans laquelle le second composant membranaire comprend en outre une protéine ou un fragment fonctionnellement actif de celle-ci choisi dans le groupe suivant constitué de : transporteur de peptide antigénique 1, chaîne γ d'antigène d'histocompatibilité de classe II H-2, protéine tyrosine kinase SYK, sous-unité γ de récepteur epsilon d'immunoglobuline de haute affinité, substrat 2 de toxine botulique C3 lié à Ras, protéine tyrosine kinase BTK, récepteur de type C de protéine tyrosine phosphatase, Na⁺/K⁺ ATPase, ATP5A (IM CVa), protéine centrale I de l'ubiquinol-cytochrome c réductase (IM Core I), VDAC1/porine (OM Porin), cyclophiline D matricielle (Matrix CypD), cytochrome c de l'espace intermembranaire (IMS Cytc), protéine centrale de protéoglycane de sulfate d'héparane spécifique à la membrane basale (HSPG2), ATPase 1 transportant le calcium sur la membrane plasmique (ATP2b1), sous-unité 1 de complexe protéique de la membrane du réticulum endoplasmique (EMC1), protéine transmembranaire 43 (TMEM43), protéine membranaire intégrale d'une vésicule VIP36 (LMAN2), protéine A associée à une protéine membranaire associée à une vésicule (VAPA), membre 2 de la superfamille de la transmembrane 9 (TM9SF2), protéine 10 contenant le domaine transmembranaire emp24 (TMED10), protéine associée à la membrane plasmique de l'adipocyte (APAMP), protéine de la membrane de la vésicule synaptique VAT (VAT1), glycoprotéine 210 d'une membrane poreuse nucléaire (NUP210), ATPase 4 transportant le calcium d'une membrane plasmique (ATP2b4), ATPase 2 transportant le calcium d'une membrane plasmique (ATP2b2), protéine membranaire intégrale de bande d'érythrocytes 7 (STOM), protéine 9 contenant le domaine transmembranaire emp24 (TMED9), sous-unité de translocase de membrane interne d'importation mitochondriale TIM44 (TIMM44), membrane réceptrice de progestérone composant-1 (PGRMC1), sous-unité 3 de complexe protéique de membrane du réticulum endoplasmique (EMC3), protéine B associée à une protéine membranaire associée à une vésicule (VAPB), aminoxydase primaire membranaire (AOC3), protéine membranaire 7 associée à une vésicule (VAMP7), protéine membranaire 4 de Golgi (GOLIM4), membrane réceptrice de progestérone composant-2 (PGRMC2), membre 3 de la superfamille de la transmembrane 9 (TM9SF3), membre 4 de la superfamille de la transmembrane 9 (TM9SF4), sous-unité 2 du complexe protéique de la membrane du réticulum endoplasmique (EMC2), sous-unité de translocase de membrane interne d'importation mitochondriale TIM50 (TIMM50), protéine de membrane peroxysomale 11B (PEX11b), protéine 2 contenant le domaine transmembranaire emp24 (TMED2), protéine de membrane porteuse sécrétoire 3 (SCAMP3), protéine transmembranaire 4 liée à la thiorédoxine (TMX4), protéine de membrane peroxysomale PMP34 (SLC25a17), protéine de membrane peroxysomale PEX14 (PEX14), sous-unité 8 de complexe protéique de membrane du réticulum endoplasmique (EMC8), protéine transmembranaire 3 induite par l'interféron (IFITM3), glycoprotéine membranaire 2 associée au lysosome (LAMP2), protéine transmembranaire 2 liée à la thiorédoxine (TMX2), protéine membranaire 3 associée à la vésicule (VAMP3), glycoprotéine membranaire 1 associée au lysosome (LAMP1), sous-unité de translocase de membrane interne d'importation mitochondriale TIM8 A (TIMM8a1), protéine 2 de membrane intégrale lysosomale (SCARB2), région C de la chaîne Ig gamma-2A (IGHG2a), membre 1 de la superfamille de la transmembrane 9 (TM9SF1), protéine Man1 de membrane nucléaire interne (LEMD3), protéine 4 contenant le domaine transmembranaire emp24 (TMED4), glycoprotéine membranaire Thy-1 (Thy1), sous-unité de translocase de membrane interne d'importation mitochondriale TIM23 (TIMM23), sous-unité de translocase de membrane interne d'importation mitochondriale TIM9 (TIMM9), sous-unité de translocase de membrane interne d'importation mitochondriale TIM13 (TIMM13), protéine 2 de membrane porteuse sécrétoire (SCAMP2), protéine 1 de membrane porteuse sécrétoire (SCAMP1), protéine 2B de membrane d'intégrines (ITM2b), sous-unité de translocase de membrane interne d'importation mitochondriale TIM10 (TIMM10), protéine 1 de membrane de vacuole (VMP1), protéine transmembranaire inductible par l'hormone de croissance (GHITM), protéine membranaire avec un domaine de mort (NRADD), protéine membranaire 8 associée à une vésicule (VAMP8), transformation 1 antéro-postérieure transmembranaire (TAPT1), protéine 40 d'importation et d'assemblage de l'espace intermembranaire mitochondrial (CHCHD4), protéine de membrane lysosomale glycosylée (GLMP), membre 6D de sous-famille A de 4 domaines s'étendant sur une membrane (MS4A6D), protéine membranaire 1 associée à la translocation (TRAM1), protéine transmembranaire 2 induite par l'interféron (IFITM2), protéine associée au sarcolemme (SLMAP), transporteur 1 de magnésium membranaire (MMGT1), protéine de membrane poreuse d'enveloppe nucléaire POM 121 (POM121), homologue de protéine transmembranaire C16orf54 (AI467606), protéine membranaire intégrale d'assemblage d'ATPase vacuolaire VMA21 (VMA21), protéine 1 de membrane épithéliale (EMP1), protéine 1 de transfert de phosphatidylinositol associée à une membrane (PITPNM1), petite protéine membranaire intégrale 15 (SMIM15), protéine membranaire intégrale 1 d'enveloppe nucléaire (NEMP1), protéine membranaire intégrale GPR180 (GPR180), protéine 4 de membrane porteuse sécrétoire (SCAMP4), protéine 2 de membrane associée à la translocation (TRAM2), famille 3 de domaine transmembranaire et à superhélice (TMCC3), protéine 1 associée à la membrane stromale (SMAP1), glycoprotéine de membrane neuronale M6-b (GPM6b), protéine 2 de membrane épithéliale (EMP2), protéine 1 de membrane de Golgi (GOLM1), membre 2 de la famille de la transmembrane SID1 (SIDT2), protéine membranaire intégrale 2 du réseau trans-Golgi (TGOLN2), homologue B de la protéine de membrane TVP23 de l'appareil de Golgi (FAM18b), protéine de membrane interne mitochondriale OXA1L (OXA1L), protéine transmembranaire 1 associée à l'ostéopétrose (OSTM1), protéine de membrane peroxysomale PEX13 (PEX13), protéine 1 contenant une membrane à passage unique et un domaine à superhélice (SMCO1), complexe 1 d'assemblage de bras de membrane distal (DMAC1), protéine 5 de membrane porteuse sécrétoire (SCAMP5), régulateur de conductance transmembranaire de la mucoviscidose (CFTR), protéine transmembranaire stimulatrice des ostéoclastes (OCSTAMP), protéine transmembranaire 2 induisant le stockage des graisses (FITM2) et protéine 5 de type canal transmembranaire (TMC5), et combinaison quelconque des éléments précédents.

4. Combinaison pharmaceutique selon la revendication 1, dans laquelle la bactérie comprend une bactérie Gram négatif et/ou une bactérie Gram positif.

5. Combinaison pharmaceutique selon la revendication 1, dans laquelle la bactérie est choisie dans le groupe suivant constitué de *: Escherichia, Staphylococcus, Bacillus, Lactobacillus, Klebsiella, Brucella, Proteus, Acinetobacter et Pseudomonas.*

6. Combinaison pharmaceutique selon la revendication 1, dans laquelle la membrane interne de la bactérie comprend une protéine ou un fragment fonctionnellement actif de celle-ci choisi dans le groupe suivant constitué de : protéine de division cellulaire FtsZ, protéine de membrane interne YhcB, transposase de protéine de membrane interne YidC, protéine de division cellulaire Nlpl, transporteur ABC MsbA, transporteur de membrane interne TatA, lipoprotéine de système de transport de phospholipides intermembranaire MIaA, protéine de membrane interne TolQ, lipoprotéine de transport intermembranaire PqiC, protéine de membrane externe TolC, protéine de type Usher de membrane externe FimD, porine de membrane externe OmpC, protéine de transport transmembranaire PqiB, lipoprotéine de membrane externe majeure Lpp, transglycosylase de muréine lytique liée à la membrane MItB, protéine membranaire UPF0194 YbhG, homologue de protéine membranaire putative IgaA, lipoprotéine de système de transport de phospholipides intermembranaire MIaA, protéine de membrane interne YejM, transglycosylase de muréine lytique liée à une membrane MItA, protéine de liaison de système de transport de phospholipides intermembranaire MIaC, protéine de membrane interne YlaC, protéine de transport intermembranaire YebT, transglycosylase de muréine lytique liée à une membrane MItC, protéine de liaison à l'ATP de système de transport de phospholipides intermembranaire MIaF, protéine de liaison de système de transport de phospholipides intermembranaire MIaD, protéine de membrane interne YqjE, protéine de membrane interne UPF0053 YfjD, protéine de membrane interne UPF0053 YoaE, transglycosylase de muréine de lyse de membrane interne A, protéine de membrane interne UPF0394 YedE, protéine de liaison de système de transport de phospholipides intermembranaire MlaB, protéine de membrane interne YccF, protéine de perméase de système de transport de phospholipides intermembranaire MIaE, protéine de membrane interne Yedl, protéine de membrane interne YgaP, protéine de transport transmembranaire PqiA, protéine de membrane UPF0056 YhcE, protéine de membrane interne YbhL, protéine de membrane interne YhjD, transporteur de membrane interne YbaT, protéine de membrane interne YjjP, protéine de membrane interne YhaH, protéine de membrane interne YbjJ, transporteur de membrane interne YqeG, protéine de membrane interne UPF0053 YtfL, protéine de membrane de pompe à arsenic ArsB, protéine de membrane interne YpjD, inhibiteur de lysozymes lié à la membrane de lysozymes de type C MliC, protéine membranaire UPF0283 YcjF, protéine membranaire UPF0259 YciC, protéine de membrane interne YgfX, protéine de membrane interne YbbJ, protéine transmembranaire de système de libération de lipoprotéines LolE, protéine de membrane interne YjcH, protéine de membrane d'exportation de protéines SecG, protéine de membrane interne YfdC, protéine de membrane interne UPF0324 YeiH, protéine de membrane interne UPF0266 YobD, protéine de membrane interne de la famille TVP38ITMEM64 YdjZ, protéine associée à une membrane UidC, protéine de membrane interne YbiR, protéine de membrane interne YhiM, protéine de transporteur membranaire YfcA, protéine de membrane interne YdgK, transglycosylase de muréine lytique liée à une membrane F, protéine de membrane externe multirésistante aux médicaments, MdtP, protéine membranaire UPF0208 YfbV, lipoprotéine associée au peptidoglycane, lipoprotéine YiaD, lipoprotéine YeaY, apolipoprotéine N-acyltransférase, lipoprotéine de liaison à la D-méthionine MetQ, lipoprotéine GfcD, lipoprotéine YbjP, lipoprotéine YgeR, protéine de liaison à l'ATP de système de libération des lipoprotéines LoID, lipoprotéine inductible par osmose OsmE, lipoprotéine inductible par osmose OsmB, lipoprotéine YdcL, lipoprotéine YajG, lipoprotéine NlpE, transférase de diacylglycéryl phosphatidylglycérol/prolipoprotéine, lipoprotéine YghJ, lipoprotéine YedD, lipoprotéine YifL, lipoprotéine Ygdl, lipoprotéine YbaY, peptidase signal de lipoprotéine, lipoprotéine YceB, lipoprotéine Yajl, et protéine de membrane interne YebE, et combinaison quelconque des éléments précédents.

7. Combinaison pharmaceutique selon la revendication 1, dans laquelle la combinaison pharmaceutique comprend en outre un noyau interne ; éventuellement, dans laquelle le noyau interne comprend une substance choisie dans le groupe suivant constitué de : acide poly(lactique-co-glycolique) (PLGA), structure organométallique (MOF), polycaprolactone (PCL), polyamide-amine (PAMAM), nanotube de carbone, graphène, nanoparticule d'or, nanoparticule de silice mésoporeuse, nanoparticule d'oxyde de fer, nanoparticule d'argent, nanoparticule de tungstène, nanoparticule de manganèse, nanoparticule de platine, boîte quantique, nanoparticule d'alumine, nanoparticule d'hydroxyapatite, nanoparticule lipidique (LNP), nanostructure d'ADN, nano-hydrogel, nanocristal de fluorure de terres rares et nanoparticule de NaYF₄ et combinaison quelconque des éléments précédents ; éventuellement, dans laquelle le noyau interne comprend une substance choisie dans le groupe suivant constitué de : adjuvant immunitaire, inhibiteur de point de contrôle immunitaire, molécule d'acide nucléique et agent chimiothérapeutique, et combinaison quelconque des éléments précédents.

8. Combinaison pharmaceutique selon la revendication 1, dans laquelle le rapport massique du premier composant membranaire au second composant membranaire dans la combinaison pharmaceutique est de 1:100, 1:75, 1:50, 1:25, 1:10, 1:5, 1:3, 1:1, 1:0, 0:1, 3:1, 5:1, 10:1, 25:1, 50:1, 75:1 ou 100:1.

9. Combinaison pharmaceutique selon la revendication 1, dans laquelle la combinaison pharmaceutique comprend en outre une substance choisie dans le groupe suivant constitué de : adjuvant immunitaire, inhibiteur de point de contrôle immunitaire, molécule d'acide nucléique, agent chimiothérapeutique et photosensibilisateur, et combinaison quelconque des éléments précédents.

10. Vaccin, comprenant la combinaison pharmaceutique selon l'une quelconque des revendications 1 à 9.

11. Trousse, comprenant la combinaison pharmaceutique selon l'une quelconque des revendications 1 à 9 et/ou le vaccin selon la revendication 10.

12. Combinaison pharmaceutique selon l'une quelconque des revendications 1 à 9, vaccin selon la revendication 10 et/ou trousse selon la revendication 11, pour une utilisation dans l'activation d'une cellule immunitaire.

13. Combinaison pharmaceutique selon l'une quelconque des revendications 1 à 9, vaccin selon la revendication 10 et/ou trousse selon la revendication 11, pour une utilisation pour le renforcement d'une immunité innée et/ou d'une réponse immunitaire spécifique, et/ou pour la prévention et/ou le traitement d'une tumeur.

14. Combinaison pharmaceutique pour une utilisation selon la revendication 13, dans laquelle la tumeur est une tumeur solide, de préférence est choisie dans le groupe suivant constitué de : tumeur du sein, tumeur du côlon, tumeur du foie, tumeur de l'estomac, tumeur du rein, tumeur du pancréas, tumeur de l'ovaire, lymphome, ostéosarcome, gliome, cancer de la prostate et mélanome.

15. Vaccin antitumoral approprié pour une utilisation dans la prévention d'une récidive postopératoire d'un cancer, dans lequel le vaccin antitumoral comprend une enveloppe externe de membrane cellulaire hybride et un matériau de noyau interne, et l'enveloppe externe de membrane cellulaire hybride comprend une membrane interne de bactérie Gram négatif et une membrane de cellule de tumeur solide dérivées d'une résection chirurgicale.

16. Procédé pour la préparation du vaccin antitumoral pour la prévention d'une récidive postopératoire du cancer selon la revendication 15, dans lequel le procédé de préparation comprend les étapes suivantes : (1) extraction respective d'une membrane interne de bactérie Gram négatif et d'une membrane de cellule de tumeur solide dérivées d'une résection chirurgicale ;
(2) mélange de la membrane interne de bactérie Gram négatif et de la membrane de cellule de tumeur solide dérivées d'une résection chirurgicale extraites à l'étape (1), et extrusion du mélange avec une extrudeuse de liposomes pour obtenir une nanoparticule à membrane hybride ; et
(3) mélange de la microparticule à membrane hybride obtenue à l'étape (2) avec un noyau interne de nanoparticule et extrusion du mélange avec l'extrudeuse de liposomes pour obtenir le vaccin antitumoral pour la prévention d'une récidive postopératoire du cancer.

17. Vaccin antitumoral approprié pour la prévention d'une récidive postopératoire du cancer selon la revendication 15, pour une utilisation dans l'amélioration des réponses immunitaires innées et spécifiques d'un corps.
